# FASCICULE DE BREVET EUROPEEN

(11) **EP 2 326 649 B1**
(45) Date de publication et mention de la délivrance du brevet: **28.03.2012**
(21) Numéro de dépôt: 09772661.6
(22) Date de dépôt: 25.06.2009
(51) Int. Cl.: C07D 487/04, A61K 31/407, A61P 35/00, A61P 3/10

(54) **NOUVEAUX PYRROLO[2,3-a]CARBAZOLES ET LEUR UTILISATION COMME INHIBITEURS DES KINASES PIM**
NEUE PYRROLO[2,3-a]CARBAZOLE UND DEREN VERWENDUNG ALS INHIBITOREN VON PIM-KINASE
NOVEL PYRROLO[2,3-a]CARBAZOLES AND USE THEREOF AS PIM KINASE INHIBITORS

(30) Priorité: 03.07.2008 FR 0803752
(43) Date de publication de la demande: 01.06.2011
(73) Titulaire: Centre National de la Recherche Scientifique, 75016 Paris (FR); Universite Blaise Pascal-Clermont-Ferrand II, 63006 Clermont-Ferrand Cedex (FR)
(72) Inventeur: ANIZON, Fabrice, F-63670 La Roche-Blanche (FR); MOREAU, Pascale, F-63800 Cournon d'Auvergne (FR); PRUDHOMME, Michelle, F-63000 Clermont-Ferrand (FR); COHEN, Philip, Dundee DD2 5DQ (GB); ABOAB, Bettina, F-63000 Clermont-Ferrand (FR); AKUE-GEDU, Rufine, F-63000 Clermont-Ferrand (FR); ROSSIGNOL, Emilie, F-63450 Saint Amant Tallende (FR)
(74) Mandataire: Noel, Chantal Odile
(86) Numéro de dépôt international: PCT/FR2009/000788
(87) Numéro de publication internationale: WO 2010/000978

(56) Documents cités:
- WO-A-2007/044407
- WO-A-2007/058942
- FOUSTERIS ET AL.: "Pyrrolo[2,3-a]carbazoles as potential cyclin dependant kinase 1 inhibitors" JOURNAL OF MEDICINAL CHEMISTRY, vol. 51, 2008, pages 1048-1052, XP002514650
- "Structural analysis identifies imidazol[1,2-b]pyridazines as PIM kinase inhibitors with in vitro antileukemic activity" CANCER RESEARCH, vol. 67, no. 14, 2007, pages 6916-6924, XP002514651 cité dans la demande

## Description

L'invention concerne des dérivés de pyrrolo[2,3-a]carbazole, leur procédé de préparation et leur utilisation.

Les inhibiteurs des kinases PIM sont particulièrement recherchés en raison de leur action dans les domaines cardiovasculaires, sur le diabète de type 2, leur activité antiproliférative et leur potentiel d'inhibition de la résistance à certains médicaments de chimiothérapie.

Les kinases PIM, c'est-à-dire les kinases PIM-1, PIM-2 et PIM-3, sont des proto-oncogènes impliqués, en particulier dans le processus de cancérisation.

Elles constituent une famille d'enzymes qui joue un rôle important dans la régulation de nombreux processus, en particulier la survie des cellules, la prolifération et la régulation de la transcription.

Ces enzymes sont surexprimés dans différents types de cancers, en particulier les leucémies, les lymphomes et les cancers de la prostate.

Ces enzymes interviennent également dans les processus de résistance aux agents de chimiothérapie comme la rapamycine et les inhibiteurs de récepteurs de facteurs de croissance à activité tyrosine kinase.

Ce sont des cibles de choix pour le développement de nouveaux agents thérapeutiques, comme l'illustre l'article de Giles et al., "A PIM kinase inhibitor, please", Blood, 2005, 105, 4158-4159.

Il n'existe que très peu d'inhibiteurs spécifiques des kinases PIM. Par inhibiteur spécifique des kinases PIM, on entend un composé qui inhibe fortement les seules kinases PIM, et peu ou pas la plupart des autres kinases.

Il y a quelques 500 kinases encodées par le génome humain, de sorte que le problème de la sélectivité est critique.

En général, un composé est dit spécifique de l'inhibition d'un type particulier de kinases lorsqu'il inhibe ce type de kinase alors qu'il a été testé sur au moins 30 autres kinases.

De nombreuses publications décrivent des composés inhibiteurs des kinases dont les kinases PIM mais ces inhibiteurs ne sont pas sélectifs.

Les seules familles de composés décrits comme étant sélectifs des kinases PIM sont les suivantes :
- les imidazo [1,2-b]pyridazines décrites par Pogacic et al., dans Cancer Research 2007, 67, 6916-6924,
- la quercétagétine décrite par Holder et al., dans Molecular Cancer Therapeutics 2007, 6, 163-172, et
- les complexes de ruthénium décrits par Debreczeni et al., dans Angewandte Chemie, Int. Ed., 2006, 45, 1580-1585.

L'invention repose sur la découverte que certains composés pyrrolo[2,3-a]carbazole sont des inhibiteurs sélectifs d'au moins une des kinases PIM, et que cette sélectivité s'exerce parmi un panel de 67 kinases testées.

Ainsi, l'invention propose l'utilisation d'au moins un composé de formule I suivante: 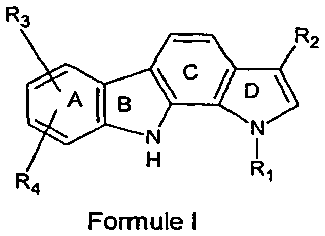 dans laquelle
- R₁ est H ou un groupe sulfophényle,
- R₂, R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocycloalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ ou -S(O)ₜ-Rₐ, -S(O)ₜ-ORₐ, -S(O)ₜNRₐR_{b}, -P(O)t-Ra, -P(O)ₜ-ORₐ, alkyle en C₁ à C₆ linéaire ou ramifié, substitué ou non par un ou plusieurs groupes R₅, alcènyle en C₁ à C₆ linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié. -ORₐ, -NRₐR_{b}, -CO₂Rₐ, -C(O)Rₐ et -C(O)NRₐR_{b}, nitrile, nitro, -NRaC(O)-T₁, alcoxy en C₁ à C₆, oxo, -S(O)ₜ-Rₐ, -S(O)ₜORₐ, -S(O)ₜ-NRₐR_{b}, -P(O)ₜ-Rₐ, - P(O)ₜ-ORₐ,
- Rₐ et R_{b} sont identiques ou différents et représentent chacun indépendamment l'un de l'autre un atome d'hydrogène, ou un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORₐ, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I et n'ont que des buts d'identification de chacun de ces cycles,
   ou un des sels, isomères optiques, mélanges racémiques de ces composés, pour la préparation d'un médicament pour inhiber l'activité d'au moins une des kinases PIM-1, PIM-2 et PIM-3.

De préférence, le au moins un composé utilisé a la formule **I** dans laquelle:
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂. C(NOH)-(C=O)N(Et)₂, NO₂, Br, avec n = 1 ou 2 et R_{c} = OCH₃, OC₂H₅, N(C₂H₅)₂,
- R₃ et R₄ sont identiques ou différents et sont indépendamment l'un de l'autre choisis parmi H, un atome d'halogène, un groupe hétéroaryle à 5 ou 6 chaînons comprenant un ou deux hétéroatomes choisis parmi O et N, alkyle en C₁ à C₆ linéaire ou ramifié, méthoxy, nitro, nitrile, carboxy, trifluorométhyle, trifluorométhoxy, SO₂R_{d}, aryle en C₆ éventuellement substitué par un groupe choisi parmi un groupe (C=O)CH₃, phényle, méthoxy, trifluorométhoxy, trifluorométhyle, carboxy ou par 1 ou 2 atome de fluor, avec R_{d} choisi parmi un groupe OH, CH₃ ou NH₂.

Plus préférablement, le au moins un composé utilisé a la formule I dans laquelle :
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, ou Br, avec n = 1 ou 2 et R_{c} représente un groupe OCH₃, OC₂H₅, ou N(C₂H₅)₂,
- R₃ et R₄ sont identiques ou différents et sont indépendamment l'un de l'autre choisis parmi H, un atome d'halogène, un groupe méthyle, éthyle, nitro, nitrile, trifluorométhyle, aryle en C₆ éventuellement substitué par un groupe choisi parmi un groupe (C=O)CH₃, phényle, méthoxy, trifluorométhoxy, trifluorométhyle, ou par 1 ou 2 atome de fluor,

Le plus préférablement, le au moins un composé de formule I est choisi parmi le 1,10-dihydropyrrolo[2,3-a]carbazole, le 1,10-dihydropyrrolo[2,3-*a*]carbazol-3-carbaldéhyde, le 1,10-dihydropyrrolo[2,3-*a*]carbazol-3-carboxamide, le 1-(1,10-dihydropyrrolo[2,3-*a*]carbazol-3-yl-2,2,2-trifluoroéthanone, le 7-bromo-1,10-dihydropyrrolo[2,3-*a*]carbazole, le 7-bromo-1,10-dihydropyrrolo[2,3-*a*]carbazol-3-carbaldéhyde, le 7-(2,4-difluorophényl)-1,10-dihydropyrrolo[2,3-*a*]carbazol-3-carbaldéhyde, le 6-bromo-1,10-dihydropyrrolo[2,3-*a*]carbazol-3-carbaldéhyde, le 9-bromo-1,10-dihydropyrrolo[2,3-*a*]carbazol-3-carbaldéhyde, le 7-fluoro-1,10-dihydropyrrolo[2,3-*a*]carbazol-3-carbaldéhyde, le 9-fluoro-1,10-dihydropyrrolo[2,3-*a*]carbazol-3-carbaldéhyde, le 6,8-dichloro-1,10-dihydropyrrolo[2,3-*a*]carbazol-3-carbaldéhyde, le 9-éthyl-1,10-dihydropyrrolo[2,3-*a*]carbazol-3-carbaldéhyde et le 8-méthyl-1,10-dihydropyrrolo[2,3-*a*]carbazol-3-carbaldéhyde.

Mais encore plus préférablement, le au moins un composé de formule I utilisé dans l'invention est choisi parmi le 1,10-dihydropyrrolo[2,3-a]carbazole, le 1,10-dihydropyrrolo[2,3-*a*]carbazol-3-carbaldéhyde, le 1,10-dihydropyrrolo[2,3-*a*]carbazol-3-carboxamide, le 1-(1,10-dihydropyrrolo[2,3-*a*]carbazol-3-yl-2,2,2-trifluoroéthanone, le 7-bromo-1,10-dihydropyrrolo[2,3-a]carbazole, le 7-bromo-1,10-dihydropyrrolo[2,3-*a*]carbazol-3-carbaldéhyde, le 7-(2,4-difluorophényl)-1,10-dihydropyrrolo[2,3-*a*]carbazol-3-carbaldéhyde, le 6-bromo-1,10-dihydropyrrolo[2,3-*a*]carbazol-3-carbaldéhyde, le 9-bromo-1,10-dihydropyrrolo[2,3-*a*]carbazol-3-carbaldéhyde, le 7-fluoro-1,10-dihydropyrrolo[2,3-*a*]carbazol-3-carbaldéhyde, le 9-fluoro-1,10-dihydropyrrolo[2,3-*a*]carbazol-3-carbaldéhyde, le 6,8-dichloro-1,10-dihydropyrrolo[2,3-*a*]carbazol-3-carbaldéhyde, le 9-éthyl-1,10-dihydropyrrolo[2,3-*a*]carbazol-3-carbaldéhyde et le 8-méthyl-1,10-dihydropyrrolo[2,3-*a*]carbazol-3-carbaldéhyde.

L'invention propose également un procédé de synthèse des composés de formule I suivante : 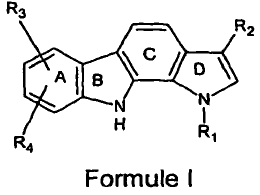 dans laquelle
- R₁ est H ou un groupe sulfophényle,
- R₂, R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocycloalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy-en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b,} -NRₐ-T₂-ORₐ, -NRₐ-T₂-CO₂Rₐ ou -S(O)ₜ-Rₐ, -S(O)ₜ-ORₐ, -S(O)ₜ-NRₐR_{b}, -P(O)ₜ-Rₐ, -P(O)ₜ-ORₐ, alkyle en C₁ à C₆ linéaire ou ramifié, substitué ou non par un ou plusieurs groupes R₅, alcènyle en C₁ à C₆ linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié, -ORₐ, -NRₐR_{b}, -CO₂Rₐ, -C(O)Rₐ et -C(O)NRₐR_{b}, nitrile, nitro, -NRₐC(O)-T₁, alcoxy en C₁ à C₆, oxo, -S(O)ₜRₐ, -S(O)ₜ-ORₐ, -S(O)ₜ-NRₐR_{b}, -P(O)ₜ-Rₐ, - P(O)ₜ-ORₐ,
- Rₐ et R_{b} sont identiques ou différents et représentent chacun indépendamment l'un de l'autre un groupe choisi parmi un atome d'hydrogène, un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé, ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORa, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I et n'ont que des buts d'identification de chacun de ces cycles,
caractérisé en ce qu'il comprend une réaction d'indolisation de Fischer du composé 1-benzènesulfonyl-1,4,5,6-tétrahydro-7*H*-indol-7-one de formule II suivante : 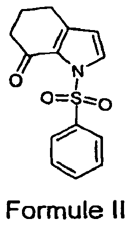 et de la phénylhydrazine ou de phénylhydrazine substituée sur le phényle par un ou plusieurs groupes R₅, en présence d'un liquide ionique qui est le chlorure de zinc-chlorure de choline 2:1 de formule III suivante :

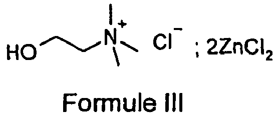

L'invention propose aussi des composés de formule I suivante: 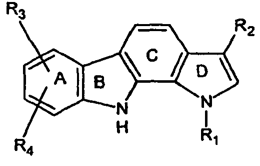 dans laquelle :
- R₁ est H ou un groupe sulfophényle,
- R₂, R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocycloalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ ou -S(O)ₜ-Rₐ, -S(O)ₜ-ORₐ, -S(O)ₜ-NRₐR_{b}, -P(O)ₜ-Rₐ, -P(O)ₜ-ORₐ, alkyle en C₁ à C₆ linéaire ou ramifié, substitué ou non par un ou plusieurs groupes R₅, alcènyle en C₁ à C₆ linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié, -ORₐ, -NRₐR_{b}, -CO₂Rₐ, -C(O)Rₐ et -C(O)NRₐR_{b}, nitrile, nitro, -NRₐC(O)-T₁, alcoxy en C₁ à C₆, oxo, -S(O)ₜ-Rₐ, -S(O)ₜ-ORₐ, -S(O)ₜ-NRₐR_{b}, -P(O)ₜ-Rₐ, - P(O)ₜ-ORₐ,
- Rₐ et R_{b} sont identiques ou différents et représentent chacun indépendamment l'un de l'autre un atome d'hydrogène, ou un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé, ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORₐ, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I et n'ont que des buts d'identification de chacun de ces cycles,
à la condition que :
- R₁, R₂, R₃ et R₄ ne soient pas tous H en même temps,
- lorsque R₁ est un groupe sulfophényle, alors R₂, R₃ et R₄ ne sont pas tous H en même temps, et
- lorsque R₂ est un groupe carboxamide ou formyle, alors R₁, R₃ et R₄ ne sont pas tous H en même temps.

De préférence, ces composés ont la formule I dans laquelle :
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, Br avec n = 1 ou 2 et R_{c} = OCH₃, OC₂H₅, N(C₂H₅)₂,
- R₃ et R₄ sont identiques ou différents et sont indépendamment l'un de l'autre choisis parmi H, un atome d'halogène, un groupe hétéroaryle à 5 ou 6 chaînons comprenant un ou deux hétéroatomes choisis parmi O et N, alkyle en C₁ à C₆ linéaire ou ramifié, méthoxy, nitro, nitrile, carboxy, trifluorométhyle, trifluorométhoxy, SO₂R_{d}, aryle en C₆ éventuellement substitué par un groupe choisi parmi un groupe (C=O)CH₃, phényle, méthoxy trifluorométhoxy, trifluorométhyle, carboxy ou par 1 ou 2 atome de fluor, avec R_{d} choisi parmi un groupe OH, CH₃ ou NH₂.

Plus préférablement, ces composés ont la formule I dans laquelle :
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, ou Br, avec n = 1 ou 2 et R_{c} représente un groupe OCH₃, OC₂H₅, ou N(C₂H₅)₂.
- R₃ et R₄ sont identiques ou différents et sont indépendamment l'un de l'autre choisis parmi H, un atome d'halogène, un groupe méthyle, éthyle, nitro, nitrile, trifluorométhyle, aryle en C₆ éventuellement substitué par un groupe choisi parmi un groupe (C=O)CH₃, phényle, méthoxy, trifluorométhoxy, trifluorométhyle, ou par 1 ou 2 atome de fluor,

Encore plus préférablement, les composés de formule I sont choisis parmi :
- le (1,10-dihydropyrrolo[2,3-*a*]carbazol-3-yl)méthanol,
- le 1-(1,10-dihydropyrrolo[2,3-*a*]carbazol-3-yl)-2,2,2-trifluoroéthanone,
- le 2-(1,10-dihydropyrrolo[2,3-*a*]carbazol-3-yl)-2-oxoacétate de méthyle,
- le 2-(1,10-dihydropyrrolo[2,3-*a*]carbazol-3-yl)-2-oxoacétate d'éthyle,
- le 2-(1,10-dihydropyrrolo[2,3-*a*]carbazol-3-yl)éthanol,
- le 2-(1,10-dihydropyrrolo[2,3-*a*]carbazol-3-yl)-*N,N*-diéthyl-2-oxoacétamide,
- le 2-(1,1 0-dihydropyrrolo[2,3-*a*]carbazol-3-yl)-*N,N*-diéthyléthanamine,
- le 1-(1,10-dihydropyrrolo[2,3-*a*]carbazol-3-yl)-2-diéthylaminoéthanol,
- le 2-(1,10-dihydropyrrolo[2,3-*a*]carbazol-3-yl)-*N,N*-diéthyl-2-hydroxyiminoéthanamide,
- le 1-benzènesulfonyl-7-bromo-1,10-dihydropyrrolo[2,3-*a*]carbazole,
- le 7-bromo-1,10-dihydropyrrolo[2,3-*a*]carbazole,
- le 7-bromo-1,10-dihydropyrrolo[2,3-*a*]carbazol-3-carbaldéhyde,
- le 7-(4-acétylphényl)-1,10-dihydropyrrolo[2,3-*a*]carbazole,
- le 7-(3-méthoxyphényl)-1,10-dihydropyrrolo[2,3-*a*]carbazole,
- le 7-(4-biphényl)-1,10-dihydropyrrolo[2,3-*a*]carbazole.
- le 7-phényl-1,10-dihydropyrrolo[2,3-*a*]carbazole,
- le 7-(4-fluorophényl)-1,10-dihydropyrrolo[2,3-*a*]carbazole,
- le 7-(2,4-difluorophényl)-1,10-dihydropyrrolo[2,3-*a*]carbazole,
- le 7-(4-trifluorométhylphényl)-1,10-dihydropyrrolo[2,3-*a*]carbazole,
- le 7-(4-trifluoromethoxyphényl)-1,10-dihydropyrrolo[2,3-*a*]carbazole,
- le 8-bromo-1,10-dihydropyrrolo[2,3-*a*]carbazole,
- le 6-bromo-1,10-dihydropyrrolo[2,3-*a*]carbazole,
- le 9-bromo-1,10-dihydropyrrolo[2,3-*a*]carbazole,
- le 7-fluoro-1,10-dihydropyrrolo[2,3-*a*]carbazole,
- le 9-fluoro-1,10-dihydropyrrolo[2,3-*a*]carbazole,
- le 6,8-dichloro-1,10-dihydropyrrolo[2,3-*a*]carbazole,
- le 1,10-dihydropyrrolo[2,3-*a*]carbazol-7-carbonitrile,
- le 7-nitro-1,10-dihydropyrrolo[2,3-*a*]carbazole,
- le 9-éthyl-1,10-dihydropyrrolo[2,3-*a*]carbazole,
- le 9-(trifluorométhyl)-1,10-dihydropyrrolo[2,3-*a*]carbazole,
- le 8-méthyl-1,10-dihydropyrrolo[2,3-*a*]carbazole,
- le 7-(3-methoxyphényl)-1,10-dihydropyrrolo[2,3-*a*]carbazol-3-carbaldéhyde,
- le 7-(4-biphényl)-1,10-dihydropyrrolo[2,3-*a*]carbazol-3-carbaldéhyde,
- le 7-phényl-1,10-dihydropyrrolo[2,3-*a*]carbazol-3-carbaldéhyde,
- le 7-(4-fluorophényl)-1,10-dihydropyrrolo[2,3-*a*]carbazol-3-carbaldéhyde,
- le 7-(2,4-difluorophényl)-1,10-dihydropyrrolo[2,3-*a*]carbazol-3-carbaldéhyde,
- le 7-(4-trifluorométhylphényl)-1,10-dihydropyrrolo[2,3-*a*]carbazol-3-carbaldéhyde,
- le 7-(4-trifluoromethoxyphényl)-1,10-dihydropyrrolo[2,3-*a*]carbazol-3-carbaldéhyde,
- le 8-bromo-1,10-dihydropyrrolo[2,3-*a*]carbazol-3-carbaldéhyde,
- le 6-bromo-1,10-dihydropyrrolo[2,3-*a*]carbazol-3-carbaldéhyde,
- le 9-bromo-1,10-dihydropyrrolo[2,3-*a*]carbazol-3-carbaldéhyde,
- le 7-fluoro-1,10-dihydropyrrolo[2,3-*a*]carbazol-3-carbaldéhyde,
- le 9-fluoro-1,10-dihydropyrrolo[2,3-*a*]carbazol-3-carbaldéhyde,
- le 6,8-dichloro-1,10-dihydropyrrolo[2,3-*a*]carbazol-3-carbaldéhyde,
- le 3-formyl-1,10-dihydropyrrolo[2,3-*a*]carbazol-7-carbonitrile,
- le 7-nitro-1,10-dihydropyrrolo[2,3-*a*]carbazol-3-carbaldéhyde,
- le 9-éthyl-1,10-dihydropyrrolo[2,3-*a*]carbazol-3-carbaldéhyde, .
- le 8-méthyl-1,10-dihydropyrrolo[2,3-*a*]carbazol-3-carbaldéhyde.

Les composés les plus préférés de l'invention sont le 1,10-dihydropyrrolo[2,3-*a*]carbazol-3-yl-2,2,2-trifluoroéthanone, le 7-bromo-1,10-dihydropyrrolo[2,3-*a*]carbazole, le 7-bromo-1,10-dihydropyrrolo[2,3-*a*]carbazol-3-carbaldéhyde, le 7-(2,4-difluorophényle)-1,10-dihydropyrrolo[2,3-*a*]carbazol-3-carbaldéhyde, le 6-bromo-1,10-dihydropyrrolo[2,3-*a*]carbazol-3-carbaldéhyde, le 9-bromo-1,10-dihydropyrrolo[2,3-*a*]carbazol-3-carbaldéhyde, le 7-fluoro-1,10-dihydropyrrolo[2,3-*a*]carbazol-3-carbaldéhyde, le 9-fluoro-1,10-dihydropyrrolo[2,3-*a*]carbazol-3-carbaldéhyde, le 6,8-dichloro-1,10-dihydropyrrolo[2,3-*a*]carbazol-3-carbaldéhyde, le 9-éthyl-1,10-dihydropyrrolo[2,3-*a*]carbazol-3-carbaldéhyde et le 8-méthyl-1,10-dihydropyrrolo[2,3-*a*]carbazol-3-carbaldéhyde.

L'invention sera mieux comprise et d'autres avantages et caractéristiques de celle-ci apparaîtront plus clairement à la lecture de la description explicative qui suit.

L'invention repose sur la découverte que les composés ayant la formule I suivante : 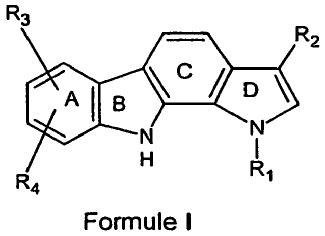 dans laquelle
- R₁ est H ou un groupe sulfophényle,
- R₂, R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocycloalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ ou -S(O)ₜ-Rₐ, -S(O)ₜORₐ, -S(O)ₜ-NRₐR_{b}, -P(O)ₜ-Rₐ, -P(O)ₜ-ORₐ, alkyle en C₁ à C₆ linéaire ou ramifié, substitué ou non par un ou plusieurs groupes R₅, alcènyle en C₁ à C₆ linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié, -ORₐ, -NRₐR_{b}, -CO₂Rₐ, -C(O)Rₐ et -C(O)NRₐR_{b}, nitrile, nitro, -NRₐC(O)-T₁, alcoxy en C₁ à C₆, oxo, -S(O)ₜ-Rₐ, -S(O)ₜ-ORₐ, -S(O)ₜ-NRₐR_{b}, -P(O)ₜ-Rₐ, - P(O)ₜ-ORₐ,
- Rₐ et R_{b} sont identiques ou différents et représentent chacun indépendamment l'un de l'autre un groupe choisi parmi un atome d'hydrogène, un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé, ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORₐ, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I et n'ont que des buts d'identification de chacun de ces cycles,
   sont des inhibiteurs sélectifs d'au moins une kinase PIM-1, PIM-2 et PIM-3 lorsque testés sur 67 kinases.

En raison de leurs propriétés d'inhibition sélective d'au moins une kinase PIM-1, PIM-2 ou PIM-3, ils présentent des propriétés particulièrement intéressantes dans le domaine anticancéreux. Ces composés peuvent être utilisés soit seuls soit en association thérapeutique, en particulier avec d'autres anticancéreux tels que, par exemple, le paclitaxel, le tamoxifène et ses dérivés, le cis-platine et ses analogues, l'irinotécan et ses métabolites, les divers alkylants, l'étoposide, les alcaloïdes de Vinca, les anthracyclines, les nitrosourées, l'hormonothérapie et la radiothérapie.

Ces composés de formule I peuvent être utilisés en tant que tels ou sous la forme de leurs isomères optiques ou sous la forme de mélange racémique de ces isomères optiques ou d'un des sels d'addition de ces composés de la formule I avec un acide ou une base pharmaceutiquement acceptable.

Les composés de formule I préférés, plus préférés, et les plus préférés et encore plus préférés utilisés en tant qu'inhibiteurs sélectifs d'au moins une kinase PIM-1, PIM-2 et PIM-3 ont été définis ci-dessus.

Lorsque R₂, R₃ et/ou R₄ est un groupe qui est lui-même substitué par un ou plusieurs groupes R₅, de préférence il est substitué par un ou deux groupes R₅.

Lorsque le groupe R₂, R₃ ou R₄ est un groupe lui-même substitué par deux groupes R₅, alors les deux groupes R₅ sont de préférence identiques.

Certains composés de formule I ont déjà été décrits. Il s'agit des composés dans lesquels R₁, R₂, R₃ et R₄ sont tous H en même temps, des composés dans lequel R₁ est un groupe sulfophényle et R₂ et R₃ et R₄ sont tous H en même temps, et des composés dans lesquels R₂ est un groupe carboxamide ou un groupe formyle et R₁, R₃ et R₄ sont tous H en même temps.

Cependant, ces composés n'ont jamais été décrits comme des inhibiteurs sélectifs d'au moins une kinase PIM-1, PIM-2 et PIM-3. Il n'a jamais non plus été signalé, avant l'invention, qu'ils pouvaient être de tels inhibiteurs et encore moins avec une telle sélectivité.

En revanche, les composés de formule I dans lesquels R₁, R₂, R₃ et R₄ ne sont pas tous H en même temps ou dans lesquels lorsque R₁ est un groupe sulfophényle mais dans lesquels R₂, R₃ et R₄ ne sont pas tous H en même temps, ou encore dans lesquels R₂ est un groupe carboxamide ou un groupe formyle mais dans lesquels R₁, R₃ et R₄ ne sont pas tous H en même temps, n'ont jamais été décrits auparavant.

Ces composés sont donc un objet de l'invention.

De tels composés préférés, plus préférés et les plus préférés ont été définis auparavant.

L'invention propose également un procédé de fabrication des composés de formule I qui peut être utilisé en alternative au procédé classique de fabrication de ces composés pyrrolo[2,3-a]carbazole.

Ce procédé comprend une réaction d'indolisation de Fischer, de la 1-benzènesulfonyl-1,4,5,6-tétrahydro-7H-indol-7-one avec la phénylhydrazine ou une phénylhydrazine substituée sur le phényle par un ou plusieurs groupes R₅, en présence d'un liquide ionique, le chlorure de zinc-chlorure de choline 2:1.

Afin de mieux faire comprendre l'invention, on va en décrire, à titre d'exemples purement illustratifs et non limitatifs, plusieurs modes de réalisation.

### I) Exemples de synthèses

Les structures des composés décrits dans les exemples qui suivent ont été déterminées selon les techniques usuelles (spectrométrie infrarouge, spectrométrie de masse, spectrométrie de résonance magnétique nucléaire, etc...). Dans les données de spectrométrie de résonnance magnétique nucléaire, l'abréviation "se" désigne un signal élargi.

### Préparation A: synthèse du 1-benzènesulfonylpyrrole

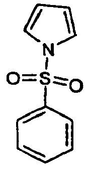

Le 1-benzènesulfonylpyrrole sert à préparer la 1-benzènesulfonyl-1,4,5,6-tétrahydro-7*H*-indol-7-one qui sera utilisée pour la synthèse des composés selon l'invention.

Ce produit est obtenu selon le procédé décrit par B.P. Smart et al., dans J. Med. Chem., 2006, 49, 2858-2860.

### Préparation B : synthèse du 1-benzènesulfonyl-1,4,5,6-tétrahydro-7H-indol-7-one

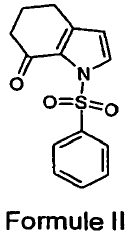

Ce produit est obtenu selon le procédé décrit par M. Kakushima et al., dans J. Org. Chem., 1983, 48, pages 3214-3219.

### Préparation C : préparation du liquide ionique chlorure de zinc-chlorure de choline 2:1

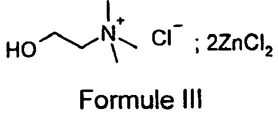

Un mélange de chlorure de choline (10,0 g ; 71,6 mmol) et du chlorure de zinc (19,5 g ; 143 mmol) dans le toluène (200 mL) est porté à reflux dans un Dean-Stark sous agitation vigoureuse pendant 15 heures. Après refroidissement et décantation, le liquide ionique est obtenu sous la forme d'une huile jaune. Le liquide ionique, très hygroscopique, peut être utilisé directement, ou conservé sous argon dans l'éther anhydre.

### EXEMPLE 1: 1-benzènesulfonyl-1,10-dihydropyrrolo[2,3-a]carbazole

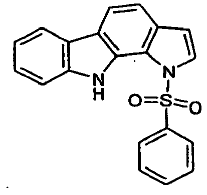

### Méthode 1 :

**Stade A :** Une solution du composé de la préparation B (9,65 g ; 35,0 mmol) et de phénylhydrazine (6,92 mL ; 70,3 mmol) dans le nitrométhane (360 mL) est agitée pendant 3 heures à reflux.

**Stade B :** Du P₂O₅ (9,91 g ; 69,8 mmol) est ajouté à une solution d'hexaméthyldisiloxane (23,9 mL ; 112 mmol) dans le dichlorométhane (350 mL) puis le mélange est agité à reflux pendant 40 minutes. Le solvant est éliminé par distillation et le liquide résiduel est chauffé à 180 °C pendant 5 minutes. L'huile obtenue (PPSE) est diluée dans le nitrométhane (180 mL) et la solution est transférée dans la préparation issue du stade précédent. Le mélange est chauffé sous agitation à reflux pendant 6 heures puis du DDQ (3,50 g ; 15,4 mmol) est ajouté. Le mélange est agité à température ambiante pendant 12 heures avant d'être filtré sur Célite puis concentré sous vide. Et₂O est ensuite ajouté au résidu et la solution est filtrée sur silice. Le filtrat est concentré puis purifié par chromatographie sur colonne de silice flash (éluant : cyclohexane/Et₂O 9:1). Après évaporation, le solide obtenu est lavé au méthanol (20 mL) puis avec Et₂O (2 x 50 mL) ce qui permet d'obtenir le composé voulu (4,40 g ; 12,7 mmol ; R = 36%) sous la forme d'un solide beige.

### Méthode 2 :

Un mélange de phénylhydrazine (393 mg ; 3,63 mmol), du composé de la préparation B (500 mg ; 1,82 mmol) et du liquide ionique de la préparation C (5,25 g ; 12,7 mmol) est agité à 120 °C pendant 2 heures. L'huile brune obtenue est refroidie et une solution aqueuse d'HCl 0,5 M est ajoutée. Le mélange est extrait à l'acétate d'éthyle (3 x 50 mL). Les phases organiques assemblées sont lavées avec une solution aqueuse saturée de N_{A}Cl, séchées sur MgSO₄ et filtrées. La solution obtenue contient le produit d'indolisation, majoritaire, et le produit attendu, minoritaire.

Du DDQ (0,29 g ; 1,28 mmol ; la quantité nécessaire DDQ est déterminée à partir du spectre RMN ¹H du mélange intermédiaire) est ajouté à cette solution et le mélange est agité à température ambiante pendant 15 heures. Le mélange est lavé avec une solution aqueuse saturée de NaCl, séché sur MgSO₄, filtré, puis concentré sur silice, avant purification par chromatographie sur gel de silice flash (pentane/acétate d'éthyle 95:5 puis 90:10) ce qui permet d'obtenir le composé attendu (0,49 g ; 1,41 mmol ; R = 78%) sous la forme d'un solide blanc.
Point de fusion : 148 °C
IR (KBr) : 3430, 1630, 1610 cm⁻¹
Spectre de masse haute résolution (ESI+) : calculée pour C₂₀H₁₅N₂O₂S (M+H)⁺ 347,0854 ; trouvée 347,0861
RMN ¹H (400 MHz, DMSO-*d₆*) 6,85 (1H, d, *J* = 3,5 Hz) ; 7,28-7,39 (4H, m) ; 7,44-7,52 (2H, m) ; 7,53 (1H, d, *J* = 3,5 Hz) ; 7,64 (1H, d, *J* = 8,0 Hz) ; 7,75-7,80 (2H, m) ; 8,01 (1H, d, *J* = 8,0 Hz) ; 8,12 (1H, d, *J* = 8,0 Hz) ; 10,12 (1H, se, NH)
RMN ¹³C (100 MHz, DMSO-*d₆*) 111,4 ; 112,6 ; 113,0 ; 117,1 ; 119,9 (2C) ; 125,5 ; 126,1 ; 126,7 (2C) ; 129,5 (2C) ; 133,9 (CH) ; 120,4 ; 121,9 ; 123,8 ; 127,0 ; 130,6 ; 137,9 ; 138,8 (C)

### EXEMPLE 2 : 1,10-dihydropyrrolo[2,3-a]carbazole

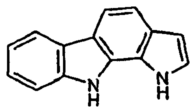

Une solution aqueuse 5M de NaOH (125 mL) est ajoutée à une suspension du composé de l'exemple 1 (3,12 g ; 9,0 mmol) dans le méthanol (1,0 L). Le mélange est agité à reflux pendant 12 heures. Le mélange réactionnel est concentré sous vide jusqu'à la formation d'un précipité qui est récupéré par filtration. Le solide obtenu est lavé avec MeOH (10 mL) puis avec Et₂O (2 x 30 mL) ce qui permet d'obtenir le composé attendu (1,71 g ; 8,3 mmol ; R= 92%) sous la forme d'un solide blanc-cassé.
Point de fusion : 295 °C
IR (KBr): 3420, 3385, 1650, 1455, 1445, 1395, 1345, 1310 cm⁻¹
Spectre de masse haute résolution (ESI+) : calculée pour C₁₄H₁₁N₂ (M+H)⁺ 207,0922 ; trouvée 207,0925
RMN ¹H (400 MHz, DMSO-d₆) : 6,61 (1H, dd, *J*₁ = 3,0 Hz, *J*₂ = 2,0 Hz) ; 7,18 (1H, ddd, *J*₁ = 8,0 Hz, *J*₂ = 7,0 Hz, *J*₃ = 1,0 Hz) ; 7,33 (1H, ddd, *J*₁ = 8,0 Hz, *J*₂ = 7,0 Hz, *J*₃ = 1,0 Hz) ; 7,37 (1H, d, *J* = 8,5 Hz) ; 7,42 (1H, t, *J* = 3,0 Hz) ; 7,64 (1H, d, *J* = 8,0 Hz) ; 7,75 (1H, d, *J* = 8,5 Hz) ; 8,07 (1H, d, *J* = 7,5 Hz) ; 10,88 (1H, se, NH) ; 10,95 (1H, se, NH)
RMN ¹³C (100 MHz, DMSO-*d₆*) : 102,8 ; 111,2 ; 112,0 ; 112,1 ; 118,6 ; 119,0 ; 123,3 ; 123,6 (CH) ; 116,2 ; 121,8 ; 124,1 ; 126,2 ; 126,4 ; 138,1 (C)

### EXEMPLE 3 : 1,10-dihydropyrrolo[2,3-a]carbazol-3-carbaldéhyde

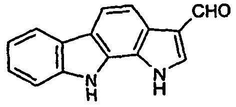

Une solution de chlorure d'oxalyle (19,5 µL ; 0,223 mmol) et de DMF (18,9 µL ; 0,244 mmol) dans le dichlorométhane anhydre (6 mL) est agité à 0 °C pendant 20 minutes. Une solution du composé de l'exemple 2 (43,6 mg ; 0,211 mmol) dans le dichlorométhane anhydre (6 mL) est ensuite ajoutée goutte à goutte. Le mélange est agité à 0 °C pendant 20 minutes puis pendant 2,5 heures à température ambiante. Le solvant est évaporé puis une solution aqueuse de NaOH m/v à 5% (12 mL) est ajoutée. Le mélange est agité à température ambiante pendant 12 heures puis de l'eau est ajoutée. Après extraction à l'AcOEt, la phase organique est séchée sur MgSO₄, filtrée et évaporée. Le résidu est purifié par chromatographie sur gel de silice flash (gradient d'élution de cyclohexane/AcOEt 7:3 à 5:5) ce qui permet d'obtenir le composé attendu (32,4 mg ; 0,138 mmol ; R = 66%) sous la forme d'un solide blanc.
Point de fusion > 290°C
IR (KBr) : 3450-3150, 1630, 1615 cm⁻¹
Spectre de masse haute résolution (ESI+) : calculée pour C₁₅H₁₁N₂O (M+H)⁺ 235,0871 ; trouvée 235,0882
RMN ¹H (500 MHz, DMSO-*d*₆): 7,23 (1H, t, *J* = 7,5 Hz) ; 7,40 (1H, t, *J* = 7,5 Hz); 7,70 (1H, d, *J* = 8,0 Hz) ; 7,96 (1H, d, *J* = 8,5 Hz) ; 8,00 (1H, d, *J* = 8,5 Hz) ; 8,15 (1H, d, *J* = 7,5 Hz) ; 8,33 (1H, d, *J* = 2,0 Hz) ; 10,07 (1H, s) ; 11,10 (1H, se) ; 11,93 (1H, se)
RMN ¹³C (125 MHz, DMSO-d₆) : 111,5 ; 112,1 ; 115,0 ; 119,0 ; 119,5 ; 124,3 ; 136,7 (CH) ; 118,3 ; 119,5 ; 122,5 ; 122,8 ; 123,5 ; 126,0 ; 138,5 (C) ; 185,3 (C=O)

### EXEMPLE 4 : (1,10-dihydropyrrolo[2,3-a]carbazol-3-yl)méthanol

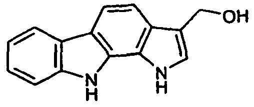

NaBH₄ (320 mg ; 8,46 mmol) est ajouté à une solution de composé de l'exemple 3 (147 mg ; 0,63 mmol) dans le méthanol (30 mL) et le mélange est agité à température ambiante pendant 1 heure. De l'eau (10 mL) est ajoutée et le précipité est récupéré par filtration. Le solide obtenu est lavé au méthanol (5 mL) puis avec Et₂O (2 × 20 mL) ce qui permet d'obtenir le composé attendu (100 mg ; 0,423 mmol, R = 67%) sous la forme d'un solide gris.
Point de fusion > 295 °C
IR (KBr): 3375, 1648, 1456 cm⁻¹
Spectre de masse haute résolution (ESI+): calculée pour C₁₅H₁₁N₂ (M-OH)⁺ 219,0922 ; trouvée 219,0922
RMN ¹H (400 MHz, DMSO-*d*₆): 4,72 (2H, s) ; 4,54-4,96 (1H, se) ; 7,14 (1H, t, *J* = 7,5 Hz) ; 7,26-7,31 (2H, m) ; 7,40 (1H, d, *J* = 8,5 Hz) ; 7,58 (1H, d, *J* = 8,0 Hz) ; 7,71 (1H, d, *J* = 8,5 Hz) ; 8,03 (1H, d, *J* = 7,5 Hz) ; 10,48-11,46 (2H, 2 se)
RMN ¹³C (100 MHz, DMSO-*d*₆) 55,7 (CH₂) ; 110,9 ; 111,1 ; 111,5 ; 118,5 ; 119,0 ; 121,8 ; 123,3 (CH) ; 116,3 ; 117,7 ; 122,1 ; 124,1 ; 125,1 ; 126,4 ; 138,1 (C)

### EXEMPLE 5 : 1,10-dihydropyrrolo[2,3-a]carbazol-3-carboxamide

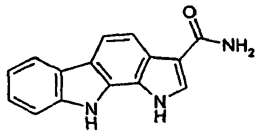

Du chlorosulfonylisocyanate (26,7 µL ; 0,307 mmol) est ajouté à 0°C à une solution du composé de l'exemple 2 (57,4 mg ; 0,278 mmol) dans l'acétonitrile anhydre (10 mL). Le mélange est agité à 0 °C pendant 1,5 heure puis une solution aqueuse d'HCl 1N (5 mL) et du THF (2 mL) sont ajoutés. Le mélange est agité à température ambiante pendant 36 heures puis de l'eau est ajoutée. Après extraction à l'acétate d'éthyle, les phases organiques combinées sont lavées avec une solution aqueuse saturée de NaHCO₃, une solution aqueuse saturée de NaCl puis séchées sur MgSO₄, filtrées et évaporées. Le résidu est purifié par chromatographie sur gel de silice flash (THF) ce qui permet d'obtenir le produit attendu (13,8 mg ; 0,055 mmol ; R = 20%) sous la forme d'un solide beige.
Point de fusion > 300 °C
IR (KBr) : 3430, 3370, 3335, 1650 cm⁻¹
Spectre de masse haute résolution (ESI+) : calculée pour C₁₅H₁₂N₃O (M+H)⁺ 250,0980 ; trouvée 250,0966
RMN ¹H (500 MHz, DMSO-*d*₆) : 6,86 (1H, se) ; 7,20 (1H, t, *J* = 8,0 Hz) ; 7,36 (1H, ddd, *J*₁ = 8,0 Hz, *J*₂ = 7,0 Hz, *J*₃ = 1,0 Hz) ; 7,46 (1H, se) ; 7,65 (1H, d, *J* = 8,0 Hz) ; 7,84 (1H, d, *J* = 8,5 Hz) ; 8,02 (1H, d, *J* = 8,5 Hz) ; 8,08 (1H, d, *J* = 3,0 Hz) ; 8,10 (1H, d, *J* = 8,0 Hz)
RMN ¹³C (100 MHz, DMSO-*d*₆) 111,3 ; 112,8 ; 113,2 ; 118,7 ; 119,2 ; 123,7 ; 126,8 (CH) ; 112,1 ; 116,8 ; 112,1 ; 123,8 ; 124,8 ; 126,2 ; 138,3 (C) ; 166,7 (C=O)

### EXEMPLE 6 : 1-(1,10-dihydropyrrolo[2,3-a]carbazol-3-yl)-2,2,2-trifluoroéthanone

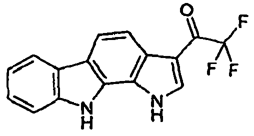

De l'anhydride trifluoroacétique (45 µL ; 0,32 mmol) est ajouté à température ambiante à une solution de composé de l'exemple 2 (52 mg ; 0,25 mmol) dans le DMF (10 mL). Le mélange est agité à température ambiante pendant 18 heures puis de la glace et de l'eau sont ajoutées. Le mélange est extrait à l'acétate d'éthyle (3 × 10 mL) puis les fractions organiques assemblées sont lavées avec une solution aqueuse saturée en NaCl (15 mL), séchées sur MgSO₄, filtrées et évaporées. Le résidu obtenu est trituré avec Et₂O (20 mL) et le mélange est filtré ce qui permet d'obtenir le composé attendu (32 mg ; 0,106 mmol ; R = 42%) sous la forme d'un solide vert.
Point de fusion > 295 °C
IR (KBr) : 3274, 1639 cm⁻¹
Spectre de masse haute résolution (ESI+): calculée pour C₁₆H₁₀F₃N₂O (M+H)⁺ 303,0745 ; trouvée 303,0732
RMN ¹H (400 MHz, DMSO-*d*₆) : 7,22 (1H, t, *J* = 7,5 Hz) ; 7,40 (1H, ddd, *J*₁ = 8,0 Hz, *J*₂ = 7,0 Hz, *J*₃ = 1,0 Hz) ; 7,70 (1H, d, *J* = 8,0 Hz) ; 8,01 (1H, d, *J* = 8,5 Hz) ; 8,07 (1H, d, *J* = 8,5 Hz) ; 8,15 (1H, d, *J* = 8,0 Hz) ; 8,49-8,53 (1H, se) ; 11,03 (1H, s) ; 12,39-12,48 (1H, se)
RMN ¹³C (100 MHz, DMSO-*d*₆) : 111,7 ; 112,1 ; 116,3 ; 119,3 ; 119,7 ; 124,7 ; 135,8 (q, *J*_{CF} = 5 Hz) (CH) ; 110,1 ; 117,0 (q, *J*_{CF} = 292 Hz) ; 119,1 ; 122,3 ; 123,2 ; 124,1 ; 126,0 ; 138,6 (C) ; 174,0 (q, *J*_{CF} = 34 Hz, C=O)

### EXEMPLE 7 : 2-(1,10-dihydropyrrolo[2,3-a]carbazol-3-yl)-2-oxoacétate de méthyle

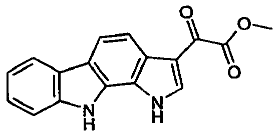

**Stade A :** du chlorure d'oxalyle (660 µL ; 7,7 mmol) est ajouté à 0 °C et à une suspension de composé de l'exemple 2 (825 mg ; 4,00 mmol) dans l'Et₂O anhydre (40 mL). Le mélange est agité à température ambiante pendant 4 heures. Après filtration, le solide est lavé à l'Et₂O (2 × 40 mL) ce qui permet d'obtenir le chlorure d'acide intermédiaire (972 mg) sous la forme d'un solide brun-rouge qui est utilisé sans autre purification.

**Stade B :** NEt₃ (138 µL ; 0,99 mmol) est ajouté à une suspension du chlorure d'acide intermédiaire (243 mg) dans le méthanol (5 mL). Le mélange est agité à température ambiante pendant 4 heures. Après filtration, le solide est lavé avec MeOH (5 mL) puis avec Et₂O (2 × 20 mL) ce qui permet d'obtenir le composé attendu (176 mg ; 0,60 mmol ; R = 60% à partir du composé de l'exemple 2) sous la forme d'un solide orange foncé.
Point de fusion > 290 °C
IR (KBr) : 3461, 3306, 1735, 1590 cm⁻¹
Spectre de masse haute résolution (ESI+): calculée pour C₁₇H₁₂N₂NaO₃ (M+Na)⁺ 315,0746 ; trouvée 315,0754
RMN ¹H (400 MHz, DMSO-*d*₆) : 3,92 (3H, s) ; 7,20 (1H, ddd, *J*₁ = 8,0 Hz, J₂ = 7,0 Hz, J₃ = 1,0 Hz) ; 7,38 (1H, ddd, *J*₁ = 8,0 Hz, J₂ = 7,0 Hz, J₃ = 1,0 Hz) ; 7,67 (1H, d, J = 8,0 Hz) ; 7,99 (1H, d, J = 8,5 Hz) ; 8,02 (1H, d, J = 8,5 Hz) ; 8,13 (1H, d, *J* = 8,0 Hz) ; 8,49 (1H, d, J = 3,5 Hz) ; 11,05 (1H, s, NH) ; 12,14-12,21 (1H, se)
RMN ¹³C (100 MHz, DMSO-d₆) : 52,5 (CH₃) ; 111,6 ; 112,3 ; 115,7 ; 119,1 ; 119,6 ; 124,5 ; 136,7 (CH) ; 113,7 ; 118,7 ; 122,4 ; 123,3 ; 123,8 ; 126,1 ; 138,5 (C) ; 164,1 ; 178,9 (C=O)

### EXEMPLE 8 : 2-(1,10-dihydropyrrolo[2,3-a]carbazol-3-yl)-2-oxoacétate d'éthyle

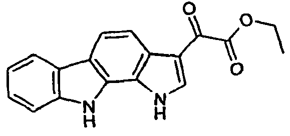

NEt₃ (275 µL ; 1,97 mmol) est ajouté à une suspension du composé préparé au stade A de l'exemple 7 (486 mg) dans l'éthanol (10 mL). Le mélange est agité à température ambiante pendant 4 heures. Après filtration, le solide est lavé avec EtOH (10 mL) puis avec Et₂O (2 × 20 mL) ce qui permet d'obtenir le composé attendu (446 mg ; 1,46 mmol ; R = 73% à partir du composé de l'exemple 2) sous la forme d'un solide jaune-vert.
Point de fusion > 295 °C
IR (KBr) : 3292, 1725, 1602 cm⁻¹
Spectre de masse haute résolution (ESI+): calculée pour C₁₈H₁₄N₂NaO₃ (M+Na)⁺ 329,0902 ; trouvée 329,0907
RMN ¹H (400 MHz, DMSO-*d*₆): 1,37 (3H, t, *J* = 7,0 Hz) ; 4,39 (2H, q, *J* = 7,0 Hz) ; 7,20 (1H, t, *J* = 7,5 Hz) ; 7,38 (1H, ddd, *J*₁ = 8,0 Hz, *J*₂ = 7,0 Hz, *J*₃ = 1,0 Hz) ; 7,67 (1H, d, *J* = 8,0 Hz) ; 7,98-8,03 (2H, m) ; 8,12 (1H, d, *J*= 8,0 Hz) ; 8,47 (1H, d, *J* = 3,0 Hz) ; 11,07 (1H, s) ; 12,09-12,20 (1H, se)
RMN ¹³C (100 MHz, DMSO-*d*₆) : 14,0 (CH₃) ; 61,6 (CH₂); 111,6 ; 112,3 ; 115,7 ; 119,1 ; 119,6 ; 124,5 ; 136,6 (CH) ; 113,7 ; 118,7 ; 122,4 ; 123,3 ; 123,8 ; 126,1 ; 138,5 (C) ; 163,7 ; 179,4 (C=O)

### EXEMPLE 9 : 2-(1,10-dihydropyrrolo[2,3-a]carbazol-3-yl)éthanol

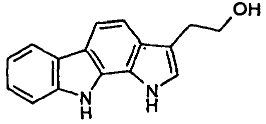

LiAlH₄ (2,00 mL ; 2,00 mmol ; 1 M dans le THF) est ajouté à température ambiante à une suspension du composé de l'exemple 8 (153 mg ; 0,50 mmol) dans le dioxane (10 mL). Le THF est distillé puis le mélange est chauffé à reflux pendant 12 heures. Après retour à température ambiante, de l'eau est ajoutée (5 mL) et le mélange est extrait avec AcOEt (3 × 10 mL). Les fractions organiques combinées sont lavées à l'eau (10 mL), séchées sur MgSO₄, filtrées et évaporées. Le résidu est trituré à l'Et₂O (20 mL) puis filtré ce qui permet d'obtenir le composé attendu (90 mg ; 0,36 mmol, R = 72%) sous la forme d'un solide gris-brun.
Point de fusion: 269-271 °C
IR (KBr) : 3416, 3382, 3234, 1649, 1457 cm⁻¹
Spectre de masse haute résolution (ESI+): calculée pour C₁₆H₁₅N₂O (M+H)⁺ 251,1184 ; trouvée 251,1177
RMN ¹H (400 MHz, DMSO-*d*₆) : 2,92 (2H, t, *J* = 7,5 Hz) ; 3,71 (2H, dt, *J*₁ = 7,5 Hz, *J*₂ = 5,5 Hz) ; 4,63 (1H, t, *J* = 5,5 Hz) ; 7,13 (1H, ddd, *J*₁ = 8,0 Hz, *J*₂ = 7,0 Hz, *J*₃ = 1,0 Hz) ; 7,19 (1H, d, *J* = 2,0 Hz) ; 7,28 (1H, ddd, *J*₁ = 8,0, *J*₂ = 7,0 Hz, *J*₃ = 1;0 Hz) ; 7,31 (1H, d, *J* = 8,5 Hz) ; 7,58 (1H, d, *J* = 8,0 Hz) ; 7,70 (1H, d, *J* = 8,5 Hz) ; 8,02 (1H, d, *J* = 7,5 Hz) ; 10,53-10,57 (1H, se) ; 10,86 (1H, s)
RMN ¹³C (100 MHz, DMSO-*d*₆) : 29,1 ; 61,8 (CH₂) ; 110,4 ; 111,1 ; 111,3; 118,5 ; 119,0 ; 121,2 ; 123,2 (CH) ; 113,3; 116,2 ; 121,9; 124,1 ; 125,9 ; 126,5 ; 138,1 (C)

### EXEMPLE 10 : 2-(1,10-dihydropyrrolo[2,3-a]carbazol-3-yl)-N,N-diéthyl-2-oxoacétamide

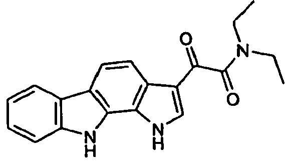

De la diéthylamine (305 µL ; 2,92 mmol) est ajoutée à une suspension du composé préparé au stade A de l'exemple 7 (486 mg) dans l'Et₂O anhydre (10 mL). Le mélange est agité à température ambiante pendant 4 heures. Après filtration, le solide est lavé avec MeOH (3 mL) puis avec Et₂O (2 × 20 mL) ce qui permet d'obtenir le composé attendu (439 mg ; 1,32 mmol, R= 66% à partir du composé de l'exemple 2) sous la forme d'un solide gris pâle.
Point de fusion > 295 °C
IR (KBr) : 3450-3100, 1608, 1429 cm⁻¹
Spectre de masse haute résolution (ESI+): calculée pour C₂₀H₂₀N₃O₂ (M+H)⁺ 334,1556 ; trouvée 334,1543
RMN ¹H (400 MHz, DMSO-*d*₆) : 1,10 (3H, t, *J* = 7,0 Hz) ; 1,22 (3H, t, *J* = 7,0 Hz) ; 3,28 (2H, q, *J* = 7,0 Hz) ; 3,47 (2H, q, *J* = 7,0 Hz) ; 7,20 (1H, ddd, *J*₁ = 8,0 Hz, *J*₂ = 7,0 Hz, *J*₃ = 1,0 Hz) ; 7,37 (1H, ddd, *J*₁ = 8,0 Hz, *J*₂ = 7,0 Hz, *J*₃ = 1,5 Hz) ; 7,66 (1H, d, *J* = 8,0 Hz) ; 7,93 (1H, d, *J* = 8,5 Hz) ; 7,99 (1H, d, *J* = 8,5 Hz) ; 8,07 (1H, d, *J* = 3,0 Hz) ; 8,12 (1H, d, *J* = 8,0 Hz) ; 11,04 (1H, s, NH) ; 11,96-12,01 (1H, se, NH)
RMN ¹³C (100 MHz, DMSO-*d*₆) : 12,7 ; 14,1 (CH₃) ; 38,1 ; 41,7 (CH₂) 111,4 ; 112,1 ; 115,3; 119,0 ; 119,5; 124,4; 134,9 (CH); 114,3 ; 118,3; 122,7 ; 123,2; 123,4 ; 126,1 ; 138,4 (C) ; 167,2 ; 187,0 (C=O)

### EXEMPLE 11 : 2-(1,10-dihydropyrrolo[2,3-a]carbazol-3-yl)-N,N-diéthyléthanamine

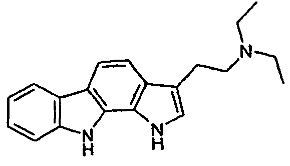

LiAlH₄ (2,00 mL ; 2,00 mmol ; 1 M dans le THF) est ajouté sous argon à temperature ambiante à une suspension du composé de l'exemple 10 (120 mg; 0,360 mmol) dans le dioxane (10 mL). Le THF est distillé puis le mélange est chauffé à reflux pendant 12 heures. Après retour à température ambiante, une goutte d'eau est ajoutée et le mélange est filtré. Le filtrat est concentré puis le résidu est trituré avec Et₂O (20 mL). Après filtration, le solide est lavé avec Et₂O (20 mL) ce qui permet d'obtenir le composé attendu (27 mg ; 0,088 mol ; R = 25%) sous la forme d'un solide gris.
Point de fusion: 224-226 °C
IR (KBr) : 3388, 1649, 1560, 1450 cm⁻¹
Spectre de masse haute résolution (ESI+): calculée pour C₂₀H₂₄N₃ (M+H)⁺ 306,1970 ; trouvée 306,1960
RMN ¹H (400 MHz, DMSO-*d*₆) : 1,02 (6H, t, *J* = 7,0 Hz) ; 2,59 (4H, q, *J* = 7,0 Hz) ; 2,71-2,76 (2H, m) ; 2,83-2,89 (2H, m) ; 7,13 (1H, ddd, *J*₁ = 8,0 Hz, *J*₂ = 7,0 Hz, *J*₃ = 1,0 Hz) ; 7,18-7,20 (1H, m) ; 7,26-7,31 (2H, m) ; 7,58 (1H, d, *J* = 8,0 Hz) ; 7,70 (1H, d, *J* = 8,5 Hz) ; 8,02 (1H, d, *J* = 7,5 Hz) ; 10,52-10,56 (1H, se) ; 10,86 (1H, s)
RMN ¹³C (100 MHz, DMSO-*d*₆) : 12,0 (CH₃) ; 22,8 ; 46,3 (2C) ; 53,4 (CH₂) ; 110,2 ; 111,1 ; 111,3 ; 118,5 ; 118,9 ; 120,9 ; 123,2 (CH) ; 114,7 ; 116,2 ; 121,9 ; 124,0; 125,8 ; 126,5 ; 138,1 (C)

### EXEMPLE 12: 1-(1,10-dihydropyrrolo[2,3-a]carbazol-3-yl)-2-diéthylaminoéthanol

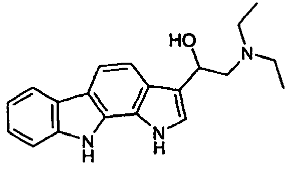

LiAlH₄ (2,00 mL ; 2,00 mmol ; 1M dans le THF) est ajouté à une suspension du composé de l'exemple 10 (181 mg ; 0,543 mmol) dans le THF (10 mL) et le mélange est agité à reflux pendant 24 heures. De l'eau (76 µL), une solution aqueuse à 15% de NaOH (76 µL) puis de l'eau H₂O (228 µL) sont successivement ajoutés. Le mélange est filtré et le filtrat concentré. Le résidu obtenu est lavé à l'eau (3 mL), séché, lavé avec MeOH (3 mL) puis enfin avec Et₂O (10 mL) ce qui permet d'obtenir le composé attendu (57 mg ; 0,177 mmol ; R = 33%) sous la forme d'un solide blanc-cassé.
Point de fusion: 148-150 °C
IR (KBr) : 3274, 1652, 1615, 1455 cm⁻¹
Spectre de masse haute résolution (ESI+): calculée pour C₂₀H₂₄N₃O (M+H)⁺ 322,1919, trouvée 322,1912
RMN ¹H (400 MHz, DMSO-*d*₆) : 0,99 (6H, t, *J* = 7,0 Hz) ; 2,53-2,69 (4H, m) ; 2,74 (1H, dd, *J*₁ = 13,0 Hz, *J*₂ = 5,0 Hz) ; 2,78 (1H, dd, *J*₁ = 13,0 Hz, *J*₂ = 7,5 Hz) ; 4,55-4,63 (1H, se) ; 4,98 (1H, dd, *J*₁ = 7,5 Hz, *J*₂ = 5,0 Hz) ; 7,13 (1H, t, *J* = 7,5 Hz) ; 7,26-7,31 (2H, m) ; 7,41 (1H, d, *J* = 8,5 Hz) ; 7,58 (1H, d, *J* = 8,0 Hz) ; 7,69 (1H, d, *J* = 8,5 Hz) ; 8,02 (1H, d, *J* = 7,5 Hz) ; 10,60-10,65 (1H, se) ; 10,87 (1H, s)
RMN ¹³C (100 MHz, DMSO-*d*₆) : 11,9 (CH₃) ; 46,8 (2C) ; 60,3 (CH₂) ; 65,0 ; 111,1 ; 111,3 ; 111,4 ; 118,5 ; 119,0 ; 120,7 ; 123,3 (CH) ; 116,1 ; 119,6 ; 122,2 ; 124,0 ; 124,4 ; 126,4 ; 138,1 (C)

### EXEMPLE 13 : 2-(1,10-dihydropyrrolo[2,3-a]carbazol-3-yl)-N,N-diéthyl-2-hydroxyiminoéthanamide

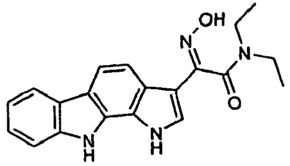

Du chlorhydrate d'hydroxylamine (261 mg ; 3,76 mmol) est ajouté à une solution du composé de l'exemple 10 (167 mg ; 0,50 mmol) dans la pyridine (5 mL) puis le mélange est porté à reflux pendant 12 heures. De l'eau (5 mL) est ajoutée et la pyridine est évaporée sous pression réduite. Le mélange est extrait à l'acétate d'éthyle (3 × 10 mL), les fractions organiques combinées sont lavées avec une solution aqueuse saturée de NaCl (10 mL), séchées sur MgSO₄, filtrées et évaporées. Le résidu obtenu est trituré avec AcOEt (20 mL) et le mélange filtré, ce qui permet d'obtenir le composé attendu (33 mg ; 0,095 mmol ; R= 19%) sous la forme d'un solide beige.
Point de fusion: 268-269 °C
IR (KBr) : 3266, 1609, 1464 cm⁻¹
Spectre de masse haute résolution (ESI+): calculée pour C₂₀H₂₁N₄O₂ (M+H)⁺ 349,1665 ; trouvée 349,1675
RMN ¹H (400 MHz, DMSO-*d*₆) : 1,03 (3H, t, *J* = 7,0 Hz) ; 1,19 (3H, t, *J* = 7,0 Hz) ; 3,26 (2H, q, *J* = 7,0 Hz) ; 3,39-3,59 (2H, m) ; 7,17 (1H, t, *J* = 7,5 Hz) ; 7,33 (1H, t, *J* = 7,5 Hz) ; 7,36 (1H, d, *J* = 2,5 Hz) ; 7,61 (1H, d, *J* = 8,0 Hz) ; 7,83 (1H, d, *J* = 8,5 Hz) ; 7,86 (1H, d, *J* = 8,5 Hz) ; 8,07 (1H, d, *J* = 7,5 Hz) ; 10,90 (1H, s) ; 11,03-11,07 (1H, se) ; 11,21-11,26 (1H, se)
RMN ¹³C (100 MHz, DMSO-*d*₆) : 12,8 ; 13,8 (CH₃) ; 37,6 ; 41,7 (CH₂) ; 111,3 ; 113,2 ; 113,3 ; 118,7 ; 119,3 ; 123,8 ; 125,7 (CH) ; 109,7 ; 117,3 ; 122,5 ; 122,7 ; 123,7 ; 126,2 ; 138,3 (C) ; 150,6 (C=N) ; 164,4 (C=O)

### EXEMPLE 14: 1-benzènesulfonyl-7-bromo-1,10-dihydropyrrolo[2,3-a]carbazole

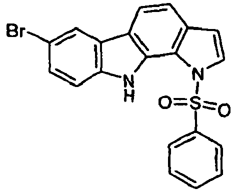

Une suspension de chlorure de 4-bromophénylhydrazine (1,62 g ; 7,3 mmol) et d'acétate de sodium anhydre (0,60 g ; 7,3 mmol) dans le DME (20 mL) est agitée à température ambiante pendant 1 heure. Le composé de la préparation B (1,00 g ; 3,63 mmol) et le liquide ionique de la préparation C (10,0 g ; 24,3 mmol) sont ajoutés. Le solvant est évaporé et le mélange réactionnel est agité à 120 °C pendant 2 heures. L'huile brune obtenue est refroidie et une solution aqueuse d'HCl 0,5 M est ajoutée. Le mélange est extrait 3 fois à l'acétate d'éthyle. Les phases organiques assemblées sont lavées avec une solution aqueuse saturée de NaCl, séchées sur MgSO₄, filtrées et évaporées. Le résidu obtenu est un mélange contenant le produit d'indolisation, majoritaire, et le produit attendu, minoritaire.

Une solution du résidu obtenu et de DDQ (0,7 g ; 3,1 mmol ; la quantité nécessaire DDQ est déterminée à partir du spectre RMN ¹H du résidu obtenu) dans le dioxane (20 mL) est agitée à température ambiante pendant 15 heures. Après évaporation du solvant, de l'acétate d'éthyle est ajouté et le mélange est lavé avec de l'eau et une solution aqueuse saturée de NaCl. La solution organique est séchée sur MgSO₄, filtrée, puis concentrée sur silice, avant purification par chromatographie sur gel de silice flash (pentane/acétate d'éthyle 9:1) ce qui permet d'obtenir le composé attendu (1,15 g ; 2,70 mmol ; R = 74%) sous la forme d'un solide brun.
Point de fusion: 173-175 °C
IR (KBr) : 3434, 1631 cm⁻¹
Spectre de masse haute résolution (ESI+) : calculée pour C₂₀H₁₄⁷⁹BrN₂O₂S (M+H)⁺ 424,9959 ; trouvée 424,9976
RMN ¹H (400 MHz, DMSO-*d*₆) : 7,04 (1H, d, *J* = 3,5 Hz) ; 7,41 (1H, d, *J* = 8,0 Hz) ; 7,49-7,55 (3H, m) ; 7,62 (1H, t, *J* = 7,5 Hz) ; 7,84 (1H, d, *J* = 3,5 Hz) ; 7,92-7,99 (3H, m) ; 8,12 (1H, d, *J* = 8,0 Hz) ; 8,38 (1H, d, *J* = 1,5 Hz) ; 10,81 (1H, s, NH).
RMN ¹³C (100 MHz, DMSO-*d*₆) : 112,4 ; 113,2 ; 114,9 ; 117,4 ; 121,9 ; 126,7 (2C) ; 127,0 ; 127,5 ; 129,9 (2C) ; 134,6 (CH) ; 111,7 ; 119,4 ; 120,1 ; 124,7 ; 126,6 ; 130,8 ; 136,8 ; 137,7 (C).

### EXEMPLE 15 : 7-bromo-1,10-dihydropyrrolo[2,3-a]carbazole

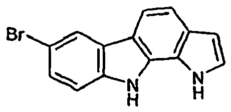

Une solution aqueuse de NaOH 5 N (10 mL) est ajoutée à une suspension du composé de l'exemple 14 (500 mg ; 1,18 mmol) dans le méthanol (100 mL). Le mélange est chauffé à reflux pendant 12 heures. Le mélange réactionnel est concentré sous vide jusqu'à formation d'un précipité. Le mélange est neutralisé avec une solution aqueuse d'HCl concentré puis le solide est collecté par filtration et lavé à l'eau ce qui permet d'obtenir le composé attendu (310 mg ; 1,09 mmol ; R = 92%) sous la forme d'un solide brun.
Point de fusion: 250 °C (décomposition)
Spectre de masse haute résolution (ESI+) calculée pour C₁₄H₁₀⁷⁹BrN₂ (M+H)⁺ 285,0027 ; trouvée 285,0040
IR (KBr) : 3400, 1648, 1438 cm⁻¹
RMN ¹H (400 MHz, DMSO-*d*₆) : 6,59 (1H, dd, *J*₁ = 3,0 Hz, *J*₂ = 2,0 Hz) ; 7,35 (1H, d, *J* = 8,5 Hz) ; 7,38-7,42 (2H, m) ; 7,58 (1H, d, *J* = 8,5 Hz) ; 7,74 (1H, d, *J* = 8,5 Hz) ; 8,25 (1H, d, *J* = 2,0 Hz) ; 10,88-10,92 (1H, se, NH) ; 11,05-11,09 (1H, se, NH)
RMN ¹³C (100 MHz, DMSO-*d*₆) : 102,9 ; 112,2 ; 112,6 ; 113,1 ; 121,5 ; 124,0 ; 125,6 (CH) ; 110,8 ; 115,3 ; 121,5 ; 126,1 ; 126,7 ; 127,1 ; 136,8 (C)

### EXEMPLE 16 : 7-bromo-1,10-dihydropyrrolo[2,3-a]carbazol-3-carbaldéhyde

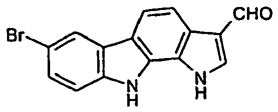

Une solution de chlorure d'oxalyle (32,5 µL ; 0,38 mmol) et de DMF (31,2 µL ; 0,40 mmol) dans le dichlorométhane anhydre (10 mL) est agitée à 0 °C pendant 20 minutes. Une solution du composé de l'exemple 15 (100 mg ; 0,35 mmol) dans le dichlorométhane anhydre (10 mL) est ajoutée goutte à goutte. Le mélange est agité à 0 °C pendant 20 minutes avant de revenir à température ambiante. Le solvant est évaporé puis une solution aqueuse de NaOH à 5% m/v (20 mL) est ajoutée. Le mélange est agité à température ambiante pendant 12 heures puis extrait à l'acétate d'éthyle. La phase organique est lavée à l'eau, avec une solution aqueuse saturée de NaCl, séchée sur MgSO₄, filtrée puis évaporée. Le résidu est purifié par chromatographie sur gel de silice flash (cyclohexane/AcOEt 7:3 puis 5:5) ce qui permet d'obtenir le composé attendu (72 mg ; 0,230 mmol ; R = 66%) sous la forme d'un solide gris.
Point de fusion > 250 °C
Spectre de masse haute résolution (ESI+) calculée pour C₁₅H₁₀⁷⁹BrN₂O (M+H)⁺ 312,9976 ; trouvée 312,9979
IR (KBr) : 3400-3100, 1630 cm⁻¹
RMN ¹H (400 MHz, DMSO-*d*₆) : 7,48 (1H, dd, *J*₁ = 8,5 Hz, *J*₂ = 2,0 Hz) ; 7,65 (1H, d, *J* = 8,5 Hz) ; 7,94 (1H, d, *J* = 8,5 Hz) ; 8,00 (1H, d, *J* = 8,5 Hz) ; 8,31 (1H, d, *J* = 3,0 Hz) ; 8,34 (1H, d, *J* = 2,0 Hz) ; 10,04 (1H, s) ; 11,18-11,23 (1H, se, NH) ; 11,89-11,96 (1H, se, NH)
RMN ¹³C (100 MHz, DMSO-*d*₆) : 112,6 ; 113,5 ; 115,3 ; 122,0 ; 126,6 ; 137,0 (CH) ; 111,2 ; 117,4 ; 119,5 ; 122,5 ; 123,1 ; 125,5 ; 126,6 ; 137,2 (C) ; 185,4 (C=O)

### Procédure générale pour la préparation des composés des exemples 17-19 :

A une solution du composé de l'exemple 14 (425 mg ; 1 mmol) dans le THF (5 mL) sont ajoutés une solution de carbonate de sodium (212 mg ; 2 équivalents) dans l'eau (1 mL), Pd(PPh₃)₂Cl₂ (35,1 mg ; 5 mol%) et l'acide borique correspondant (1,1 équivalent). Le mélange réactionnel est porté à reflux pendant une nuit sous argon. Après retour à température ambiante, le mélange est filtré sur Célite et le solide est lavé à l'acétone. Après évaporation, du méthanol (40 mL) et une solution aqueuse de NaOH 5 M (20 mL) sont ajoutés au résidu obtenu et le mélange est porté à reflux pendant 12 heures. Le mélange réactionnel est concentré sous vide puis neutralisé avec une solution aqueuse d'HCl concentré. De l'acétate d'éthyle est ajouté, la phase organique est collectée, et la phase aqueuse est extraite à l'acétate d'éthyle (3 × 30 mL). Les phases organiques combinées sont lavées avec une solution aqueuse saturée de NaCl, séchées sur MgSO₄, filtrées et évaporées. Le résidu est purifié par chromatographie sur gel de silice flash (pentane/acétate d'éthyle 9:1, 8:2 puis 7:3) ce qui permet d'obtenir le composé attendu.

### EXEMPLE 17 : 7-(4-acétylphényl)-1,10-dihydropyrrolo[2,3-a]carbazole

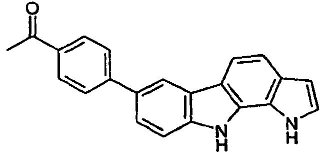

Le composé attendu (195 mg ; 0,60 mmol ; R = 60%) est obtenu sous la forme d'un solide brun.
Point de fusion: 185 °C (décomposition)
Spectre de masse haute résolution (ESI+) : calculée pour C₂₂H₁₇N₂O (M+H)⁺ 325,1341 ; trouvée 325,1354
IR (KBr) : 3700-3100, 1649, 1597 cm⁻¹
RMN ¹H (400 MHz, DMSO-*d*₆) : 2,63 (3H, s, CH₃) ; 6,59-6,61 (1H, m) ; 7,38 (1H, d, *J* = 8,5 Hz) ; 7,39-7,41 (1H, m) ; 7,71-7,73 (2H, m) ; 7,84 (1H, d, *J* = 8,5 Hz) ; 7,96 (2H, d, *J* = 8,5 Hz) ; 8,06 (2H, d, *J* = 8,5 Hz) ; 8,48 (1H, s) ; 10,86-10,90 (1H, se, NH) ; 11,05-11,08 (1H, se, NH)
RMN ¹³C (100 MHz, DMSO-*d*₆) 26,7 (CH₃) ; 102,9 ; 111,8 ; 112,2 ; 112,5 ; 117,7 ; 122,6 ; 123,9 ; 126,5 (2C) ; 128,9 (2C) (CH) ; 116,3 ; 121,7 ; 124,9 ; 126,6 ; 127,0 ; 129,6 ; 134,5 ; 138,3 ; 146,1 (C) ; 197,4 (C=O)

### EXEMPLE 18 : 7-(3-méthoxyphényl)-1,10-dihydropyrrolo[2,3-a]carbazole

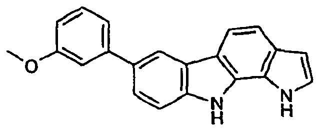

Le composé attendu (172 mg ; 0,55 mmol ; R = 55%) est obtenu sous la forme d'un solide brun.
Point de fusion: 202-205 °C
Spectre de masse haute résolution (ESI+) calculée pour C₂₁H₁₇N₂O (M+H)⁺ 313,1341 ; trouvée 313,1343
IR (KBr) : 3390, 1654, 1609, 1584, 1461 cm⁻¹
RMN ¹H (400 MHz, DMSO-*d*₆) : 3,86 (3H, s, CH₃) ; 6,59 (1H, dd, *J*₁ = 3,0 Hz, *J*₂ = 2,0 Hz) ; 6,90 (1H, ddd, *J*₁ = 8,0 Hz, *J*₂ = 2,5 Hz, *J*₃ = 1,5 Hz) ; 7,29-7,31 (1H, m) ; 7,34 (1H, dt, *J*₁ = 7,5 Hz, *J*₂ = 1,5 Hz) ; 7,36 (1H, d, *J* = 8,5 Hz) ; 7,39 (1H, t, *J* = 7,5 Hz) ; 7,39 (1H, t, *J* = 2,5 Hz) ; 7,61 (1H, dd, *J*₁ = 8,5 Hz, *J*₂ = 1,5 Hz) ; 7,67 (1H, d, *J* = 8,5 Hz) ; 7,82 (1H, d, *J* = 8,5 Hz) ; 8,36 (1H, d, *J* = 1,5 Hz) ; 10,83-10,88 (1H, se, NH) ; 10,95-11,0 (1H, se, NH)
RMN ¹³C (100 MHz, DMSO-*d*₆) : 55,1 (CH₃) ; 102,8 ; 111,5 ; 111,8 ; 112,2 (3C) ; 117,3 ; 119,1 ; 122,6 ; 123,7 ; 129,8 (CH) ; 116,4 ; 121,8 ; 124,7 ; 126,4 ; 127,0 ; 131,0; 137,8; 143,2 ; 159,7 (C)

### EXEMPLE 19 : 7-(4-biphényl)-1,10-dihydropyrrolo[2,3-a]carbazole

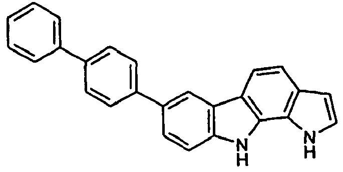

Le composé attendu (230 mg ; 0,64 mmol ; R = 64%) est obtenu sous la forme d'un solide brun.
Point de fusion > 250 °C
Spectre de masse haute résolution (ESI+): calculée pour C₂₆H₁₈N₂ (M)⁺ 358,1470 ; trouvée 358,1492
IR (KBr) : 3650-3100, 1698, 1648 cm⁻¹
RMN ¹H (400 MHz, DMSO-*d*₆) : 6,60 (1H, dd, *J*₁ = 3,0 Hz, *J*₂ = 2,0 Hz) ; 7,36-7,41 (3H, m) ; 7,47-7,52 (2H, m) ; 7,68 (1H, dd, *J*₁ = 8,5 Hz, *J*₂ = 1,5 Hz) ; 7,71 (1H, dd, *J*₁ = 8,5 Hz, *J*₂ = 0,5 Hz) ; 7,73-7,76 (2H, m) ; 7,77-7,80 (2H, m) ; 7,83 (1H, d, *J* = 8,5 Hz) ; 7,87-7,91 (2H, m) ; 8,41-8,43 (1H, m) ; 10,83-10,89 (1H, se, NH) ; 10,98-11,02 (1H, se, NH)
RMN ¹³C (100 MHz, DMSO-*d*₆) : 102,8 ; 111,6 ; 112,2 ; 112,3 ; 117,1 ; 122,4 ; 123,7 ; 126,4 (2C) ; 127,0 (2C) ; 127,1 (2C) ; 127,3 ; 128,9 (2C) (CH) ; 116,4 ; 121,8 ; 124,8 ; 126,5 ; 127,0 ; 130,4 ; 137,8 ; 137,9 ; 139,8 ; 140,6 (C)

### EXEMPLE 20 : 7-phényl-1,10-dihydropyrrolo[2,3-a]carbazole

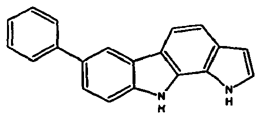

Le composé attendu (169 mg ; 0,60 mmol ; R = 60%) est obtenu sous la forme d'un solide gris.
Point de fusion > 250 °C
Spectre de masse haute résolution (ESI+) calculée pour C₂₀H₁₅N₂ (M⁺H)⁺ 283,1235 ; trouvée 283,1248
IR(KBr): 3434, 3368, 1652 cm⁻¹
RMN ¹H (400 MHz, DMSO-*d₆*) : 6,59 (1H, dd, *J*₁ = 3,0 Hz, *J*₂ = 2,0 Hz) ; 7,30-7,35 (1H, m) ; 7,37 (1H, *J*₁ = 8,5 Hz, *J*₂ = 0,5 Hz) ; 7,40 (1H, dd, *J*₁ = 3,0 Hz, *J*₂ = 2,5 Hz) ; 7,45-7,50 (2H, m) ; 7,61 (1H, dd, *J*₁ = 8,5 Hz, *J*₂ = 2,0 Hz) ; 7,68 (1H, dd, *J*₁ = 8,5 Hz, *J*₂ = 0,5 Hz) ; 7,76-7,79 (2H, m) ; 7,82 (1H, dd, *J*₁= 8,5 Hz, *J*₂ = 0,5 Hz) ; 8,35 (1H, d, *J* = 2,0 Hz) ; 10,83-10,89 (1H, se, NH) ; 10,96-11,00 (1H, se, NH)
RMN ¹³C (100 MHz, DMSO-*d*₆) : 102,9 ; 111,6 ; 112,2 ; 112,3 ; 117,2 ; 122,5 ; 123,7 ; 126,2 ; 126,7 (2C) ; 128,8 (2C) (CH) ; 116,4 ; 121,8 ; 124,8 ; 126,4 ; 127,0; 131,1 ; 137,8 ; 141,6 (C)

### EXEMPLE 21 : 7-(4-fluorophény)-1,10-dihyropyrolo[2,3-a]carbazole

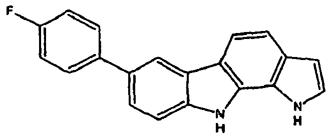

Le composé attendu (150 mg ; 0,50 mmol ; R = 50%) est obtenu sous la forme d'un solide gris-vert.
Point de fusion > 250 °C
Spectre de masse haute résolution (ESI+) calculée pour C₂₀H₁₄FN₂ (M+H)⁺ 301,1141 ; trouvée 301,1154
IR(KBr): 3410, 3397, 1651, 1605, 1514 cm⁻¹
RMN ¹H (400 MHz, DMSO-*d₆*) : 6,59 (1H, dd, *J*₁ = 3,0 Hz, *J*₂ = 2,0 Hz) ; 7,27-7,33 (2H, m) ; 7,36 (1H, d, *J* = 8,5 Hz) ; 7,39 (1H, dd, *J*₁ = 3,0 Hz, *J*₂ = 2,5 Hz) ; 7,58 (1H, dd, *J*₁ = 8,5 Hz, *J*₂ = 2,0 Hz) ; 7,68 (1H, dd, *J*₁ = 8,5 Hz, *J*₂ = 0,5 Hz) ; 7,77-7,83 (3H, m) ; 8,33 (1H, d, *J* = 2,0 Hz) ; 10,84-10,87 (1H, se, NH) ; 10,96-10,98 (1H, se, NH)
RMN ¹³C (100 MHz, DMSO-*d₆*) : 102,8 ; 111,6 ; 112,2 ; 112,3 ; 115,5 (2C, d, *J*_{CF} = 21 Hz) ; 117,2 ; 122,4 ; 123,8 ; 128,5 (2C, d, *J*_{CF} = 8 Hz) (CH) ; 116,3 ; 121,8 ; 124,8 ; 126,5 ; 127,0 ; 130,1 ; 137,7 ; 138,1 (d, *J*_{CF} = 3 Hz) ; 161,2 (d, *J*_{CF} = 243 Hz) (C)

### EXEMPLE 22 : 7-(2,4-difluorophényl)-1,10 dihydropyrrolo[2,3-a]carbazole

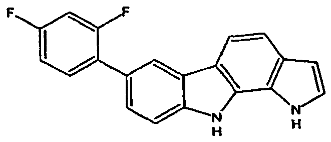

Le composé attendu (165 mg ; 0,52 mmol ; R = 52%) est obtenu sous la forme d'un solide gris-vert.
Point de fusion > 250 °C
Spectre de masse haute résolution (ESI+) calculée pour C₂₀H₁₃F₂N₂ (M+H)⁺ 319,1047 ; trouvée 319,1058
IR (KBr) : 3401, 1652, 1616, 1594, 1510 cm⁻¹
RMN ¹H (400 MHz, DMSO-*d₆*) : 6,59 (1H, dd, *J*₁ = 3,0 Hz, *J*₂ = 2,0 Hz) ; 7,21 (1H, tdd, *J*₁ = 8,5 Hz, *J*₂ = 3,0 Hz, *J*₃ = 1,0 Hz) ; 7,33-7,39 (2H, m) ; 7,40 (1H, t, *J* = 2,5 Hz) ; 7,45 (1H, dt, *J*₁ = 8,5 Hz, *J*₂ = 2,0 Hz) ; 7,67 (1H, td, *J*₁ = 9,0 Hz, *J*₂ = 6,5 Hz) ; 7,69 (1H, d, *J* = 8,5 Hz) ; 7,78 (1H, d, *J* = 8,5 Hz) ; 8,19 (1H, s) ; 10,85-10,88 (1H, se, NH) ; 11,03-11,06 (1H, se, NH)
RMN ¹³C (100 MHz, DMSO-*d₆*) : 102,9 ; 104,3 (dd, *J*_{CF1} = 27 Hz, *J*_{CF2} = 26 Hz) ; 111,3 ; 111,8 (dd, *J*_{CF1} = 21 Hz, *J*_{CF2} = 4 Hz) ; 112,1 ; 112,4 ; 119,4 (d, *J*_{CF} = 3 Hz) ; 123,8 ; 124,3 (d, *J*_{CF} = 3 Hz) ; 132.1 (dd, *J*_{CF1} = 10 Hz, *J*_{CF2} = 5 Hz) (CH) ; 116,2 ; 121,7 ; 124,4 ; 124,8 (d, *J*_{CF} = 1 Hz) ; 126,3. (dd, *J*_{CF1} = 14 Hz, *J*_{CF2} = 4 Hz) ; 126,5 ; 126,9 ; 137,7 ; 159,1 (dd, *J*_{CF1} = 247 Hz, *J*_{CF2} = 12 Hz) ; 161,1 (dd, *J*_{CF1} = 246 Hz, *J*_{CF2} = 12 Hz)

### EXEMPLE 23 : 7-(4-trifluorométhylphényl)-1,10-dihydropyrrolo[2,3-a]carbazole

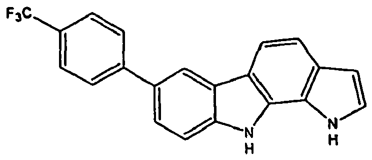

Le composé attendu (227 mg ; 0,65 mmol ; R = 65%) est obtenu sous la forme d'un solide gris.
Point de fusion > 250 °C
Spectre de masse haute résolution (ESI+) calculée pour C₂₁H₁₄F₃N₂ (M+H)⁺ 351,1109 ; trouvée 351,1125
IR(KBr): 3390, 3362, 1654, 1614 cm⁻¹
RMN ¹H (400 MHz, DMSO-d₆) : 6,60 (1H, dd, *J*₁ = 3,0 Hz, *J*₂ = 2,0 Hz) ; 7,39 (1H, d, *J* = 8,5 Hz) ; 7,41 (1H, dd, *J*₁ = 3,0 Hz, *J*₂ = 2,5 Hz) ; 7,69 (1H, dd, *J*₁ = 8,5 Hz, *J*₂ = 2,0 Hz) ; 7,73 (1H, dd, *J*₁ = 8,5 Hz, *J*₂ = 0,5 Hz) ; 7,81 (2H, d, *J* = 8,0 Hz) ; 7,84 (1H, d, *J* = 8,5 Hz) ; 8,01 (2H, d, *J* = 8,0 Hz) ; 8,47 (1H, d, *J* = 1,5 Hz); 10,86-10,92 (1H, se, NH); 11,06-11,10 (1H, se, NH)
RMN ¹³C (100 MHz, DMSO-*d₆*) : 102,9 ; 111,8 ; 112,2 ; 112,5 ; 117,7 ; 122,6 ; 123,9 ; 125,6 (2C, q, *J*_{CF} = 4 Hz) ; 127,2 (2C) (CH) ; 116,3 ; 121,8 ; 124,6 (q, *J*_{CF} = 272 Hz) ; 124,9 ; 126,5 (q, *J*_{CF} 32 Hz) ; 126,6 ; 127,1 ; 129,3 ; 138,3 ; 145,6 (C)

### EXEMPLE 24 : 7-(4-trifluoromethoxyphényl)-1,10-dihydropyrrolo[2,3-a]carbazole

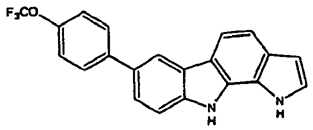

Le composé attendu (226 mg ; 0,62 mmol ; R = 62%) est obtenu sous la forme d'un solide gris.
Point de fusion > 250 °C
Spectre de masse haute résolution (ESI+) calculée pour C₂₁H₁₄F₃N₂O (M+H)⁺ 367,1058 ; trouvée 367,1044
IR(KBr) : 3401, 1652, 1514, 1464 cm⁻¹
RMN ¹H (400 MHz, DMSO-*d₆*) : 6,60 (1H, dd, *J*₁ = 3,0 Hz, *J*₂ = 2,0 Hz) ; 7,37 (1H, d, *J* = 8,5 Hz) ; 7,40 (1H, t, *J* = 2,5 Hz) ; 7,46 (2H, d, *J* = 8,0 Hz) ; 7,62 (1H, dd, *J*₁ = 8,5Hz, J₂ = 1,5Hz) ; 7,70(1H, d, *J* = 8,5Hz) ; 7,81 (1H, d, *J* = 8,5 Hz) ; 7,87-7,91 (2H, m) ; 8,38 (1H, d, *J* = 1,5 Hz) ; 10,85-10,90 (1H, se, NH) ; 11,00-11,05 (1H, se, NH)
RMN ¹³C (100 MHz, DMSO-*d₆*) : 102,9 ; 111,7 ; 112,2 ; 112,4 ; 117,5 ; 121,4 (2C) ; 122,5 ; 123,8 ; 128,3 (CH) ; 116,3 ; 120,2 (q, *J*_{CF} = 256 Hz) ; 121,8 ; 124,8 ; 126,5 ; 127,0 ; 129,6 ; 138,0; 141,0; 147,0 (q, *J*_{CF} = 2 Hz) (C)

### Procédure générale pour la préparation des composés des exemples 25-34

Stade A : une suspension de chlorhydrate de phénylhydrazine substituée and d'acétate de sodium anhydre dans le DME (20 mL) est agitée à température ambiante pendant 1 heure. Le composé de la préparation B (1,10 g ; 4,0 mmol) et le liquide ionique de la préparation C (11,54 g ; 28 mmol) sont ajoutés. The solvant est évaporé et le mélange est chauffé à 120 °C pendant 12 heures. Après refroidissement, une solution aqueuse d'acide chlorhydrique 0,5 M est ajoutée avant extraction avec de l'acétate d'éthyle. Les fractions organiques sont lavées avec une solution aqueuse saturée de NaCl, séchées sur MgSO₄ et filtrées. La solution contient le produit attendu et est utilisée directement pour l'étape suivante d'oxydation.

Stade B : Du DDQ est ajouté à cette solution et le mélange réactionnel est agité à température ambiante pendant une nuit. Le mélange est lavé avec une solution aqueuse saturée de NaCl, séché sur MgSO₄ et concentré sous vide. Stade C : le brut réactionnel est dissous dans le méthanol (100 mL), une solution aqueuse de KOH 5 M (25 mL) est ajoutée et le mélange est chauffé à reflux pendant 12 heures. Après refroidissement, le solvant est éliminé sous vide. Le résidu est neutralisé à l'aide d'acide chlorhydrique concentré. Après extraction avec de l'acétate d'éthyle, les fractions organiques sont rassemblées puis lavées avec une solution aqueuse saturée de NaCl, séchées sur MgSO₄ et évaporées. Le solide obtenu est purifié par chromatographie sur gel de silice.

### EXEMPLE 25 : 8-bromo-1,10-dihydropyrrolo[2,3-a]carbazole et

### EXEMPLE 26 : 6-bromo-1,10-dihidropyrrolo[2,3-a]carbazole

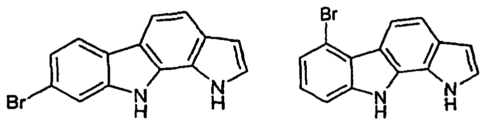

Stade A : chlohydrate de 3-bromophénylhydrazine (1,00 g ; 4,47 mmol), acétate de sodium (367 mg ; 4,47 mmol) ; stade B : DDQ (454 mg ; 2,00 mmol) ; stade C : une chromatographie sur gel de silice (cyclohexane/AcOEt, 9:1 puis 8:2) permet d'isoler le composé de l'exemple 25 (513 mg ; 1,80 mmol ; R = 45%) et le composé de l'exemple 26 (342 mg ; 1,20 mmol ; R = 30%) sous la forme de solides gris.

### Exemple 25 :

Point de fusion > 250 °C
Spectre de masse (El) *m*/*z :* 284/286, M⁺
IR (ATR) : 3397, 1649, 1607 cm⁻¹
RMN ¹H (400 MHz, DMSO-*d₆*) : 6,59 (1H, dd, *J*₁ = 3,0 Hz, *J*₂ = 2,0 Hz) ; 7,27 (1H, dd, *J*₁ = 8,5 Hz, *J*₂ = 2,0 Hz) ; 7,37 (1H, dd, *J*₁ = 8,5 Hz, *J*₂ = 0,5 Hz) ; 7,40 (1H, dd, *J*₁ = 3,0 Hz, *J₂* = 2,5 Hz) ; 7,71 (1H, dd, *J*₁ = 8,5 Hz, *J*₂ = 0,5 Hz) ; 7,85 (1H, dd, *J*₁ = 2,0 Hz, *J*₂ = 0,5 Hz) ; 7,99 (1H, d, *J* = 8,5 Hz) ; 10,94 (1H, se, NH) ; 11,01 (1H, se, NH)
RMN ¹³C (100 MHz, DMSO-*d₆*) : 102,9 ; 111,9 ; 112,7 ; 113,9 ; 120,7 ; 121,3 ; 124,0 (CH) ; 115,6 ; 115,8 ; 121,6 ; 123,3 ; 126,6 ; 126,7 ; 139,1 (C)

### Exemple 26 :

Point de fusion : 249-251 °C
Spectre de masse haute résolution (ESI+) calculée pour C₁₄H₁₀⁷⁹BrN₂ (M+H)⁺ 285,0027 ; trouvée 285,0019
IR(ATR) : 3406, 3364, 1653, 1613 cm⁻¹
RMN ¹H (400 MHz, DMSO-*d₆*) : 6,62 (1H, dd, *J*₁ = 3,0 Hz, *J*₂ = 2,0 Hz) ; 7,23 (1H, t, *J* = 8,0Hz) ; 7,35 (1H, dd, *J*₁ = 7,5 Hz, *J*₂ = 0,5 Hz) ; 7,42 (1H, d, *J* = 8,5 Hz) ; 7,44 (1H, t, *J* = 2,5 Hz) ; 7,66 (1H, dd, *J*₁ = 8,0 Hz, *J*₂ = 0,5 Hz) ; 8,24 (1H, d, *J*= 8,5 Hz) ; 10,90 (1H, se, NH) ; 11,31 (1H, se, NH)
RMN ¹³C (100 MHz, DMSO-*d₆*) 102,8 ; 110,6 ; 112,3 ; 113,4 ; 122,5 ; 124,3 ; 124,4 (CH) ; 114,2 ; 115,4 ; 121,4 ; 122,4 ; 126,6 ; 127,0 ; 139,4 (C)

### EXEMPLE 27 : 9-bromo-1,10-dihydropyrrolo[2,3-a]carbazole

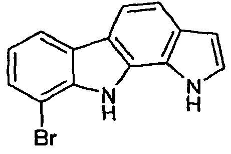

Stade A : chlorhydrate de 2-bromophénylhydrazine (1,00 g ; 4,47 mmol), acétate de sodium (367 mg ; 4,47 mmol) ; stade B : DDQ (636 mg ; 2,80 mmol) ; stade C : une chromatographie sur gel de silice (cyclohexane/AcOEt, de 9:1 à 7:3) permet d'isoler le composé attendu (780 mg ; 2,74 mmol ; R = 68%) sous la forme d'un solide blanc.
Point de fusion > 250 °C
Spectre de masse haute résolution (ESI+) calculée pour C₁₄H₁₀⁷⁹BrN₂ (M+H)⁺ 285,0027 ; trouvée 285,0012
IR(ATR) : 3314, 1636, 1622 cm⁻¹
RMN ¹H (400 MHz, DMSO-*d₆*) : 6,60 (1H, dd, *J*₁ = 3,0 Hz, *J*₂ = 2,0 Hz) ; 7,11 (1H, t, *J* = 7,5 Hz) ; 7,40 (1H, dd, *J*₁ = 8,5 Hz, *J*₂ = 0,5 Hz) ; 7,45 (1H, dd, *J*₁ = 3,0 Hz, *J*₂ = 2,5 Hz) ; 7,52 (1H, dd, *J*₁ = 7,5 Hz, *J*₂ = 1,0 Hz) ; 7,73 (1H, dd, *J*₁ = 8,5 Hz, *J*₂ = 0,5 Hz) ; 8,07 (1H, dt, *J*₁ = 7,5 Hz, *J*₂ = 0,5 Hz) ; 10,79 (1H, se, NH) ; 11,12 (1H, se, NH)
RMN ¹³C (100 MHz, DMSO-d₆) : 103,0 ; 112,3 ; 113,2 ; 118,6 ; 120,3 ; 124,2 ; 125,7 (CH) ; 103,6 ; 116,4 ; 121,7 ; 125,9 ; 126,5 ; 126,7 ; 136,5 (C)

### EXEMPLE 28 : 7-fluoro-1,10-dihydropyrrolo[2,3-a]carbazole

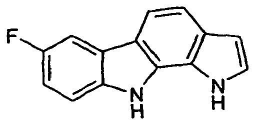

Stade A : chlorhydrate de 4-fluorophénylhydrazine (1,30 g ; 8,0 mmol), acétate de sodium (656 mg ; 8,0 mmol) ; stade B : DDQ (545 mg ; 2,40 mmol) ; stade C : une chromatographie sur gel de silice (cyclohexane/AcOEt, de 9:1 à 7:3) permet d'isoler le composé attendu (702 mg ; 3,13 mmol ; R = 78%) sous la forme d'un solide gris-vert.
Point de fusion > 250 °C
Spectre de masse haute résolution (ESI+) calculée pour C₁₄H₁₀FN₂ (M+H)⁺ 225,0828 ; trouvée 225,0815
IR(ATR) : 3418, 3387, 1645, 1584 cm⁻¹
RMN ¹H (400 MHz, DMSO-*d₆*) : 6,58 (1H, dd, *J*₁ = 3,0 Hz, *J*₂ = 2,0 Hz) ; 7,12 (1H, ddd, *J*₁ = 9,5 Hz, *J*₂ = 9,0 Hz, *J*₃ = 2,5 Hz) ; 7,34 (1H, d, *J* = 8,5 Hz) ; 7,39 (1H, t, *J* = 2,5 Hz) ; 7,60 (1H, dd, *J*₁ = 8,5 Hz, *J*₂ = 4,5 Hz) ; 7,71 (1H, d, *J* = 8,5 Hz) ; 7,84 (1H, dd, *J*₁ = 9,5 Hz, *J*₂ = 2,5 Hz) ; 10,89 (1H, se, NH) ; 10,92 (1H, se, NH) .
RMN ¹³C (100 MHz, DMSO-*d₆*) : 102,9 ; 104,5 (d, *J*_{CF} = 24 Hz) ; 110,7 (d, *J*_{CF} = 25 Hz) ; 112,0 (d, *J*_{CF} = 9 Hz) ; 112,2 (2C) ; 123,9 (CH) ; 116,0 (d, *J*_{CF} = 4 Hz) ; 121,6 ; 124,6 (d, *J*_{CF} = 10 Hz) ; 126,5 ; 127,8 ; 134,6 ; 156,7 (d, *J*_{CF} = 232) (C)

### EXEMPLE 29 : 9-fluoro-1,10-dihydropyrrolo[2,3-a]carbazole

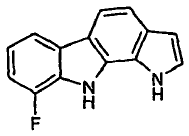

Stade A : chlorhydrate de 2-fluorophénylhydrazine (1,00 g ; 6,15 mmol), acétate de sodium (505 mg ; 6,16 mmol) ; stade B : DDQ (450 mg ; 1,98 mmol) ; stade C : une chromatographie sur gel de silice (cyclohexane/AcOEt, de 9:1 à 7:3) permet d'isoler le composé attendu (495 mg ; 2,21 mmol ; R = 55%) sous la forme d'un solide beige.
Point de fusion > 250 °C
Spectre de masse haute résolution (ESI+) calculée pour C₁₄H₁₀FN₂ (M+H)⁺ 225,0828 ; trouvée 225,0832
IR(ATR) : 3424, 3376, 1651, 1578 cm⁻¹
RMN ¹H (400 MHz, DMSO-*d₆*) 6,60 (1H, dd, *J*₁ = 3,0 Hz, *J*₂ = 2,0 Hz) ; 7,08-7,19 (2H, m) ; 7,39 (1H, d, *J* = 8,5 Hz) ; 7,42 (1H, t, *J* = 2,5 Hz) ; 7,73 (1H, d, *J* = 8,5Hz) ; 7,88 (1H, d, *J* = 7,5Hz) ; 10,61 (1H, se, NH) ; 11,40 (1H, se, NH)
RMN ¹³C (100 MHz, DMSO-*d₆*) : 102,9 ; 108,6 (d, *J*_{CF} = 16 Hz) ; 112,1 ; 112,9 ; 115,3 (d, *J*_{CF} = 3 Hz) ; 119,0 (d, *J*_{CF} = 6 Hz) ; 124,1 (CH) ; 116,2 (d, *J*_{CF} = 2,5 Hz) ; 121,7 ; 125,5 (d, *J*_{CF} = 13 Hz) ; 126,7 ; 126,9 ; 128,0 (d, *J*_{CF} = 6 Hz) ; 148,9 (d, *J*_{CF} = 241 Hz) (C)

### EXEMPLE 30 : 6,8-dichloro-1,10-dihydropyrrolo[2,3-a]carbazole

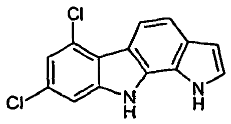

Stade A : chlorhydrate de 3,5-dichlorophénylhydrazine (1,00 g ; 4,68 mmol), acétate de sodium (384 mg ; 4,68 mmol) ; stade B : DDQ (908 mg ; 4,00 mmol) ; stade C : une chromatographie sur gel de silice (cyclohexane/AcOEt, de 9:1 à 7:3) permet d'isoler le composé attendu (971 mg ; 3,53 mmol ; R = 88%) sous la forme d'un solide gris.
Point de fusion > 250 °C
Spectre de masse haute résolution (ESI+) calculée pour C₁₄H₉³⁵Cl₂N₂ (M+H)⁺ 275,0143 ; trouvée 275,0135
IR(ATR): 3431, 3368, 1653, 1616 cm⁻¹
RMN ¹H (400 MHz, DMSO-*d₆*) : 6,62 (1H, dd, *J*₁ = 3,0 Hz, *J*₂ = 2,0 Hz) ; 7,27 (1H, d, *J* = 1,5 Hz) ; 7,44 (1H, dd, *J*₁ = 8,5 Hz, *J*₂ = 0,5 Hz) ; 7,46 (1H, dd, *J*₁ = 3,0 Hz, *J*₂ = 2,5 Hz) ; 7,76 (1H, d, *J* = 1,5 Hz) ; 8,05 (1H, d, *J* = 8,5 Hz) ; 11,00 (1H, se, NH) ; 11,42 (1H, se, NH)
RMN ¹³C (100 MHz, DMSO-*d₆*) : 102,9 ; 110,2 ; 113,3 ; 113,4 ; 118,9 ; 124,7 (CH) ; 114,4 ; 120,0 ; 121,2 ; 126,3 ; 126,9 ; 127,3 ; 127,5 ; 139,5 (C)

### EXEMPLE 31 : 1,10-dihydropyrrolo[2,3-a]carbazol-7-carbonitrile

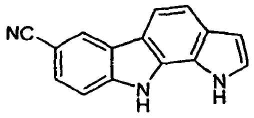

Stade A : chlorhydrate de 4-cyanophénylhydrazine (1,36 g ; 8,0 mmol), acétate de sodium (656 mg ; 8,0 mmol) ; stade B : DDQ (545 mg ; 2,40 mmol) ; stade C : une chromatographie sur gel de silice (cyclohexane/AcOEt, de 9:1 à 7:3) permet d'isoler le composé attendu (517 mg ; 2,24 mmol ; R = 56%) sous la forme d'un solide beige.
Point de fusion > 250 °C
Spectre de masse haute résolution (ESI+) calculée pour C₁₅H₁₀N₃ (M+H)⁺ 232,0875 ; trouvée 232,0872
IR(ATR) : 3439, 3256, 2226, 1655, 1614 cm⁻¹
RMN ¹H (400 MHz, DMSO-d₆) : 6,63 (1H, dd, *J*₁ = 3,0 Hz, *J*₂ = 2,0 Hz) ; 7,42-7,46 (2H, m) ; 7,67 (1H, dd, *J*₁ = 8,5 Hz, *J*₂ = 1,5 Hz) ; 7,78 (1H, dd, *J*₁ = 8,5 Hz, *J*₂ = 0,5 Hz) ; 7,83 (1H, d, *J* = 8,5 Hz) ; 8,61 (1H, m) ; 10,97 (1H, se, NH) ; 11,53 (1H, se, NH)
RMN ¹³C (100 MHz, DMSO-*d₆*) : 103,0 ; 112,2 ; 112,3 ; 113,5 ; 124,2 ; 124,6 ; 126,5 (CH) ; 100,3 ; 115,4 ; 120,9 ; 121,4 ; 124,3 ; 127,2 ; 127,3 ; 140,1 (C)

### EXEMPLE 32 : 7-nitro-1,10-dihydropyrrolo[2,3-a]carbazole

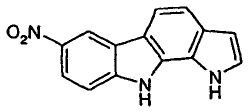

Stade A : chlorhydrate de 4-nitrophénylhydrazine (1,52 g ; 8,0 mmol), acétate de sodium (656 mg ; 8,0 mmol) ; stade B : DDQ (545 mg ; 2,40 mmol) ; stade C : une chromatographie sur gel de silice (cyclohexane/AcOEt, de 9:1 à 3:7) permet d'isoler le composé attendu (450 mg ; 1,79 mmol ; R = 45%) sous la forme d'un solide brun-jaune.
Point de fusion : 220 °C (décomposition)
Spectre de masse haute résolution (ESI+) calculée pour C₁₄H₁₀N₃O₂ (M+H)⁺ 252,0773 ; trouvée 252,0763
IR(ATR) : 3406, 3298, 1655, 1616 cm⁻¹
RMN ¹H (400 MHz, DMSO-*d₆*) : 6,64 (1H, dd, *J*₁ = 3,0 Hz, *J*₂ = 2,0 Hz) ; 7,46-7,49 (2H, m) ; 7,79 (1H, d, *J* = 9,0 Hz) ; 7,93 (1H, d, *J* = 8,5 Hz) ; 8,22 (1H, dd, *J*₁ = 9,0 Hz, *J*₂ = 2,5 Hz) ; 9,06 (1H, d, *J* = 2,5 Hz) ; 11,00 (1H, se, NH) ; 11,74 (1H, se, NH)
RMN ¹³C (100 MHz, DMSO-*d₆*) : 103,1 ; 111,4 ; 112,4 ; 113,9 ; 115,9 ; 119,1 ; 124,8 (CH) ; 116,3 ; 121,5 ; 123,9 ; 127,6 ; 127,9 ; 140,0 ; 141,7 (CH)

### EXEMPLE 33 : 9-éthyl-1,10-dihydropyrrolo[2,3-a]carbazole

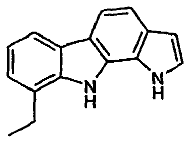

Stade A : chlorhydrate de 2-éthylphénylhydrazine (1,00 g ; 5,79 mmol), acétate de sodium (475 mg ; 5,79 mmol) ; stade B : DDQ (454 mg ; 2,00 mmol) ; stade C : une chromatographie sur gel de silice (cyclohexane/AcOEt, de 9:1 à 7:3) permet d'isoler le composé attendu (490 mg ; 2,09 mmol ; R = 52%) sous la forme d'un solide gris.
Point de fusion : 256-258 °C
Spectre de masse haute résolution (ESI+) calculée pour C₁₆H₁₅N₂ (M+H)⁺ 235,1235 ; trouvée 235,1228
IR(ATR) 3426, 3416, 1649 cm⁻¹
RMN ¹H (400 MHz, DMSO-*d₆*) : 1,40 (3H, t, *J* = 7,5 Hz) ; 2,99 (2H, q, *J* = 7,5 Hz) ; 6,57 (1H, dd, *J*₁ = 3,0 Hz, *J*₂ = 2,0 Hz) ; 7,09 (1H, t, *J* = 7,5 Hz) ; 7,13-7,16 (1H, m) ; 7,33 (1H, dd, *J*₁ = 8,5 Hz, *J*₂ = 0,5 Hz) ; 7,40 (1H, dd, *J*₁ = 3,0 Hz, *J*₂ = 2,5 Hz) ; 7,69 (1H, d, *J*₁ = 8,5 Hz, *J*₂ = 0,5 Hz) ; 7,87 (1H, d, *J* = 7,5 Hz) ; 10,59 (1H, se, NH) ; 10,85 (1H, se, NH)
RMN ¹³C (100 MHz, DMSO-*d₆*) 14,2 (CH₃) ; 24,1 (CH₂) ; 102,8 ; 112,1 (2C) ; 116,9 ; 118,9 ; 122,2 ; 123,5 (CH) ; 116,5 ; 121,9 ; 123,8 ; 126,0 (2C) ; 126,3 ; 136,6 (C)

### EXEMPLE 34 : 9-(trifluorométhyl)-1,10-dihydropyrrolo[2,3-a]carbazole

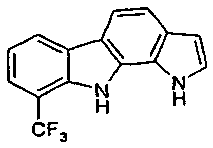

Stade A : chlorhydrate de 2-(trifluorométhyl)phénylhydrazine (1,00 g ; 4,70 mmol), acétate de sodium (386 mg ; 4,71 mmol) ; stade B : DDQ (636 mg ; 2,80 mmol) ; stade C : une chromatographie sur gel de silice (cyclohexane/AcOEt, de 9:1 à 7:3) permet d'isoler le composé attendu (550 mg ; 2,01 mmol ; R = 50%) sous la forme d'un solide beige.
Point de fusion > 250 °C
Spectre de masse haute résolution (ESI+) calculée pour C₁₅H₁₀F₃N₂ (M+H)⁺ 275,0796 ; trouvée 275,0781
IR(ATR) 3453, 3385, 1649 cm⁻¹
RMN ¹H (400 MHz, DMSO-*d₆*) : 6,62 (1H, dd, *J*₁ = 3,0 Hz, *J*₂ = 2,0 Hz) ; 7,32 (1H, t, *J* = 7,5 Hz) ; 7,44 (1H, d, *J* = 8,5 Hz) ; 7,47 (1H, t, *J* = 2,5 Hz) ; 7,63 (1H, d, *J* = 7,5 Hz) ; 7,80 (1H, d, *J* = 8,5 Hz) ; 8,37 (1H, d, *J* = 8,0 Hz) ; 10,86 (1H, se, NH) ; 11,34 (1H, se, NH)
RMN ¹³C (100 MHz, DMSO-*d₆*) 102,9 ; 111,9 ; 113,4 ; 118,3 ; 120,3 (q, *J*_{CF} = 4,5 Hz) ; 123,6 ; 124,3 (CH) ; 111,3 (q, *J*_{CF} = 32 Hz) ; 115,3 ; 121,5 ; 125,1 (q, *J*_{CF} = 271 Hz) ; 126,1 ; 126,9 ; 127,0 ; 133,2 (q, *J*_{CF} = 2 Hz) (C)

### EXEMPLE 35: 8-méthyl-1,10-dihydropyrrolo[2,3-a]carbazole

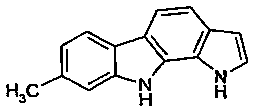

Stade A : un mélange de 3-méthylphénylhydrazine (1,00 g ; 8,2 mmol), du composé de la préparation B (1,65 g ; 6,0 mmol) et du liquide ionique de la préparation C (11,54 g ; 28 mmol) et agité à 120 °C pendant 12 heures. Après refroidissement, une solution aqueuse d'HCl 0,5 M est ajoutée avant extraction avec de l'acétate d'éthyle. Les fractions organiques rassemblées sont lavées avec une solution aqueuse saturée de NaCl, séchées sur MgSO₄ et filtrées. La solution contient le produit attendu et est utilisée directement pour l'étape suivante d'oxydation.

Les stades B and C sont réalisés de la même façon que pour les exemples 25-34.

Stade B : DDQ (953 mg ; 4,20 mmol) ; stade C : une chromatographie sur gel de silice (cyclohexane/AcOEt, de 9:1 à 7:3) permet d'isoler le composé attendu (860 mg ; 3,90 mmol ; R = 65%) sous la forme d'un solide gris.
Point de fusion > 250 °C
Spectre de masse (EI) *m*/*z:* 220, M⁺
IR(ATR): 3410, 3387, 1649, 1622 cm⁻¹
RMN ¹H (400 MHz, DMSO-*d₆*) : 2,48 (3H, s) ; 6,55 (1H, dd, *J*₁ = 3,0 Hz, *J*₂ = 2,0 Hz) ; 6,95-6,98 (1H, m) ; 7,30 (1H, d, *J* = 8,5 Hz) ; 7,35 (1H, t, *J* = 2,5 Hz) ; 7,39 (1H, m) ; 7,65(1H, d, *J* = 8,5Hz) ; 7,89(1H, d, *J* = 8,0Hz); 10,73 (1H, se, NH) ; 10,76 (1H, se, NH)
RMN ¹³C (100 MHz, DMSO-*d₆*) 21,6 (CH₃) ; 102,7 ; 111,2 ; 111,8 ; 111,9 ; 118,8 ; 120,1 ; 123,4 (CH) ; 116,2 ; 121,9 (2C) ; 125,9 ; 126,2 ; 132,6 ; 138,6 (C)

### EXEMPLE 36 : 7-(3-methoxyphényl)-1,10-dihydropyrrolo[2,3-a]carbazol-3-carbaldéhyde

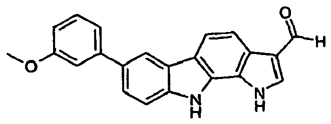

Du POCl₃ (90 µL, 0,97 mmol) est ajouté lentement à 0 °C à du DMF anhydre (2 mL). Le mélange est agité à 0-10 °C pendant 45 minutes jusqu'à ce qu'une solution jaune soit obtenue. Une solution du composé de l'exemple 18 (100 mg ; 0,320 mmol) dans le DMF (1 mL) est ensuite ajoutée. Le mélange est chauffé à 120 °C pendant 24 heures. Après refroidissement, une solution aqueuse de NaOH à 5% (20 mL) est ajoutée et le mélange est agité à température ambiante pendant une nuit. Après extraction avec de l'acétate d'éthyle, les fractions organiques rassemblées sont séchées sur MgSO₄, évaporées, puis le résidu est purifié par chromatographie sur gel de silice (pentane/AcOEt, de 7:3 à 1:9) ce qui permet d'obtenir le composé attendu (49 mg ; 0,144 mmol ; R = 45%) sous la forme d'un solide brun clair.
Point de fusion 215°C (décomposition)
Spectre de masse haute résolution (ESI+) calculée pour C₂₂H₁₇N₂O₂ (M+H)⁺ 341,1290 ; trouvée 341,1305
IR (KBr) : 3325, 1619 cm⁻¹
RMN ¹H (400 MHz, DMSO-*d*₆) : 3,87 (3H, s, CH₃) ; 6,91 (1H, d, *J* = 7,5 Hz) ; 7,31-7,42 (3H, m) ; 7,69 (1H, dd, *J*₁ = 8,5 Hz, *J*₂ = 1,5 Hz) ; 7,74 (1H, d, *J* = 8,5 Hz) ; 7,95 (1H, d, *J* = 8,5 Hz) ; 8,08 (1H, d, *J* = 8,5 Hz) ; 8,31 (1H, d, *J* = 3,0 Hz) ; 8,45 (1H, s) ; 10,04 (1H, s) ; 11,09-11,13 (1H, se, NH) ; 11,88-11,92 (1H, se, NH)
RMN ¹³C (100 MHz, DMSO-*d*₆): 55,1 (CH₃) ; 111,8 ; 112,0 ; 112,2 (2C) ; 115,3 ; 117,8 ; 119,1 ; 123,5 ; 129,8 ; 136,9 (CH) ; 118,6 ; 119,6 ; 122,8 (2C) ; 124,1 ; 126,5 ; 131,4 ; 138,2 ; 142,9 ; 159,7 (C) ; 185,3 (C=O)

### Procédure générale pour le préparation des composés des exemples 37-52

Du POCl₃ (3 équiv.) est ajouté lentement à 0 °C à du DMF anhydre (2 mL). La solution est agitée à 0-10 °C pendant 45 minutes jusqu'à ce qu'une solution jaune soit obtenue, puis est ajoutée à 0 °C à une solution des composés des exemples 19-35 (100 mg) dans le DMF (1 mL), préparée dans un tube CEM de 10 mL. Le tube est scellé et le mélange est chauffé à 100 °C sous irradiation micro-ondes pendant 20 minutes (150 W). Après refroidissement, le mélange est ajouté à une solution aqueuse saturée de NaHCO₃ (20 mL). Après 30 minutes d'agitation, le solide est filtré et une solution aqueuse de NaOH à 5% lui est ajoutée (20 mL). Le mélange est agité à température ambiante pendant une nuit. Le solide est filtré puis purifié par chromatographie sur gel de silice.

### EXEMPLE 37 : 7-(4-biphényl)-1,10-dihydropyrrolo[2,3-a]carbazol-3-carbaldéhyde

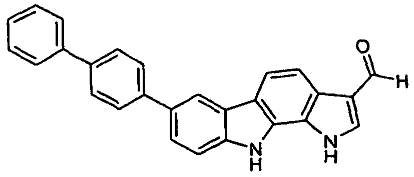

Une chromatographie sur gel de silice (pentane/AcOEt, de 7:3 à 1:9) permet d'isoler le composé attendu (32 mg ; 0,083 mmol ; R= 30%) sous la forme d'un solide brun foncé.
Point de fusion > 250 °C
Spectre de masse haute résolution (ESI+) calculée pour C₂₇H₁₉N₂O (M+H)⁺ 387,1497 ; trouvée 387,1492
IR (KBr): 3436, 1622 cm⁻¹
RMN ¹H (400 MHz, DMSO-*d*₆) : 7,36-7,41 (1H, m) ; 7,47-7,52 (2H, m) ; 7,73-7,81 (6H, m) ; 7,88-7,92 (2H, m) ; 7,96 (1H, d, *J* = 8,5 Hz) ; 8,09 (1H, d, *J* = 8,5 Hz) ; 8,31 (1H, d, *J* = 3,0 Hz) ; 8,51 (1H, s) ; 10,05 (1H, s) ; 11,12-11,16 (1H, se, NH) ; 11,90 (1H, de, *J* = 2,5 Hz, NH)
RMN ¹³C (100 MHz, DMSO-*d*₆) : 112,0 ; 112,3 ; 115,3 ; 117,5 ; 123,3 ; 126,5 (2C) ; 127,1 (4C) ; 127,3 ; 129,0 (2C) ; 136,9 (CH) ; 118,6 ; 119,6 ; 122,8 (2C) ; 124,2 ; 126,5 ; 130,9 ; 138,0; 138,2 ; 139,8 ; 140,4 (C) ; 185,3 (C=O)

### EXEMPLE 38 : 7-phényl-1,10-dihydropyrrolo[2,3-a]carbazol-3-carbaldéhyde

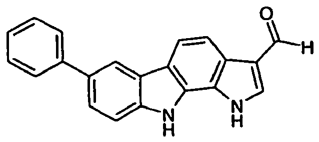

Une chromatographie sur gel de silice (pentane/AcOEt, de 7:3 à 1:9) permet d'isoler le composé attendu (57 mg ; 0,184 mmol ; R = 52%) sous la forme d'un solide brun foncé.
Point de fusion 205 °C (décomposition)
Spectre de masse haute résolution (ESI+) calculée pour C₂₁H₁₅N₂O (M+H)⁺ 311,1184 ; trouvée 311,1201
IR (KBr) : 3260, 1622 cm⁻¹
RMN ¹H (400 MHz, DMSO-*d*₆) : 7,31-7,36 (1H, m) ; 7,46-7,51 (2H, m) ; 7,68 (1H, dd, *J*₁ = 8,5 Hz, *J*₂ = 2,0 Hz) ; 7,75 (1H, dd, *J*₁ = 8,5 Hz, *J*₂ = 0,5 Hz) ; 7,77-7,81 (2H, m) ; 7,95 (1H, d, *J* = 8,5 Hz) ; 8,06 (1H, d, *J* = 8,5 Hz) ; 8,31 (1H, d, *J* = 3,0 Hz ) ; 8,43 (1H, d, *J* = 2,0 Hz) ; 10,04 (1H, s) ; 11,08-11,14 (1H, se, NH) ; 11,86-11,93 (1H, se, NH)
RMN ¹³C (100 MHz, DMSO-*d*₆): 111,9 ; 112,3 ; 115,3 ; 117,7 ; 123,4 ; 126,4 ; . 126,7 (2C) ; 128,8 (2C) ; 136,9 (CH) ; 118,6 ; 119,6 ; 122,8 (2C) ; 124,2 ; 126,5 ; 131,5 ; 138,1 ; 141,4 (C) ; 185,4 (C=O)

### EXEMPLE 39 : 7-(4-fluorophényl)-1,10-dihydropyrrolo[2,3-a]carbazol-3-carbaldéhyde

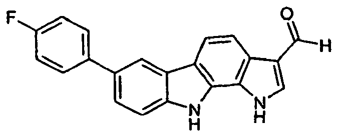

Une chromatographie sur gel de silice (pentane/AcOEt, de 7:3 à 1:9) permet d'isoler le composé attendu (55 mg ; 0,168 mmol ; R = 50%) sous la forme d'un solide brun clair.
Point de fusion > 250 °C
Spectre de masse haute résolution (ESI+) calculée pour C₂₁H₁₄N₂OF (M+H)⁺ 329,1090 ; trouvée 329,1101
IR (KBr) : 3436, 3297, 1619 cm⁻¹.
RMN ¹H (400 MHz, DMSO-*d*₆) : 7,27-7,34 (2H, m) ; 7,65 (1H, dd, *J*₁ = 8,5 Hz, *J*₂ = 2,0 Hz) ; 7,74 (1H, d, *J* = 8,5 Hz) ; 7,78-7,84 (2H, m) ; 7,95 (1H, d, *J* = 8,5 Hz) ; 8,06 (1H, d, *J* = 8,5 Hz) ; 8,31 (1H, s) ; 8,41 (1H, d, *J* = 1,5 Hz) ; 10,04 (1H, s) ; 11,10-11,15 (1H, se, NH) ; 11,88-11,95 (1H, se, NH)
RMN ¹³C (100 MHz, DMSO-d₆) : 112,0 ; 112,3 ; 115,3 ; 115,6 (2C, d, *J*_{CF} = 21 Hz) ; 117,7 ; 123,4 ; 128,5 (2C, d, *J*_{CF} = 8 Hz) ; 136,9 (CH) ; 118,6 ; 119,6 ; 122,8 (2C) ; 124,2 ; 126,6 ; 130,6 ; 137,9 (d, *J*_{CF} = 3 Hz) ; 138,1 ; 161,3 (d, *J*_{CF} = 243 Hz) ; 185,4 (C=O)

### EXEMPLE 40 : 7-(2,4-difluorophényl)-1,10-dihydropyrrolo[2,3-a]carbazol-3-carbaldéhyde

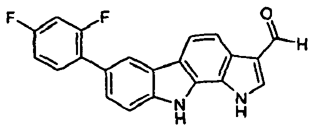

Une chromatographie sur gel de silice (pentane/AcOEt, de 7:3 à 1:9) permet d'isoler le composé attendu (48 mg ; 0,139 mmol ; R = 44%) sous la forme d'un solide beige.
Point de fusion 220 °C (décomposition)
Spectre de masse haute résolution (ESI+) calculée pour C₂₁H₁₃F₂N₂O (M+H)⁺ 347,0996 ; trouvée 347,0996
IR (KBr) : 3264, 1645, 1616 cm⁻¹
RMN ¹H (400 MHz, DMSO-*d*₆) : 7,22 (1H, td, *J*₁ = 8,5 Hz, *J*₂ = 2,5 Hz) ; 7,37 (1H, ddd, *J*₁ = 11,5 Hz, *J*₂ = 9,5 Hz, *J*₃ = 2,5 Hz) ; 7,52 (1H, dt, *J*₁ = 8,5 Hz, *J*₂ = 2,0 Hz) ; 7,69 (1H, td, *J*₁ = 9,0 Hz, *J*₂ = 7,0 Hz) ; 7,76 (1H, d, *J* = 8,5 Hz) ; 7,95 (1H, d, *J* = 8,5 Hz) ; 8,02 (1H, d, *J* = 8,5 Hz) ; 8,27 (1H, s) ; 8,31 (1H, s) ; 10,04 (1H, s) ; 11,19-11,26 (1H, se, NH) ; 11,90-12,01 (1H, se, NH)
RMN ¹³C (100 MHz, DMSO-*d*₆) : 104,3 (dd, *J*_{CF1} = 27 Hz, *J*_{CF2} = 26 Hz) ; 111,6 ; 111,8 (dd, *J*_{CF1} = 21 Hz, *J*_{CF2} = 4 Hz) ; 112,4 ; 115,2 ; 119,9 (d, *J*_{CF} = 2 Hz) ; 125,2 ; 132,1 (dd, *J*_{CF1} = 9 Hz, *J*_{CF2} = 5 Hz) ; 136,9 (CH) ; 118,3 ; 119,6 ; 122,8 (2C) ; 123,8 ; 125,2 ; 126,1 (dd, *J*_{CF1} = 14 Hz, *J*_{CF2} = 4 Hz) ; 126,5 ; 138,1 ; 159,1 (dd, *J*_{CF1} = 247 Hz, *J*_{CF2} = 12 Hz) ; 161,1 (dd, *J*_{CF1} = 246 Hz, *J*_{CF2} = 12 Hz (C) ; 185,3 (C=O)

### EXEMPLE 41 : 7-(4-trifluorométhylphényl)-1,10-dihydropyrrolo[2,3-a]carbazol-3-carbaldéhyde

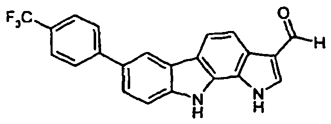

Une chromatographie sur gel de silice (pentane/AcOEt, de 7:3 à 1:9) permet d'isoler le composé attendu (70 mg ; 0,185 mmol ; R = 65%) sous la forme d'un solide brun clair.
Point de fusion > 250 °C
Spectre de masse haute résolution (ESI+) calculée pour C₂₂H₁₄F₃N₂O (M+H)⁺ 379,1058 ; trouvée 379,1059
IR (KBr) : 3460, 3294, 1617 cm⁻¹
RMN ¹H (400 MHz, DMSO-*d*₆) 7,76 (1H, dd, *J*₁ = 8,5 Hz, *J*₂ = 1,5 Hz) ; 7,80 (1H, dd, *J*₁ = 8,5 Hz, *J*₂ = 1,0 Hz) ; 7,83 (2H, d, *J* = 8,0 Hz) ; 7,97 (1H, d, *J* = 8,5 Hz) ; 8,03 (2H, d, *J* = 8,0 Hz) ; 8,09 (1H, d, *J* = 8,5 Hz) ; 8,32 (1H, s) ; 8,56 (1H, s) ; 10,04 (1H, s) ; 11,18-11,25 (1H, se, NH) ; 11,88-11,97 (1H, se, NH)
RMN ¹³C (100 MHz, DMSO-*d*₆) : 112,2 ; 112,5 ; 115,3 ; 118,2 ; 123,5 ; 125,7 (2C, q, *J*_{CF} = 4 Hz) ; 127,3 (2C) ; 137,0 (CH) ; 118,5 ; 119,6 ; 122,8 ; 122,9 ; 124,3 ; 124,6 (q, *J*_{CF} = 272 Hz) ; 126,6 ; 126,7 (q, *J*_{CF} = 32 Hz) ; 129,7 ; 138,7 ; 145,4 (C) ; 185,4 (C=O)

### EXEMPLE 42 : 7-(4-trifluoromethoxyphényl)-1,10-dihydropyrrolo[2,3-a]carbazol-3-carbaldéhyde

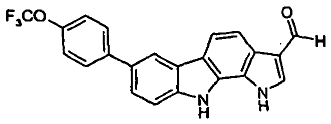

Une chromatographie sur gel de silice (pentane/AcOEt, de 7:3 à 1:9) permet d'isoler le composé attendu (72 mg ; 0,183 mmol ; R = 67%) sous la forme d'un solide beige.
Point de fusion > 250 °C
Spectre de masse haute résolution (ESI+) calculée pour C₂₂H₁₄F₃N₂O₂ (M+H)⁺ 395,1007 ; trouvée 395,1017
IR (KBr) : 3276, 1620 cm⁻¹
RMN ¹H (500 MHz, DMSO-*d*₆) : 7,46 (2H, d, *J* = 8,5 Hz) ; 7,69 (1H, dd, *J*₁ = 8,5 Hz, *J*₂ = 2,0 Hz) ; 7,75 (1H, d, *J* = 8,5 Hz) ; 7,88-7,92 (2H, m) ; 7,95 (1H, d, *J* = 8,5 Hz) ; 8,06 (1H, d, *J* = 8,5 Hz) ; 8,31 (1H, d, *J* = 3,0 Hz) ; 8,46 (1H, d, *J* = 2,0 Hz) ; 10,03 (1H, s) ; 11,15-11,18 (1H, se, NH) ; 11,93 (1H, de, *J* = 2,5 Hz, NH)
RMN ¹³C (125 MHz, DMSO-*d*₆) : 112,1 ; 112,5 ; 115,4 ; 118,0 ; 121,5 (2C) ; 123,6 ; 128,5 (2C) ; 137,0 (CH) ; 118,6 ; 119,7 ; 120,3 (q, *J*_{CF} = 256 Hz) ; 122,9 ; 123,0 ; 124,3 ; 126,7 ; 130,1 ; 138,4 ; 140,8 ; 147,2 (q, *J*_{CF} = 2 Hz) (C) ; 185,5 (C=O)

### EXEMPLE 43 : 8-bromo-1,10-dihydropyrrolo[2,3-a]carbazol-3-carbaldéhyde

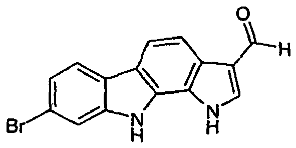

Une chromatographie sur gel de silice (cyclohexane/AcOEt, de 7:3 à 1:9) permet d'isoler le composé attendu (66 mg ; 0,21 mmol ; R = 60%) sous la forme d'un solide brun.
Point de fusion > 250 °C
Spectre de masse (EI) *m*/*z* : 312/314, M⁺
IR (ATR) : 3393, 3330-3160, 1624 cm⁻¹
RMN ¹H (400 MHz, DMSO-*d*₆) : 7,33 (1H, dd, *J*₁ = 8,5 Hz, *J*₂ = 2,0 Hz) ; 7,93 (1H, d, *J* = 1,5 Hz) ; 7,94 (1H, d, *J* = 8,5 Hz) ; 7,97 (1H, d, *J* = 8,5 Hz) ; 8,07 (1H, d, *J* = 8,5 Hz) ; 8,31 (1H, d, *J* = 3,0 Hz) ; 10,03 (1H, s, CHO) ; 11,13 (1H, se, NH) ; 11,96 (1H, se, NH)
RMN ¹³C (100 MHz, DMSO-*d*₆) : 112,7 ; 114,3 ; 115,1 ; 121,2 ; 121,8 ; 137,0 (CH) ; 116,8 ; 117,8 ; 119,5 ; 122,6 ; 122,7 ; 123,0 ; 126,3 ; 139,4 (C) ; 185,4 (C=O)

### EXEMPLE 44 : 6-bromo-1,10-dihydropyrrolo[2,3-a]carbazol-3-carbaldéhyde

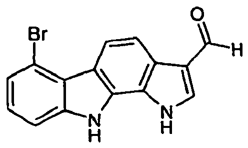

Une chromatographie sur gel de silice (cyclohexane/AcOEt, de 7:3 à 1:9) permet d'isoler le composé attendu (55 mg ; 0,176 mmol ; R= 50%) sous la forme d'un solide brun.
Point de fusion > 250 °C
Spectre de masse (EI) *m*/*z:* 312/314, M⁺
IR (ATR) : 3300, 1620 cm⁻¹
RMN ¹H (400 MHz, DMSO-*d*₆) : 7,30 (1H, t, *J* = 8,0 Hz) ; 7,41 (1H, d, *J* = 7,5 Hz) ; 7,73 (1H, d, *J* = 8,0 Hz) ; 7,99 (1H, d, *J* = 8,5 Hz) ; 8,34 (1H, d, *J* = 3,0 Hz) ; 8,46 (1H, d, *J* = 8,5 Hz) ; 10,05 (1H, s, CHO) ; 11,46 (1H, se, NH) ; 11,91 (1H, se, NH)
RMN ¹³C (100 MHz, DMSO-*d*₆) : 111,0 ; 112,3 ; 116,3 ; 123,0 ; 125,2 ; 137,3 (CH) ; 114,6 ; 117,5 ; 119,5 ; 121,8 ; 122,4 ; 122,9 ; 126,6 ; 139,8 (C) ; 185,4 (C=O)

### EXEMPLE 45 : 9-bromo-1,10-dihydropyrrolo[2,3-a]carbazol-3-carbaldéhyde

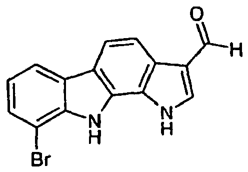

Une chromatographie sur gel de silice (cyclohexane/AcOEt, de 7:3 à 1:9) permet d'isoler le composé attendu (90 mg ; 0,287 mmol ; R = 82%) sous la forme d'un solide brun.
Point de fusion > 250 °C
Spectre de masse haute résolution (ESI+) calculée pour C₁₅H₁₀⁷⁹ BrN₂O (M+H)⁺ 312,9976 ; trouvée 312,9991
IR (ATR) : 3400-3100, 1630 cm⁻¹
RMN ¹H (400 MHz, DMSO-*d*₆) : 7,16 (1H, t, *J* = 7,5 Hz) ; 7,59 (1H, dd, *J₁* = 7,5 Hz, *J*₂ = 1,0 Hz) ; 7,98 (2H, s) ; 8,15 (1H, d, *J* = 7,5 Hz) ; 8,34 (1H, d, *J* = 3,0 Hz) ; 10,05 (1H, s, CHO) ; 11,27 (1H, se, NH) ; 11,69 (1H, se, NH)
RMN ¹³C (100 MHz, DMSO-*d*₆) : 113,1 ; 115,4 ; 119,1 ; 120,6 ; 126,5 ; 137,1 (CH) ; 103,8 ; 118,5 ; 119,5 ; 122,7 ; 123,0 ; 125,2 ; 126,0 ; 136,7 (C) ; 185,5 (C=O)

### EXEMPLE 46 : 7-Fluoro-1,10-dihydropyrrolo[2,3-a]carbazol-3-carbaldéhyde

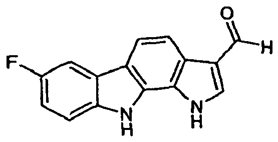

Une chromatographie sur gel de silice (cyclohexane/AcOEt, de 3:7 à 1:9) permet d'isoler le composé attendu (96 mg ; 0,381 mmol ; R = 85%) sous la forme d'un solide brun.
Point de fusion > 250 °C
Spectre de masse haute résolution (ESI+) calculée pour C₁₅H₁₀FN₂O (M+H)⁺ 253,0777 ; trouvée 253,0773
IR (ATR) : 3314, 1636, 1622 cm⁻¹
RMN ¹H (400 MHz, DMSO-*d*₆) : 7,20 (1H, ddd, *J*₁ = 9,5 Hz, *J*₂ = 9,0 Hz, *J*₃ = 2,5 Hz) ; 7,67 (1H, dd, *J*₁ = 9,0 Hz, *J*₂ = 4,5 Hz) ; 7,91 (1H, d, *J* = 8,5 Hz) ; 7,92-7,95 (1H, m) ; 7,96 (1H, d, *J* = 8.5 Hz) ; 8,30 (1H, d, *J* = 3,0 Hz) ; 10,03 (1H, s, CHO) ; 11,07 (1H, se, NH) ; 11,92 (1H, se, NH)
RMN ¹³C (100 MHz, DMSO-*d*₆) : 105,0 (d, *J*_{CF} = 24 Hz) ; 111,8 (d, *J*_{CF} = 25 Hz) ; 112,2; 112,4 (d, *J*_{CF} = 9 Hz) ; 115,4; 136,8 (CH) ; 118,2 (d, *J*_{CF} = 4 Hz); 119,6 ; 122,7 ; 122,9 ; 124,1 (d, *J*_{CF} = 10 Hz) ; 127,3 ; 135,0 ; 156,8 (d, *J*_{CF} = 232 Hz) (C) ; 185,4 (C=O)

### EXEMPLE 47 : 9-Fluoro-1,10-dihydropyrrolo[2,3-a]carbazol-3-carbaldéhyde

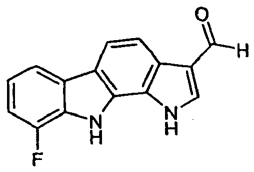

Une chromatographie sur gel de silice (cyclohexane/AcOEt, de 8:2 à 3:7) permet d'isoler le composé attendu (80 mg ; 0,317 mmol ; R = 71%) sous la forme d'un solide brun.
Point de fusion > 250 °C
Spectre de masse haute résolution (ESI+) calculée pour C₁₅H₁₀FN₂O (M+H)⁺ 253,0777 ; trouvée 253,0792
IR (ATR) : 3450-3120, 1624 cm⁻¹
RMN ¹H (400 MHz, DMSO-*d*₆) : 7,17 (1H, dt, *J*₁ = 5,0 Hz, *J*₂ = 8,0 Hz) ; 7,24 (1H, ddd, *J*₁ = 11,0 Hz, *J*₂ = 8,0 Hz, *J*₃ = 1,0 Hz) ; 7,94-7,97 (2H, m) ; 7,98 (1H, d, *J* = 8,5 Hz) ; 8,31 (1H, d, *J* = 3,0 Hz) ; 10,05 (1H, s, CHO) ; 11,52 (1H, se, NH) ; 11,57 (1H, se, NH)
RMN ¹³C (100 MHz, DMSO-*d*₆): 109,4 (d, *J*_{CF} = 16 Hz) ; 112,9 ; 115,2 ; 115,8 (d, *J*_{CF} = 3 Hz) ; 119,5 (d, *J*_{CF} = 6 Hz) ; 137,1 (CH) ; 118,3 (d, *J*_{CF} = 2,5 Hz) ; 119,5 ; 122,8 ; 123,0 ; 125,9 (d, *J*_{CF} = 13 Hz) ; 126,4 ; 127,4 (d, *J*_{CF} = 6 Hz) ; 148,9 (d, *J*_{CF} = 241 Hz) (C) ; 185,5 (C=O)

### EXEMPLE 48: 6,8-dichloro-1,10-dihydropyrrolo[2,3-a]carbazol-3-carbaldéhyde

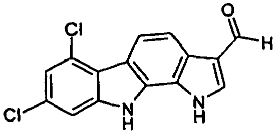

Une chromatographie sur gel de silice (cyclohexane/AcOEt, de 3:7 à 1:9) permet d'isoler le composé attendu (52 mg ; 0,172 mmol ; R = 47%) sous la forme d'un solide brun.
Point de fusion > 250 °C
Spectre de masse haute résolution (ESI+) calculée pour C₁₅H₉³⁵Cl₂N₂O (M+H)⁺ 303,0092 ; trouvée 303,0073
IR (ATR) : 3352, 1717, 1630 cm⁻¹
RMN ¹H (400 MHz, DMSO-*d*₆) : 7,34 (1H, d, *J* = 1.,5 Hz) ; 7,84 (1H, d, *J* = 1,5 Hz) ; 8,01 (1H, d, *J* = 8,5 Hz) ; 8,27 (1H, d, *J* = 8,5 Hz) ; 8,36 (1H, d, *J* = 3,0 Hz) ; 10,05 (1H, s, CHO) ; 11,54 (1H, se, NH) ; 11,99 (1H, se, NH).
RMN ¹³C (100 MHz, DMSO-*d*₆) : 110,5 ; 113,2 ; 116,4 ; 119,3 ; 137,4 (CH) ; 116,5 ; 119,4 ; 119,5 ; 122,3 ; 123,2 ; 126,7 ; 126,9 ; 128,4 ; 139,8 (C) ; 185,4 (C=O).

### EXEMPLE 49 : 3-formyl-1,10-dihydropyrrolo[2,3-a]carbazol-7-carbonitrile

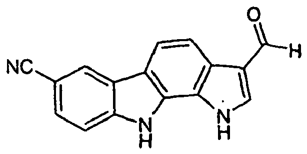

Une chromatographie sur gel de silice (cyclohexane/AcOEt, de 3:7 à 1:9) permet d'isoler le composé attendu (58,3 mg ; 0,225 mmol ; R = 52%) sous la forme d'un solide brun.
Point de fusion > 250 °C
Spectre de masse haute résolution (ESI+) calculée pour C₁₆H₁₀N₃O (M+H)⁺ 260,0824 ; trouvée 260,0833
IR (ATR) : 3287, 3239, 2212, 1630, 1618 cm⁻¹
RMN ¹H (400 MHz, DMSO-d₆) : 7,74 (1H, dd, *J*₁ = 8,5 Hz, *J*₂ = 1,5 Hz) ; 7,85 (1H, dd, *J*₁ = 8,5 Hz, *J*₂ = 0,5 Hz) ; 8,01 (1H, d, *J* = 8,5 Hz) ; 8,08 (1H, d, *J* = 8,5 Hz) ; 8,35 (1H, d, *J* = 3,0 Hz) ; 8,70 (1H, d, *J* = 1,5 Hz) ; 10,05 (1H, s, CHO) ; 11,65 (1H, se, NH) ; 11,97 (1H, se, NH)
RMN ¹³C (100 MHz, DMSO-*d*₆) : 112,7 ; 113,4 ; 115,4 ; 124,8 ; 127,3 ; 137,3 (CH) ; 100,8 ; 117,6 ; 119,5 ; 120,6 ; 122,5 ; 123,6 ; 123,7 ; 126,8 ; 140,5 (C) ; 185,5 (C=O)

### EXEMPLE 50 : 7-Nitro-1,10-dihydropyrrolo[2,3-a]carbazol-3-carbaldéhyde

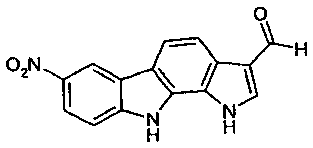

Une chromatographie sur gel de silice (cyclohexane/AcOEt, de 3:7 à 1:9) permet d'isoler le composé attendu (39 mg ; 0,14 mmol ; R = 35%) sous la forme d'un solide brun-jaune.
Point de fusion > 250 °C
Spectre de masse haute résolution (ESI+) calculée pour C₁₅H₁₀N₃O₃ (M+H)⁺ 280,0722 ; trouvée 280,0714
IR (ATR) : 3350-3150, 1636, 1614 cm⁻¹
RMN ¹H (400 MHz, DMSO-d₆) : 7,85 (1H, d, J = 9,0 Hz) ; 8,04 (1H, d, J = 8,5 Hz) ; 8,19 (1H, d, J = 8,5 Hz) ; 8,28 (1H, dd, J₁ = 9,0 Hz, J₂ = 2,5 Hz) ; 8,37 (1H, d, J = 2,5 Hz) ; 9,14 (1H, d, *J* = 2,0 Hz) ; 10,06 (1H, s, CHO) ; 11,87 (1H, se, NH) ; 12,03 (1H, se, NH)
RMN ¹³C (100 MHz, DMSO-d₆) : 111,8 ; 113,8 ; 115,7 ; 116,5 ; 119,9 ; 137,5 (CH) ; 118,4 119,5 ; 122,6 ; 123,3 ; 123,9 ; 127,5 ; 140,3 ; 142,0 (C) ; 185,5 (C=O)

### EXEMPLE 51 : 9-éthyl-1,10-dihydropyrrolo[2,3-a]carbazol-3-carbaldéhyde

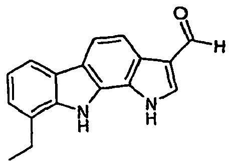

Une chromatographie sur gel de silice (cyclohexane/AcOEt, de 8:2 à 3:7) permet d'isoler le composé attendu (56 mg ; 0,213 mmol ; R = 50%) sous la forme d'un solide brun.
Point de fusion > 250 °C
Spectre de masse haute résolution (ESI+) calculée pour C₁₇H₁₅N₂O (M+H)⁺ 263,1184; trouvée 263,1169
IR (ATR) : 3450-3150, 1626 cm⁻¹
RMN ¹H (400 MHz, DMSO-*d₆*): 1,40 (3H, t, *J* = 7,5 Hz) ; 3,00 (2H, q, *J* = 7,5 Hz) ; 7,14 (1H, t, *J* = 7,5 Hz) ; 7,20-7,23 (1H, m) ; 7,90 (1H, d, *J* = 8,5 Hz) ; 7,93 (1H, d, *J* = 8,0 Hz) ; 7,93-7,96 (1H, m) ; 8,29 (1H, d, *J* = 3,0 Hz) ; 10,03 (1H, s, CHO) ; 11,00 (1H, se, NH) ; 11,54 (1H, se, NH)
RMN ¹³C (100 MHz, DMSO-*d₆*) : 14,1 (CH₃) ; 24,0 (CH₂) ; 112,1 ; 115,1 ; 117,3 ; 119,3 ; 123,1 ; 136,7 (CH) ; 118,7 ; 119,5 ; 122,3 ; 122,9 ; 123,2 ; 125,9 ; 126,2 ; 137,0 (C) ; 185,4 (C=O)

### EXEMPLE 52 : 8-méthyl-1,10-dihydropyrrolo[2,3-a]carbazol-3-carbaldéhyde

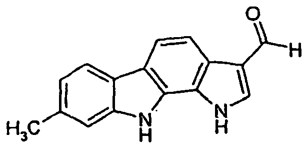

Une chromatographie sur gel de silice (cyclohexane/AcOEt, de 7:3 à 1:9) permet d'isoler le composé attendu (65 mg ; 0,26 mmol ; R = 58%) sous la forme d'un solide brun.
Point de fusion : 250 °C (décomposition)
Spectre de masse haute résolution (ESI+) calculée pour C₁₆H₁₃N₂O (M+H)⁺ 249,1028 ; trouvée 249,1026
IR (ATR) : 3379, 3341, 1634 cm⁻¹
RMN ¹H (400 MHz, DMSO-*d*₆): 2,49 (3H, s) ; 7,02 (1H, d, *J* = 8,0 Hz) ; 7,46 (1H, s) ; 7,89 (2H, s) ; 7,97 (1H, d, *J* = 8,0 Hz) ; 8,27 (1H, d, *J* = 3,0 Hz) ; 10,02 (1H, s, CHO) ; 11,88 (1H, se, NH) ; 11,81 (1H, se, NH)
RMN ¹³C (100 MHz, DMSO-*d*₆) : 21,6 (CH₃) ; 111,5 ; 112,0 ; 114,8 ; 119,2 ; 120,6 ; 136,8 (CH) ; 118,5 ; 119,5 ; 121,3 ; 122,2 ; 122,8 ; 125,8 ; 133,7 ; 139,0 (C) ; 185,3 (C=O)

### II) Test de l'activité des composés des exemples 2, 3 et 5 sur 67 kinases

Les composés de l'exemple 2, c'est-à-dire le 1,10-dihydropyrrolo[2,3-a]carbazole, de l'exemple 3, c'est-à-dire le 1,10-dihydropyrrolo[2,3-a]carbazol-3-carbaldéhyde ainsi que le composé de l'exemple 5, c'est-à-dire le 1,10-dihydropyrrolo[2,3-a]carbazol-3-carboxamide ont été testés sur les 67 kinases suivantes :
MKK1, ERK 1, ERK 2, JNK1, JNK2, JNK3, p38a MAPK, p38b MAPK, p38g MAPK, p38s MAPK, ERK8, RSK1, RSK2, PDK1, PKBa, PKBb, SGK1, S6K1, PKA, ROCK2, PRK2, PKCa, PKC zeta, PKD1, MSK1, MNK1, MNK2, MAPKAP-K2, MAPKAP-K3, PRAK, CAMKKa, CAMKKb, CAMK1, SmMLCK, PHK, CHK1, CHK2, GSK3b, CDK2/cyclin A , PLK1, PLK1(Okadaic acid), Aurora B, Aurora C, AMPK, MARK3, BRSK2, MELK, CK1, CK2, NEK2a, NEK6, NEK7, IKKb, PIM-1, PIM-2, PIM-3, SRPK1, MST2, EF2K, HIPK2, HIPK3, PAK4, PAK5, PAK6, Src, Lck, CSK.

Les résultats en termes d'activité résiduelle des kinases après mise en contact avec les composés des exemples 2, 3 et 5, à une concentration de 10µM (10µmoles/L), sont montrés au tableau 1 suivant.

Plus l'activité résiduelle de la kinase testée est élevée, moins l'activité d'inhibition des composés testés sur la kinase testée est élevée.

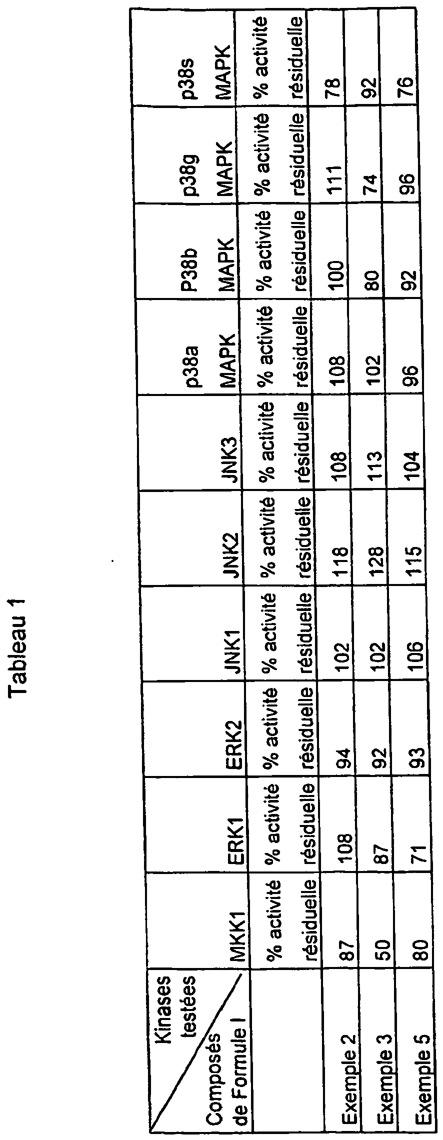

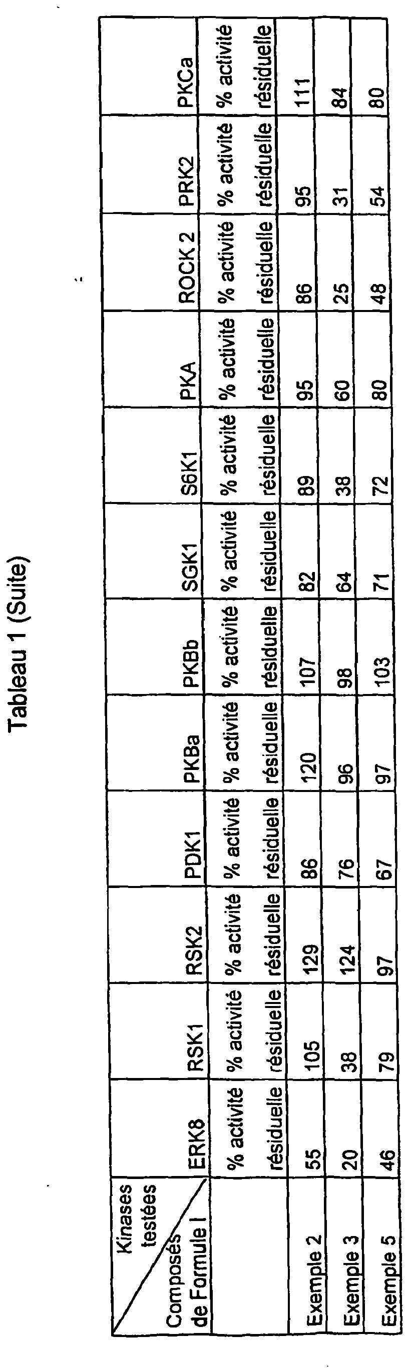

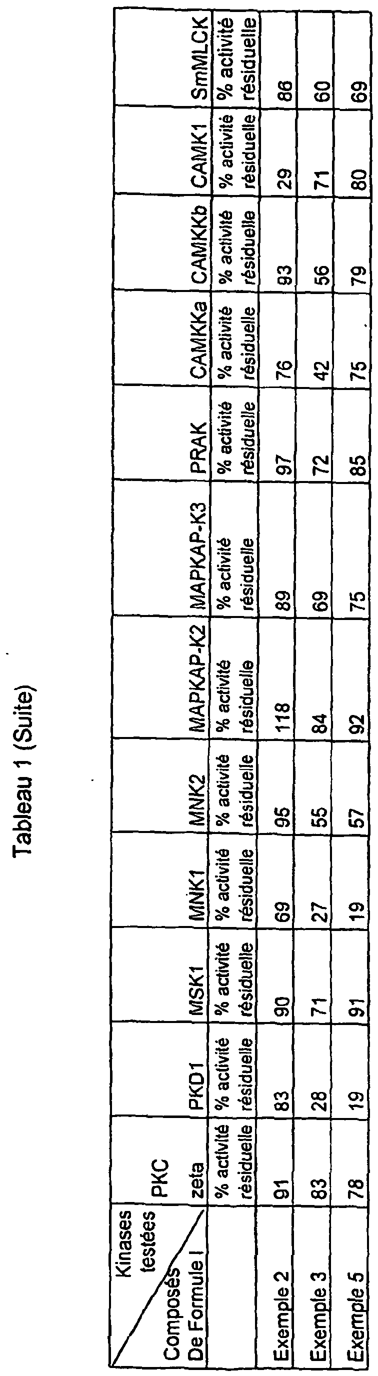

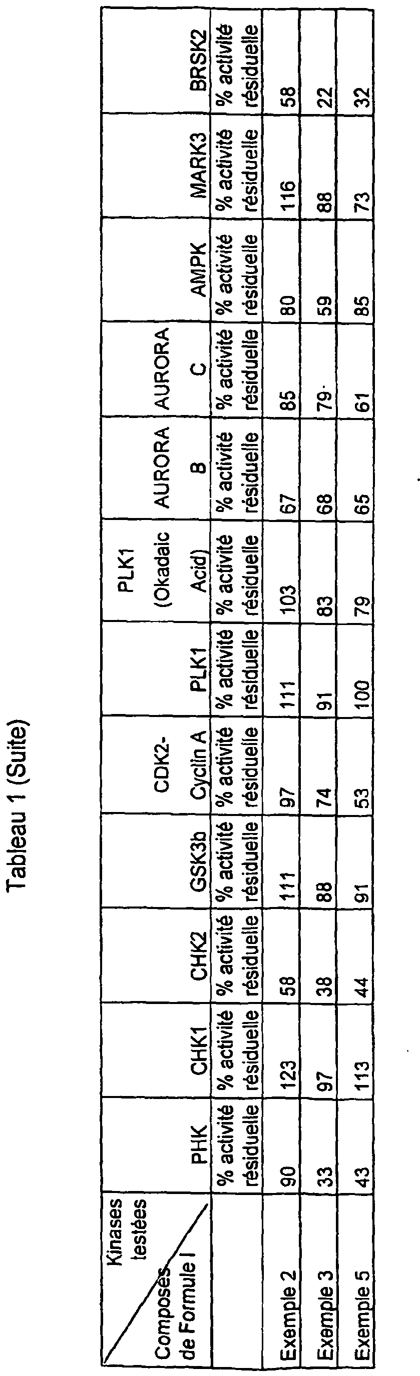

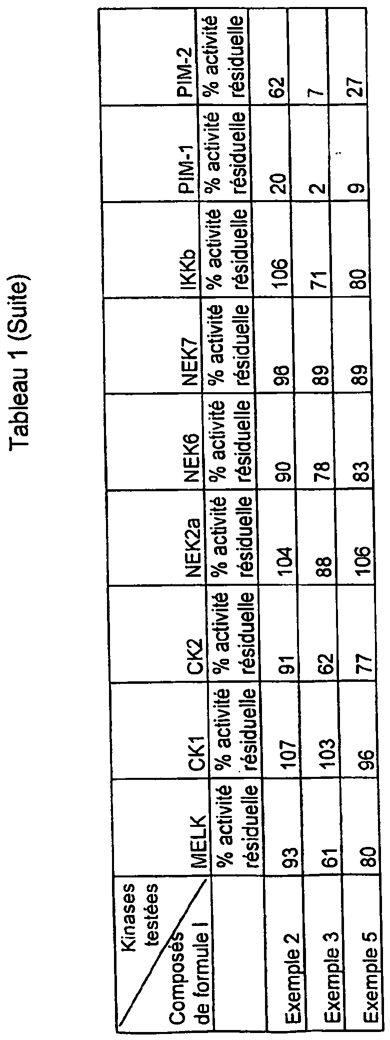

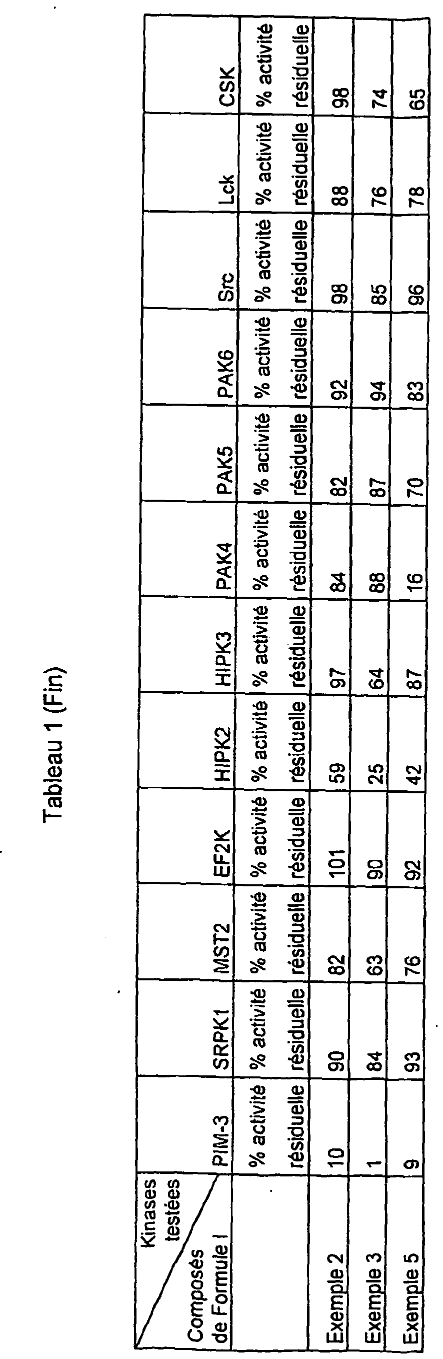

### III) Test de l'activité des composés de l'invention sur les kinases PIM-1, PIM-2 ou PIM-3

Les composés selon l'invention ont ensuite été testés sur les kinases PIM-1, PIM-2 ou PIM-3.

Les résultats en termes de pourcentage d'activité résiduelle après mise en contact des composés selon l'invention avec chacune des kinases PIM-1, PIM-2 ou PIM-3 sont reportés au tableau 2 suivant.

Plus l'activité résiduelle est élevée, moins l'activité d'inhibition des composés testés est élevée.

**Tableau 2**

| | | | |
|---|---|---|---|
| Pourcentage d'activité résiduelle à une concentration en produit selon l'invention de 10 micromoles/L sur les kinases PIM-1, PIM-2 ou PIM-3. | | | |

| Composés | PIM-1 | PIM-2 | PIM-3 |
|---|---|---|---|
| Exemple 1 | 61 | 62 | 64 |
| Exemple 2 | 20 | 62 | 10 |
| Exemple 3 | 2 | 7 | 1 |
| Exemple 4 | 28 | 47 | 18 |
| Exemple 5 | 9 | 27 | 9 |
| Exemple 6 | 20 | 30 | 7 |
| Exemple 7 | | N.D | |
| Exemple 8 | 44 | 47 | 33 |
| Exemple 9 | 35 | 50 | 21 |
| Exemple 10 | 54 | 51 | 39 |
| Exemple 11 | 39 | 78 | 25 |
| Exemple 12 | 24 | 53 | 16 |
| Exemple 13 | 41 | 27 | 21 |
| Exemple 14 | 51 | 52 | 61 |
| Exemple 15 | 12 | 46 | 8 |
| Exemple 16 | 6 | 57 | 6 |
| Exemple 17 | | N.D | |
| Exemple 18 | 46 | 51 | 39 |
| Exemple 19 | | N.D | |
| Exemple 20 | | N.D | |
| Exemple 21 | | N.D | |
| Exemple 22 | | N.D | |
| Exemple 23 | | N.D | |
| Exemple 24 | | N.D | |
| Exemple 25 | 74 | 93 | 15 |
| Exemple 26 | 8 | 37 | 11 |
| Exemple 27 | 90 | N.I | 43 |
| Exemple 28 | 35 | 84 | 20 |
| Exemple 29 | N.I | N.I | 43 |
| Exemple 30 | 10 | 36 | 14 |
| Exemple 31 | 9 | 26 | 4 |
| Exemple 32 | 9 | 23 | 11 |
| Exemple 33 | 28 | 96 | 23 |
| Exemple 34 | 85 | N.I | 45 |
| Exemple 35 | 81 | N.I | 13 |
| Exemple 36 | 17 | 31 | 12 |
| Exemple 37 | 39 | 70 | 48 |
| Exemple 38 | 16 | 53 | 15 |
| Exemple 39 | 15 | 55 | 15 |
| Exemple 40 | 6 | 32 | 6 |
| Exemple 41 | 28 | 52 | 23 |
| Exemple 42 | 29 | 70 | 31 |
| Exemple 43 | 44 | 39 | 15 |
| Exemple 44 | 2 | 6 | 5 |
| Exemple 45 | 3 | 28 | 9 |
| Exemple 46 | 4 | 21 | 5 |
| Exemple 47 | 5 | 11 | 3 |
| Exemple 48 | 8 | 34 | 6 |
| Exemple 49 | 92 | N.I | 14 |
| Exemple 50 | N.I | N.I | 35 |
| Exemple 51 | 1 | 14 | 10 |
| Exemple 52 | 15 | 39 | 8 |

| | | | |
|---|---|---|---|
| N.D : non déterminé N.I : Non inhibiteur. | | | |

Les composés préférés de l'invention sont ceux qui ont un pourcentage d'activité résiduelle inférieur ou égal à 10%, c'est-à-dire les composés des exemples 2, 3, 5, 6, 15, 16, 40, 44, 45, 46, 47, 48, 51 et 52.

### IV) Détermination de la concentration inhibitrice moyenne des composés préférés de l'invention.

Les concentrations inhibitrices moyennes (Cl₅₀) de certains composés préférés de l'invention ont été déterminées de la façon suivante : les Cl₅₀ ont été mesurées après avoir effectué les tests d'inhibition des kinases PIM-1, PIM-2 ou PIM-3 à 10 concentrations différentes pour chaque composé testé et la Cl₅₀ a été déterminée à partir des courbes dose-inhibition obtenues.

Les résultats sont reportés au tableau 3 suivant.

**Tableau 3**

| | | | |
|---|---|---|---|
| Cl₅₀ (micromoles/L) sur les kinases PIM-1, PIM-2 ou PIM-3. | | | |

| | PIM-1 | PIM-2 | PIM-3 |
|---|---|---|---|
| Composé exemple 3 | 0.12 | 0.51 | 0.01 |
| Composé exemple 5 | 0.78 | N.D | 0.21 |
| Composé exemple 6 | N.D | N.D | 0.17 |
| Composé exemple 15 | N.D | N.D | 0.44 |
| Composé exemple 16 | 0.57 | N.D | 0.04 |
| Composé exemple 40 | 0.66 | N.D | 0.20 |

| | | | |
|---|---|---|---|
| N.D : non déterminé | | | |

## Revendications

1. Utilisation d'au moins un composé de formule I suivante: 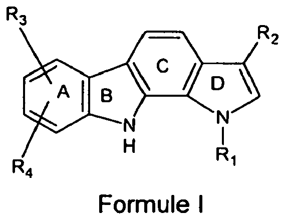 dans laquelle
- R₁ est H ou un groupe sulfophényle,
- R₂, R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocycloalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ ou -S(O)ₜ-Rₐ, -S(O)ₜ-ORₐ, -S(O)ₜ-NRₐR_{b}, -P(O)ₜ-Rₐ, -P(O)ₜ-ORₐ, alkyle en C₁ à C₆ linéaire ou ramifié, substitué ou non par un ou plusieurs groupes R₅, alcènyle en C₁ à C₆ linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié, -ORₐ, -NRₐR_{b}, -CO₂Rₐ, -C(O)Rₐ et -C(O)NRₐR_{b}, nitrile, nitro, -NRₐC(O)-T₁, alcoxy en C₁ à C₆, oxo, -S(O)ₜ-Rₐ, -S(O)ₜ-ORₐ, -S(O)ₜ-NRₐR_{b}, -P(O)ₜ-Rₐ, - P(O)t-ORa,
- Rₐ et R_{b} sont identiques ou différents et représentent chacun indépendamment l'un de l'autre un atome d'hydrogène, ou un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé, ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORₐ, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I et n'ont que des buts d'identification de chacun de ces cycles,
ou un des sels, isomères optiques et mélanges racémiques de ces composés, pour la préparation d'un médicament pour inhiber l'activité d'au moins une des kinases PIM-1, PIM-2 et PIM-3.

2. Utilisation selon la revendication 1 d'au moins un composé de formule I dans laquelle:
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, Br avec n = 1 ou 2 et R_{c} = OCH₃, OC₂H₅, N(C₂H₅)₂,
- R₃ et R₄ sont identiques ou différents et sont indépendamment l'un de l'autre choisis parmi H, un atome d'halogène, un groupe hétéroaryle à 5 ou 6 chaînons comprenant un ou deux hétéroatomes choisis parmi O et N, alkyle en C₁ à C₆ linéaire ou ramifié, méthoxy, nitro, nitrile, carboxy, trifluorométhyle, trifluorométhoxy, SO₂R_{d}, aryle en C₆ éventuellement substitué par un groupe choisi parmi un groupe (C=O)CH₃, phényle, méthoxy, trifluorométhoxy, trifluorométhyle, carboxy ou par 1 ou 2 atomes de fluor, avec R_{d} choisi parmi un groupe OH, CH₃ ou NH₂.

3. Utilisation selon la revendication 1 ou 2 d'au moins un composé de formule I dans laquelle
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, ou Br, avec n = 1 ou 2 et R_{c} représente un groupe OCH₃, OC₂H₅,ou N(C₂H₅)₂,
- R₃ et R₄ sont identiques ou différents et sont indépendamment l'un de l'autre choisis parmi H, un atome d'halogène, un groupe méthyle, éthyle, nitro, nitrile, trifluorométhyle, aryle en C₆ éventuellement substitué par un groupe choisi parmi un groupe (C=O)CH₃, phényle, méthoxy, trifluorométhoxy, trifluorométhyle, ou par 1 ou 2 atomes de fluor,

4. Utilisation selon l'une quelconque des revendications 1 à 3 **caractérisée en ce que** le au moins un composé de formule I est choisi parmi le 1,10-dihydropyrrolo[2,3-a]carbazole, le 1,10-dihydropyrrolo[2,3-a]carbazol-3-carbaldéhyde, le 1,10-dihydropyrrolo[2,3-a]carbazol-3-carboxamide, le 1-(1,10-dihydropyrrolo[2,3-a]carbazol-3-yl-2,2,2-trifluoroéthanone, le 7-bromo-1,10-dihydropyrrolo[2,3-a]carbazole, le 7-bromo-1,10-dihydropyrrolo[2,3-a]carbazol-3-carbaldéhyde, le 7-(2,4-difluorophényl)-1,10-dihydropyrrolo[2,3-a]carbazol-3-carbaldéhyde, le 6-bromo-1,10-dihydropyrrolo[2,3-a]carbazol-3-carbaldéhyde, le 9-bromo-1,10-dihydropyrrolo[2,3-a]carbazol-3-carbaldéhyde, le 7-fluoro-1,10-dihydropyrrolo[2,3-*a*]carbazol-3-carbaldéhyde, le 9-fluoro-1,10-dihydropyrrolo[2,3-*a*]carbazol-3-carbaldéhyde, le 6,8-dichloro-1,10-dihydropyrrolo[2,3-*a*]carbazol-3-carbaldéhyde, le 9-éthyl-1,10-dihydropyrrolo[2,3-*a*]carbazol-3-carbaldéhyde, et le 8-méthyl-1,10-dihydropyrrolo[2,3-*a*]carbazol-3-carbaldéhyde.

5. Utilisation selon l'une quelconque des revendications 1 à 4 **caractérisée en ce que** le au moins un composé de formule I est choisi parmi le 1,10-dihydropyrrolo[2,3-*a*]carbazole, le 1,10-dihydropyrrolo[2,3-*a*]carbazol-3-carbaldéhyde et le 1,10-dihydropyrrolo[2,3-*a*]carbazol-3-carboxamide.

6. Procédé de synthèse des composés de formule suivants : 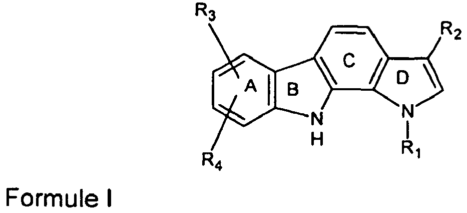 dans laquelle
- R₁ est H ou un groupe sulfophényle,
- R₂ R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocycloalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ ou -S(O)ₜ-Rₐ, -S(O)ₜ-ORₐ, -S(O)ₜ-NRₐR_{b}, -P(O)ₜ-Rₐ, -P(O)ₜ-ORₐ, alkyle en C₁ à C₆ linéaire ou ramifié, substitué ou non par un ou plusieurs groupes R₅, alcènyle en C₁ à C₆ linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié, -ORₐ, -NRₐR_{b}, -CO₂Rₐ, -C(O)Rₐ et -C(O)NRₐR_{b}, nitrile, nitro, -NRₐC(O)-T₁, alcoxy en C₁ à C₆, oxo, -S(O)ₜ-Rₐ, -S(O)ₜ-ORₐ, -S(O)ₜ-NRₐR_{b}, -P(O)ₜ-Rₐ, - P(O)ₜ-ORₐ,
- Rₐ et R_{b} sont identiques ou différents et représentent chacun indépendamment l'un de l'autre un groupe choisi parmi un atome d'hydrogène, un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé, ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORa, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I est n'ont que des buts d'identification de chacun de ces cycles,
**caractérisé en ce qu'**il comprend une réaction d'indolisation de Fischer du composé 1-benzènesulfonyl-1,4,5,6-tétrahydro-7*H*-indol-7-one de formule II suivante : 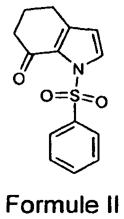 et de la phénylhydrazine ou de phénylhydrazine substituée sur le phényle par un ou plusieurs groupes R₅, en présence d'un liquide ionique qui est le chlorure de zinc-chlorure de choline 2:1 de formule III suivante :

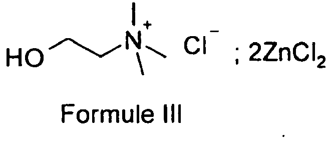

7. Composés de formule I suivante: 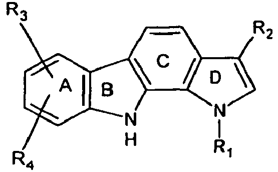 dans laquelle :
- R₁ est H ou un groupe sulfophényle,
- R₂, R₃ et R₄ sont identiques ou différents et représentent indépendamment l'un de l'autre un atome d'hydrogène, un atome d'halogène, ou un groupe nitro, nitrile, hydroxy, alcoxy en C₁ à C₆ linéaire ou ramifié, substitué ou non par un ou plusieurs groupes R₅, cycloalcoxy en C₅ à C₆ substitué ou non par un ou plusieurs groupes R₅, hétérocyctoalcoxy à 5 ou 6 chaînons substitué ou non par un ou plusieurs groupes R₅, -SH, alkylthio en C₁ à C₆ linéaire ou ramifié, substitué ou non par un ou plusieurs groupes R₅, aryle en C₆ substitué ou non par un ou plusieurs groupes R₅, aryloxy en C₆ substitué ou non par un ou plusieurs groupes R₅, -NRₐR_{b}, -NRₐC(O)-T₁, -C(N-OH)-T₃, - C(O)-T₁, -C(O)-C(O)-T₃, -C(O)-NRₐ-T₁, -NRₐ-C(O)-T₁, O-C(O)-T₁, -C(O)-O-T₁, - O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ ou -S(O)ₜ-Rₐ, -S(O)ₜ-ORₐ, -S(O)ₜ-NRₐR_{b}, -P(O)ₜ-Rₐ, -P(O)ₜ-ORₐ, alkyle en C₁ à C₆ linéaire ou ramifié, substitué ou non par un ou plusieurs groupes R₅, alcènyle en C₁ à **C₆** linéaire ou ramifié, substitué ou non par un ou plusieurs groupes R₅, alcynyle en C₁ à C₆ linéaire ou ramifié, substitué ou non par un ou plusieurs groupes R₅, hétéroaryle à 5 ou 6 chaînons substitué ou non par un ou plusieurs groupes R₅, hétérocycloalkyle à 5 ou 6 chaînons substitué ou non par un ou plusieurs groupes R₅, hétéroaryloxy à 5 ou 6 chaînons substitué ou non par un ou plusieurs groupes R₅,
- R₅ représente un atome d'halogène, ou un groupe alkyle en C₁ à C₆ linéaire ou ramifié, aryle en C₆, halogénoalkyle en C₁ à C₆ linéaire ou ramifié, -ORₐ, -NRₐR_{b}, -CO₂Rₐ, -C(O)Rₐ et -C(O)NRₐR_{b}, nitrile, nitro, -NRₐC(O)-T₁, alcoxy en C₁ à C₆, oxo, -S(O)ₜ-Rₐ, -S(O)ₜ-ORₐ, -S(O)ₜ-NRₐR_{b}, -P(O)ₜ-Rₐ, - P(O)ₜ-ORa,
- Rₐ et R_{b} sont identiques ou différents et représentent chacun indépendamment l'un de l'autre un atome d'hydrogène, ou un groupe alkyle en C₁ à C₆ linéaire ou ramifié, halogénoalkyle en C₁ à C₆ linéaire ou ramifié, aryle en C₆, où Rₐ + R_{b} forment ensemble, avec l'atome d'azote auquel ils sont liés un hétérocycle de 5 à 10 atomes, monocyclique ou bicyclique, saturé, ou insaturé, contenant éventuellement au sein des systèmes cycliques un second hétéroatome choisi parmi l'oxygène et l'azote, et étant éventuellement substitué par un ou plusieurs groupes R₅,
- T₁ représente un atome d'hydrogène, un atome d'halogène, ou un groupe alkyle en C₁ à C₆ linéaire ou ramifié, éventuellement substitué par un groupement choisi parmi -ORₐ, -NRₐR_{b}, -CO₂Rₐ, -C(O)Rₐ et -C(O)NRₐR_{b},
- T₂ représente une chaîne alkylidène (C₁-C₆) linéaire ou ramifiée,
- T₃ représente un groupement choisi parmi -halogène, - ORₐ, -NRₐR_{b}, -CO₂Rₐ, -C(O)Rₐ et -C(O)NRₐR_{b} dans lesquels Rₐ et R_{b} sont tels que définis précédemment,
- t représente un nombre entier compris entre 0 et 3 inclus,
- A, B, C, D désignent les cycles constituant les composés de formule I et n'ont que des buts d'identification de chacun de ces cycles,
à la condition que :
- R₁, R₂, R₃ et R₄ ne soient pas tous H en même temps,
- lorsque R₁ est un groupe sulfophényle, alors R₂, R₃ et R₄ ne sont pas tous H en même temps, et
- lorsque R₂ est un groupe carboxamide ou formyle, alors R₁, R₃ et R₄ ne sont pas tous H en même temps.

8. Composés de formule I selon la revendication 7, **caractérisés en ce que** dans la formule I :
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, Br avec n = 1 ou 2 et R_{c} = OCH₃, OC₂H₅, N(C₂H₅)₂,
- R₃ et R₄ sont identiques ou différents et sont indépendamment l'un de l'autre choisis parmi H, un atome d'halogène, un groupe hétéroaryle à 5 ou 6 chaînons comprenant un ou deux hétéroatomes choisis parmi O et N, alkyle en C₁ à C₆ linéaire ou ramifié, méthoxy, nitro, nitrile, carboxy, trifluorométhyle, trifluorométhoxy, SO₂R_{d}, aryle en C₆ éventuellement substitué par un groupe choisi parmi un groupe (C=O)CH₃, phényle, méthoxy, trifluorométhoxy, trifluorométhyle, carboxy ou par 1 ou 2 atome de fluor, avec R_{d} choisi parmi un groupe OH, CH₃ ou NH₂.

9. Composés selon la revendication 7 ou 8, **caractérisés en ce que** dans la formule I :
- R₁ est H ou un groupe sulfophényle,
- R₂ représente H, ou un groupe CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, ou Br, avec n = 1 ou 2 et R_{c} représente un groupe OCH₃, OC₂H₅,ou N(C₂H₅)₂,
- R₃ et R₄ sont identiques ou différents et sont indépendamment l'un de l'autre choisis parmi H, un atome d'halogène, un groupe méthyle, éthyle, nitro, nitrile, trifluorométhyle, aryle en C₆ éventuellement substitué par un groupe choisi parmi un groupe (C=O)CH₃, phényle, méthoxy, trifluorométhoxy, trifluorométhyle, ou par 1 ou 2 atome de fluor.

10. Composés de formule I selon l'une quelconque des revendications 7 à 9, **caractérisés en ce qu'**ils sont choisis parmi :
- le (1,10-dihydropyrrolo[2,3-*a*]carbazol-3-yl)méthanol,
- le 1-(1,10-dihydropyrrolo[2,3-*a*]carbazol-3-yl)-2,2,2-trifluoroéthanone,
- le 2-(1,10-dihydropyrrolo[2,3-*a*]carbazol-3-yl)-2-oxoacétate de méthyle,
- le 2-(1,10-dihydropyrrolo[2,3-*a*]carbazol-3-yl)-2-oxoacétate d'éthyle,
- le 2-(1,10-dihydropyrrolo[2,3-*a*]carbazol-3-yl)éthanol,
- le 2-(1,10-dihydropyrrolo[2,3-*a*]carbazol-3-yl)-N,N-diéthyl-2-oxoacétamide,
- le 2-(1,10-dihydropyrrolo[2,3-*a*]carbazol-3-yl)-N,N-diéthyléthanamine,
- le 1-(1,10-dihydropyrrolo[2,3-*a*]carbazol-3-yl)-2-diéthylaminoéthanol,
- le 2-(1,10-dihydropyrrolo[2,3-*a*]carbazol-3-yl)-N,N-diéthyl-2-hydroxyiminoéthanamide,
- le 1-benzènesulfonyl-7-bromo-1,10-dihydropyrrolo[2,3-*a*]carbazole,
- le 7-bromo-1,10-dihydropyrrolo[2,3-*a*]carbazole,
- le 7-bromo-1,10-dihydropyrrolo[2,3-*a*]carbazol-3-carbaldéhyde,
- le 7-(4-acétylphényl)-1,10-dihydropyrrolo[2,3-*a*]carbazole,
- le 7-(3-méthoxyphényl)-1,10-dihydropyrrolo[2,3-*a*]carbazole,
- le 7-(4-biphényl)-1,10-dihydropyrrolo[2,3-*a*]carbazole,
- le 7-phényl-1,10-dihydropyrrolo[2,3-*a*]carbazole,
- le 7-(4-fluorophényl)-1,10-dihydropyrrolo[2,3-*a*]carbazole,
- le 7-(2,4-difluorophényl)-1,10-dihydropyrrolo[2,3-*a*]carbazole,
- le 7-(4-trifluorométhylphényl)-1,10-dihydropyrrolo[2,3-*a*]carbazole,
- le 7-(4-trifluoromethoxyphényl)-1,10-dihydropyrrolo[2,3-*a*]carbazole,
- le 8-bromo-1,10-dihydropyrrolo[2,3-*a*]carbazole,
- le 6-bromo-1,10-dihydropyrrolo[2,3-*a*]carbazole,
- le 9-bromo-1,10-dihydropyrrolo[2,3-*a*]carbazole,
- le 7-fluoro-1,10-dihydropyrrolo[2,3-*a*]carbazole,
- le 9-fluoro-1,10-dihydropyrrolo[2,3-*a*]carbazole,
- le 6,8-dichloro-1,10-dihydropyrrolo[2,3-*a*]carbazole,
- le 1,10-dihydropyrrolo[2,3-*a*]carbazol-7-carbonitrile,
- le 7-nitro-1,10-dihydropyrrolo[2,3-*a*]carbazole,
- le 9-éthyl-1,10-dihydropyrrolo[2,3-*a*]carbazole,
- le 9-(trifluorométhyl)-1,10-dihydropyrrolo[2,3-*a*]carbazole,
- le 8-méthyl-1,10-dihydropyrrolo[2,3-*a*]carbazole,
- le 7-(3-methoxyphényl)-1,10-dihydropyrrolo[2,3-*a*]carbazol-3-carbaldéhyde,
- le 7-(4-biphényl)-1,10-dihydropyrrolo[2,3-*a*]carbazol-3-carbaldéhyde,
- le 7-phényl-1,10-dihydropyrrolo[2,3-*a*]carbazol-3-carbaldéhyde,
- le 7-(4-fluorophényl)-1,10-dihydropyrrolo[2,3-*a*]carbazol-3-carbaldéhyde,
- le 7-(2,4-difluorophényl)-1,10-dihydropyrrolo[2,3-*a*]carbazol-3-carbaldéhyde,
- le 7-(4-trifluorométhylphényl)-1,10-dihydropyrrolo[2,3-*a*]carbazol-3-carbaldéhyde,
- le 7-(4-trifluoromethoxyphényl)-1,10-dihydropyrrolo[2,3-*a*]carbazol-3-carbaldéhyde,
- le 8-bromo-1,10-dihydropyrrolo[2,3-*a*]carbazol-3-carbaldéhyde,
- le 6-bromo-1,10-dihydropyrrolo[2,3-*a*]carbazol-3-carbaldéhyde,
- le 9-bromo-1,10-dihydropyrrolo[2,3-*a*]carbazol-3-carbaldéhyde,
- le 7-fluoro-1,10-dihydropyrrolo[2,3-*a*]carbazol-3-carbaldéhyde,
- le 9-fluoro-1,10-dihydropyrrolo[2,3-*a*]carbazol-3-carbaldéhyde,
- le 6,8-dichloro-1,10-dihydropyrrolo[2,3-*a*]carbazol-3-carbaldéhyde,
- le 3-formyl-1,10-dihydropyrrolo[2,3-*a*]carbazol-7-carbonitrile,
- le 7-nitro-1,10-dihydropyrrolo[2,3-*a*]carbazol-3-carbaldéhyde,
- le 9-éthyl-1,10-dihydropyrrolo[2,3-*a*]carbazol-3-carbaldéhyde,
- le 8-méthyl-1,10-dihydropyrrolo[2,3-*a*]carbazol-3-carbaldéhyde.

11. Composés de formule I selon l'une quelconque des revendications 7 à 9, **caractérisés en ce qu'**ils sont choisis parmi le 1,10-dihydropyrrolo[2,3-*a*]carbazol-3-yl)-2,2,2-trifluoroéthanone, le 7-bromo-1,10-dihydropyrrolo[2,3-*a*]carbazole, le 7-bromo-1,10-dihydropyrrolo[2,3-*a*]carbazol-3-carbaldéhyde, le 7-(2,4-difluorophényl)-1,10-dihydropyrrolo[2,3-*a*]carbazol-3-carbaldéhyde, le 6-bromo-1,10-dihydropyrrolo[2,3-*a*]carbazol-3-carbaldéhyde, le 9-bromo-1,10-dihydropyrrolo[2,3-*a*]carbazol-3-carbaldéhyde, le 7-fluoro-1,10-dihydropyrrolo[2,3-*a*]carbazol-3-carbaldéhyde, le 9-fluoro-1,10-dihydropyrrolo[2,3-*a*]carbazol-3-carbaldéhyde, le 6,8-dichloro-1,10-dihydropyrrolo[2,3-*a*]carbazol-3-carbaldéhyde, le 9-éthyl-1,10-dihydropyrrolo[2,3-*a*]carbazol-3-carbaldéhyde, et le 8-méthyl-1,10-dihydropyrrolo[2,3-*a*]carbazol-3-carbaldéhyde.

## Claims

1. Use of at least one compound of the following formula I: 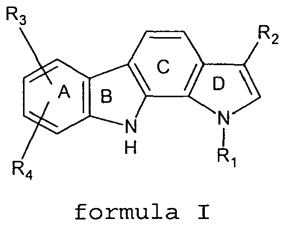 in which
- R₁ is H or a sulfophenyl group
- R₂, R₃ and R₄ are identical or different and independently of each other represent a hydrogen atom, a halogen atom or one of the groups: nitro, nitrile, hydroxyl, linear or branched C₁- to C₆-alkoxy which is unsubstituted or substituted by one or more groups designated by R₅, C₅- to C₆-cycloalkoxy which is unsubstituted or substituted by one or more groups designated by R₅, 5- or 6-membered heterocycloalkoxy which is unsubstituted or substituted by one or more groups designated by R₅, -SH, linear or branched C₁- to C₆-alkylthio which is unsubstituted or substituted by one or more groups designated by R₅, C₆-aryl which is unsubstituted or substituted by one or more groups designated by R₅, C₆-aryloxy which is unsubstituted or substituted by one or more groups designated by R₅, -NRₐR_{b}, -NRₐC(O) -T₁, -C(N-OH)-T₃, -C(O)-T₁, -C(O)-C(O)-T₃, -C(O)-NRₐ-T₁, -NRₐ-C(O)-T₁, O-C(O)-T₁, -C(O)-O-T₁, -O-T₂-NRₐR_{b}, -O-T₂-ORₐ, -0-T₂-CO₂Rₐ, -NRₐ-T₂-NRₐR_{b}, -NRₐ-T₂-ORₐ, -NRₐ-T₂-CO₂Rₐ or -S(O)ₜ-Rₐ, -S(O)ₜ-ORₐ, -S(O)ₜ-NRₐR_{b}, -P(O)ₜ-Rₐ, -P(O)ₜ-ORₐ, linear or branched C₁- to C₆-alkyl which is unsubstituted or substituted by one or more groups designated by R₅, linear or branched C₁- to C₆-alkenyl which is unsubstituted or substituted by one or more groups designated by R₅, linear or branched C₁- to C₆-alkynyl which is unsubstituted or substituted by one or more groups designated by R₅ group, 5- or 6-membered heteroaryl which is unsubstituted or substituted by one or more groups designated by R₅, 5- or 6-membered heterocycloalkyl which is unsubstituted or substituted by one or more groups designated by R₅, 5- or 6-membered heteroaryloxy which is unsubstituted or substituted by one or more groups designated by R₅,
- R₅ represents a halogen atom or one of the groups: linear or branched C₁- to C₆-alkyl, C₆-aryl, linear or branched C₁- to C₆-haloalkyl, -ORₐ, -NRₐR_{b}, -CO₂Rₐ, -C(O)Rₐ and -C(O)NRₐR_{b}, nitrile, nitro, -NRₐC(O)-T₁, C₁- to C₆-alkoxy, oxo, -S(O)ₜ-Rₐ, -S(O)ₜ-ORₐ, -S(O)ₜ-NRₐR_{b}, -P(O)ₜ-Rₐ, -P(O)ₜ-ORₐ,
- Rₐ and R_{b} are identical or different and each independently of each other represent a hydrogen atom or one of the groups: linear or branched C₁- to C₆-alkyl, linear or branched C₁- to C₆-haloalkyl, C₆-aryl, where Rₐ + R_{b} together, with the nitrogen atom to which they are bonded, form a monocyclic or bicyclic, saturated or unsaturated 5- to 10-membered heterocyclyl optionally containing within its ring systems a second hetero atom chosen from oxygen and nitrogen and optionally being substituted by one or more groups designated by R₅,
- T₁ represents a hydrogen atom, a halogen atom or a linear or branched C₁- to C₆-alkyl group optionally substituted by a grouping chosen from -ORₐ, -NRₐR_{b},
- CO₂Rₐ, -C(O)Rₐ and -C(O)NRₐR_{b},
- T₂ represents a linear or branched (C₁-C₆) - alkylidene chain,
- T₃ represents a grouping chosen from -halogen,
- ORₐ, -NRₐR_{b}, -CO₂Rₐ, -C(O)Rₐ and -C(O)NRₐR_{b}, in which Rₐ and R_{b} are as defined above,
- t represents an integer between 0 and 3 inclusive,
- A, B, C, D designate the rings which make up the compounds of the formula I and serve only for identification of each of these rings,
or one of the salts, optical isomers and racemic mixtures of these compounds, for the preparation of a medicament for inhibiting the activity of at least one of the kinases PIM-1, PIM-2 and PIM-3.

2. Use according to claim 1 of at least one compound of the formula I in which
- R₁ is H or a sulfophenyl group,
- R₂ represents H or one of the groups: CHO, (CH₂)ₙOH, C(=O)NH_{2,} C(=O)-CF₃, (C=O)₂R_{c}, (CH₂)₂NEt₂, CH(OH)CH₂N(Et)₂, C(NOH)-(C=O)N(Et)₂, NO₂ Br, where n = 1 or 2 and R_{c} = OCH₃ , OC₂H₅ , N(C₂H₅)₂,
- R₃ and R₄ are identical or different and are chosen independently of each other from H, a halogen atom or one of the groups: 5- or 6-membered heteroaryl containing one or two hetero atoms chosen from O and N, linear or branched C₁- to C₆-alkyl, methoxy, nitro, nitrile, carboxyl, trifluoromethyl, trifluoromethoxy, SO₂R_{d}, C₆-aryl optionally substituted by a group chosen from one of the groups: (C=O)CH₃, phenyl , methoxy, trifluoromethoxy, trifluoromethyl, carboxyl or by 1 or 2 fluorine atoms, where R_{d} is chosen from one of the groups: OH, CH₃ or NH₂.

3. Use according to claim 1 or 2 of at least one compound of the formula I in which
- R₁ is H or a sulfophenyl group,
- R₂ represents H or one of the groups: CHO, (CH₂)ₙOH, C(O)NH₂, C(=O)-CF₃, (C=O)₂R_{c}, (CH₂)₂NEt₂, CH(OH)CH₂N(Et)₂, C(NOH)-(C=O)N(Et)_{2,} NO₂ or Br, where n = 1 or 2 and R_{c} represents one of the groups: OCH₃, OC₂H₅ or N(C₂H₅)₂,
- R₃ and R₄ are identical or different and are chosen independently of each other from H, a halogen atom or one of the groups: methyl, ethyl, nitro, nitrile, trifluoromethyl, C₆-aryl which is optionally substituted by a group chosen from one of the groups: (C=O)CH₃, phenyl, methoxy, trifluoromethoxy, trifluoromethyl, or by 1 or 2 fluorine atoms.

4. Use according to any one of claims 1 to 3, **characterized in that** the at least one compound of the formula I is chosen from 1,10-dihydropyrrolo[2,3-a] carbazole, 1,10-dihydropyrrolo[2,3-*a*]carbazole-3-carbaldehyde, 1,10-dihydropyrrolo[2,3-*a*]carbazole-3-carboxamide, 1-(1,10-dihydropyrrolo[2,3-*a*]carbazol-3-yl-2,2,2-trifluoroethanone, 7-bromo-1,10-dihydropyrrolo[2,3-*a*]carbazole, 7-bromo-1,10-dihydropyrrolo[2,3-*a*]carbazole-3-carbaldehyde, 7-(2,4-difluorophenyl)-1,10-dihydropyrrolo[2,3-*a*]carbazole-3-carbaldehyde, 6-bromo-1,10-dihydropyrrolo[2,3-a]carbazole-3-carbaldehyde, 9-bromo-1,10-dihydropyrrolo[2,3-*a*]carbazole-3-carbaldehyde, 7-fluoro-1,10-dihydropyrrolo[2,3-*a*]carbazole-3-carbaldehyde, 9-fluoro-1,10-dihydropyrrolo[2,3-*a*]carbazole-3-carbaldehyde, 6,8-dichloro-1,10-dihydropyrrolo[2,3-*a*]carbazole-3-carbaldehyde, 9-ethyl-1,10-dihydropyrrolo[2,3-*a*]carbazole-3-carbaldehyde and 8-methyl-1,10-dihydropyrrolo[2,3-a]carbazole-3-carbaldehyde.

5. Use according to any one of claims 1 to 4, **characterized in that** the at least one compound of the formula I is chosen from 1,10-dihydropyrrolo[2,3-a] carbazole, 1,10-dihydropyrrolo[2,3-*a*]carbazole-3-carbaldehyde and 1,10-dihydropyrrolo[2,3-*a*]carbazole-3-carboxamide.

6. Process for the synthesis of compounds of the following formula I: 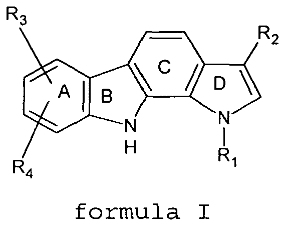 in which
- R₁ is H or a sulfophenyl group
- R₂, R₃ and R₄ are identical or different and independently of each other represent a hydrogen atom, a halogen atom or one of the groups: nitro, nitrile, hydroxyl, linear or branched C₁- to C₆-alkoxy which is unsubstituted or substituted by one or more groups designated by R₅, C₅- to C₆-cycloalkoxy which is unsubstituted or substituted by one or more groups designated by R₅, 5- or 6-membered heterocycloalkoxy which is unsubstituted or substituted by one or more groups designated by R₅, -SH, linear or branched C₁- to C₆-alkylthio which is unsubstituted or substituted by one or more groups designated by R₅, C₆-aryl which is unsubstituted or substituted by one or more groups designated by R₅, C₆-aryloxy which is unsubstituted or substituted by one or more groups designated by R₅, -NRₐR_{b}, -NRₐC(O)-T₁, -C(N-OH)-T₃, -C(O)-T₁, -C(O)-C(O)-T₃ , -C(O)-NRₐ-T₁, -NRₐ-C(O)-T₁, O-C(O)-T₁, -C(O)-O-T₁, -O-T₂-NRₐR_{b}, -O-T₂-ORₐ, -0-T₂-CO₂Rₐ, -NRₐ-T₂-NRₐR_{b}, -NRₐ-T₂-ORₐ, -NRₐ-T₂-CO₂Rₐ or -S(O)ₜ-Rₐ, -S(O)ₜ-ORₐ, -S(O)ₜ-NRₐR_{b}, -P(O)ₜ-Rₐ, -P(O)ₜ-ORₐ, linear or branched C₁-to C₆-alkyl which is unsubstituted or substituted by one or more groups designated by R₅, linear or branched C₁-to C₆-alkenyl which is unsubstituted or substituted by one or more groups designated by R₅, linear or branched C₁- to C₆-alkynyl which is unsubstituted or substituted by one or more groups designated by R₅ group, 5- or 6-membered heteroaryl which is unsubstituted or substituted by one or more groups designated by R₅, 5- or 6-membered heterocycloalkyl which is unsubstituted or substituted by one or more groups designated by R₅, 5- or 6-membered heteroaryloxy which is unsubstituted or substituted by one or more groups designated by R₅,
- R₅ represents a halogen atom or one of the groups: linear or branched C₁- to C₆-alkyl, C₆-aryl, linear or branched C₁- to C₆-haloalkyl, -ORₐ, -NRₐR_{b}, -CO₂Rₐ, -C(O)Rₐ and -C(O)NRₐR_{b}, nitrile, nitro, -NRₐC(O)-T₁, C₁- to C₆-alkoxy, oxo, -S(Oₜ-Rₐ, -S(O)ₜ-ORₐ, -S(O)ₜ-NRₐR_{b}, -P(O)ₜ-Rₐ, -P(O)ₜ-ORₐ,
- Rₐ and R_{b} are identical or different and each independently of each other represent a group chosen from a hydrogen atom or one of the groups: linear or branched C₁- to C₆-alkyl, linear or branched C₁- to C₆-haloalkyl, C₆-aryl, where Rₐ + R_{b} together, with the nitrogen atom to which they are bonded, form a monocyclic or bicyclic, saturated or unsaturated 5- to 10-membered heterocyclyl optionally containing within its ring systems a second hetero atom chosen from oxygen and nitrogen and optionally being substituted by one or more groups designated by R₅,
- T₁ represents a hydrogen atom, a halogen atom or a linear or branched C₁- to C₆-alkyl group optionally substituted by a grouping chosen from -ORₐ, -NRₐR_{b}, -CO₂Rₐ, -C(O)Rₐ, and -C(O)NRₐR_{b},
- T₂ represents a linear or branched (C₁-C₆) - alkylidene chain,
- T₃ represents a grouping chosen from -halogen, -ORₐ, -NRₐR_{b}, -CO₂Rₐ, -C(O)Rₐ and -C(O)NRₐR_{b}, in which Rₐ and R_{b} are as defined above,
- t represents an integer between 0 and 3 inclusive,
- A, B, C, D designate the rings which make up the compounds of the formula I and serve only for identification of each of these rings,
**characterized in that** it comprises a Fischer indolization reaction of the compound 1-benzenesulfonyl-1,4,5,6-tetrahydro-7H-indol-7-one of the following formula II: 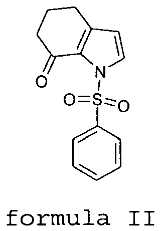 and phenylhydrazine or phenylhydrazine substituted on the phenyl by one or more groups designated by R₅, in the presence of an ionic lquid which is the 2:1 zinc dichloride of choline of the following formula III:

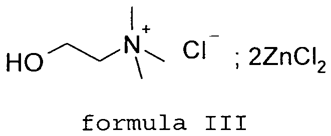

7. Compounds of the following formula I: 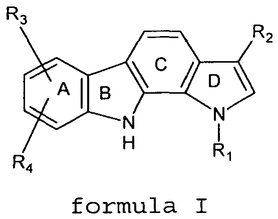 in which
- R₁ is H or a sulfophenyl group
- R₂, R₃ and R₄ are identical or different and independently of each other represent a hydrogen atom, a halogen atom or one of the groups: nitro, nitrile, hydroxyl, linear or branched C₁- to C₆-alkoxy which is unsubstituted or substituted by one or more groups designated by R₅, C₅- to C₆-cycloalkoxy which is unsubstituted or substituted by one or more groups designated by R₅, 5- or 6-membered heterocycloalkoxy which is unsubstituted or substituted by one or more groups designated by R₅, -SH, linear or branched C₁- to C₆-alkylthio which is unsubstituted or substituted by one or more groups designated by R₅, C₆-aryl which is unsubstituted or substituted by one or more groups designated by R₅, C₆-aryloxy which is unsubstituted or substituted by one or more groups designated by R₅, -NRₐR_{b}, -NRₐC(O)-T₁, -C(N-OH)-T₃, -C(O)-T₁, -C(O)-C(O)-T₃, -C(O)-NRₐ-T₁, -NRₐ-C(O)-T₁, O-C(O)-T₁, -C(O)-O-T₁, -O-T₂-NRₐR_{b}, -O-T₂ORₐ, -O-T₂-CO₂Rₐ, -NRₐ-T₂-NRₐR_{b}, -NRₐ-T₂-ORₐ, -NRₐ-TrCO₂Rₐ or -S(O)ₜ-Rₐ, -S(O)ₜ-ORₐ, -S(O)ₜ-NRₐR_{b}, -P(O)ₜ-Ra, -P(O)ₜ-ORₐ, linear or branched C₁- to C₆-alkyl which is unsubstituted or substituted by one or more groups designated by R₅, linear or branched C₁- to C₆-alkenyl which is unsubstituted or substituted by one or more groups designated by R₅, linear or branched C₁- to C₆-alkynyl which is unsubstituted or substituted by one or more groups designated by R₅ group, 5- or 6-membered heteroaryl which is unsubstituted or substituted by one or more groups designated by R₅, 5- or 6-membered heterocycloalkyl which is unsubstituted or substituted by one or more groups designated by R₅, 5- or 6-membered heteroaryloxy which is unsubstituted or substituted by one or more groups designated by R₅,
- R₅ represents a halogen atom or one of the groups: linear or branched C₁- to C₆-alkyl, C₆-aryl, linear or branched C₁- to C₆-haloalkyl, -ORₐ, -NRₐR_{b}, -CO₂Rₐ, -C(O)Rₐ and -C(O)NRₐR_{b}, nitrile, nitro, -NRₐC(O)-T₁, C₁- to C₆-alkoxy, oxo, -S(O)ₜ-Rₐ, -S(O)ₜ-ORₐ, -S(O)ₜ-NRₐR_{b}, -P(O)ₜ-Rₐ, -P(O)ₜ-ORₐ.
- Rₐ and R_{b} are identical or different and each independently of each other represent a hydrogen atom or one of the groups: linear or branched C₁- to C₆-alkyl, linear or branched C₁- to C₆-haloalkyl, C₆-aryl, where Rₐ + R_{b} together, with the nitrogen atom to which they are bonded, form a monocyclic or bicyclic, saturated or unsaturated 5- to 10-membered heterocyclyl optionally containing within its ring systems a second hetero atom chosen from oxygen and nitrogen and optionally being substituted by one or more groups designated by R₅,
- T₁ represents a hydrogen atom, a halogen atom or a linear or branched C₁- to C₆-alkyl group optionally substituted by a grouping chosen from -ORₐ, -NRₐR_{b}, -CO₂Rₐ, -C(O)Rₐ and -C(O)NRₐR_{b},
- T₂ represents a linear or branched (C₁-C₆) - alkylidene chain,
- T₃ represents a grouping chosen from -halogen, -ORₐ, -NRₐR_{b}, -CO₂Rₐ, -C(O)Rₐ and -C(O)NRₐR_{b}, in which Rₐ and R_{b} are as defined above,
- t represents an integer between 0 and 3 inclusive,
- A, B, C, D designate the rings which make up the compounds of the formula I and serve only for identification of each of these rings,
with the proviso that:
- R₁, R₂, R₃ and R₄ are not all simultaneously H,
- if R₁ is a sulfophenyl group, then R₂, R₃ and R₄ are not all simultaneously H, and
- if R₂ is a carboxamide or formyl group, then R₁, R₃ and R₄ are not all simultaneously H.

8. Compounds of the formula I according to claim 7, **characterized in that** in the formula I:
- R₁ is H or a sulfophenyl group,
- R₂ represents H or one of the groups: CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂ Br, where n = 1 or 2 and R_{c} = OCH₃ , OC₂H₅, N(C₂H₅)₂,
- R₃ and R₄ are identical or different and are chosen independently of each other from H, a halogen atom or one of the groups: 5- or 6-membered heteroaryl containing one or two hetero atoms chosen from O and N, linear or branched C₁- to C₆-alkyl, methoxy, nitro, nitrile, carboxyl, trifluoromethyl, trifluoromethoxy, SO₂R_{d}, C₆-aryl optionally substituted by a group chosen from one of the groups: (C=O)CH₃, phenyl, methoxy, trifluoromethoxy, trifluoromethyl, carboxyl or by 1 or 2 fluorine atoms, where R_{d} is chosen from one of the groups: OH, CH₃ or NH₂.

9. Compounds according to claim 7 or 8, **characterized in that** in the formula I:
- R₁ is H or a sulfophenyl group,
- R₂ represents H or one of the groups: CHO, (CH₂)ₙOH, C(O)NH₂, C(=O)-CF₃, (C=O)₂R_{c}, (CH₂)₂NEt₂, CH(OH)CH₂N(Et)₂, C(NOH)-(C=O)N(Et)₂, NO₂ or Br, where n = 1 or 2 and R_{c} represents one of the groups: OCH₃, OC₂H₅ or N(C₂H₅)₂,
- R₃ and R₄ are identical or different and are chosen independently of each other from H, a halogen atom or one of the groups: methyl, ethyl, nitro, nitrile, trifluoromethyl, C₆-aryl which is optionally substituted by a group chosen from one of the groups: (C=O)CH₃, phenyl, methoxy, trifluoromethoxy, trifluoromethyl, or by 1 or 2 fluorine atoms.

10. Compounds of the formula I according to any one of claims 7 to 9, **characterized in that** they are chosen from:
- (1,10-dihydropyrrolo[2,3-a]carbazol-3-yl)methanol,
- 1-(1,10-dihydropyrrolo[2,3-a]carbazol-3-yl)-2,2,2-trifluoroethanone,
- methyl 2-(1,10-dihydropyrrolo[2,3-*a*]carbazol-3-yl)-2-oxoacetate,
- ethyl 2-(1,10-dihydropyrrolo[2,3-*a*]carbazol-3-yl)-2-oxoacetate,
- 2-(1,10-dihydropyrrolo[2,3-*a*]carbazol-3-yl)ethanol,
- 2-(1,10-dihydropyrrolo[2,3-*a*] carbazol-3-yl)-*N,N-*diethyl-2-oxoacetamide,
- 2-(1,10-dihydropyrrolo[2,3-*a*]carbazol-3-yl)-*N,N-*diethylethanamine,
- 1-(1,10-dihydropyrrolo[2,3-*a*]carbazol-3-yl)-2-diethylaminoethanol,
- 2-(1,10-dihydropyrrolo[2,3-*a*]carbazol-3-yl)-*N,N-*diethyl-2-hydroxyiminoethanamide,
- 1-benzenesulfonyl-7-bromo-1,10-dihydropyrrolo[2,3-*a*]carbazole,
- 7-bromo-1,10-dihydropyrrolo[2,3-*a*]carbazole,
- 7-bromo-1,10-dihydropyrrolo[2,3-*a*]carbazole-3-carbaldehyde,
- 7-(4-acetylphenyl)-1,10-dihydropyrrolo[2,3-*a*] carbazole,
- 7-(3-methoxyphenyl)-1,10-dihydropyrrolo[2,3-*a*] carbazole,
- 7-(4-biphenyl)-1,10-dihydropyrrolo[2,3-*a*]carbazole,
- 7-phenyl-1,10-dihydropyrrolo[2,3-*a*]carbazole,
- 7-(4-fluorophenyl)-1,10-dihydropyrrolo[2,3-*a*]carbazole,
- 7-(2,4-difluorophenyl)-1,10-dihydropyrrolo[2,3-*a*]carbazole,
- 7-(4-trifluoromethylphenyl)-1,10-dihydropyrrolo[2,3-*a*]carbazole,
- 7-(4-trifluoromethoxyphenyl)-1,10-dihydropyrrolo[2,3-*a*]carbazole,
- 8-bromo-1,10-dihydropyrrolo[2,3-*a*]carbazole,
- 6-bromo-1,10-dihydropyrrolo[2,3-*a*]carbazole,
- 9-bromo-1,10-dihydropyrrolo[2,3-*a*]carbazole,
- 7-fluoro-1,10-dihydropyrrolo[2,3-*a*]carbazole,
- 9-fluoro-1,10-dihydropyrrolo[2,3-*a*]carbazole,
- 6,8-dichloro-1,10-dihydropyrrolo[2,3-*a*]carbazole,
- 1,10-dihydropyrrolo[2,3-*a*]carbazole-7-carbonitrile,
- 7-nitro-1,10-dihydropyrrolo[2,3-*a*]carbazole,
- 9-ethyl-1,10-dihydropyrrolo[2,3-*a*]carbazole,
- 9-(trifluoromethyl)-1,10-dihydropyrrolo[2,3-*a*] carbazole,
- 8-methyl-1,10-dihydropyrrolo[2,3-*a*]carbazole,
- 7-(3-methoxyphenyl)-1,10-dihydropyrrolo[2,3-*a*]carbazole-3-carbaldehyde,
- 7-(4-biphenyl)-1,10-dihydropyrrolo[2,3-*a*]carbazole-3-carbaldehyde,
- 7-phenyl-1,10-dihydropyrrolo[2,3-*a*]carbazole-3-carbaldehyde,
- 7-(4-fluorophenyl)-1,10-dihydropyrrolo[2,3-*a*]carbazole-3-carbaldehyde,
- 7-(2,4-difluorophenyl)-1,10-dihydropyrrolo[2,3-*a*]carbazole-3-carbaldehyde,
- 7-(4-trifluoromethylphenyl)-1,10-dihydropyrrolo[2,3-*a*]carbazole-3-carbaldehyde,
- 7-(4-trifluoromethoxyphenyl)-1,10-dihydropyrrolo[2,3-*a*]carbazole-3-carbaldehyde,
- 8-bromo-1,10-dihydropyrrolo[2,3-*a*]carbazole-3-carbaldehyde,
- 6-bromo-1,10-dihydropyrrolo[2,3-*a*]carbazole-3-carbaldehyde,
- 9-bromo-1,10-dihydropyrrolo[2,3-*a*]carbazole-3-carbaldehyde,
- 7-fluoro-1,10-dihydropyrrolo[2,3-*a*]carbazole-3-carbaldehyde,
- 9-fluoro-1,10-dihydropyrrolo[2,3-*a*]carbazole-3-carbaldehyde,
- 6,8-dichloro-1,10-dihydropyrrolo[2,3-*a*]carbazole-3-carbaldehyde,
- 3-formyl-1,10-dihydropyrrolo[2,3-*a*]carbazole-7-carbonitrile,
- 7-nitro-1,10-dihydropyrrolo[2,3-*a*]carbazole-3-carbaldehyde,
- 9-ethyl-1,10-dihydropyrrolo[2,3-*a*]carbazole-3-carbaldehyde,
- 8-methyl-1,10-dihydropyrrolo[2,3-*a*]carbazole-3-carbaldehyde.

11. Compounds of the formula according to any one of claims 7 to 9, **characterized in that** they are chosen from 1,10-dihydropyrrolo[2,3-*a*]carbazol-3-yl)-2,2,2-trifluoroethanone, 7-bromo-1,10-dihydropyrrolo[2,3-*a*]carbazole, 7-bromo-1,10-dihydropyrrolo[2,3-*a*]carbazole-3-carbaldehyde, 7-(2,4-difluorophenyl)-1,10-dihydropyrrolo[2,3-*a*]carbazole-3-carbaldehyde, 6-bromo-1,10-dihydropyrrolo[2,3-*a*]carbazole-3-carbaldehyde, 9-bromo-1,10-dihydropyrrolo[2,3-*a*]carbazole-3-carbaldehyde, 7-fluoro-1,10-dihydropyrrolo[2,3-*a*]carbazole-3-carbaldehyde, 9-fluoro-1,10-dihydropyrrolo[2,3-*a*]carbazole-3-carbaldehyde, 6,8-dichloro-1,10-dihydropyrrolo[2,3-*a*]carbazole-3-carbaldehyde, 9-ethyl-1,10-dihydropyrrolo[2,3-*a*]carbazole-3-carbaldehyde and 8-methyl-1,10-dihydropyrrolo[2,3-*a*]carbazole-3-carbaldehyde.

## Patentansprüche

1. Verwendung mindestens einer Verbindung der folgenden Formel I: 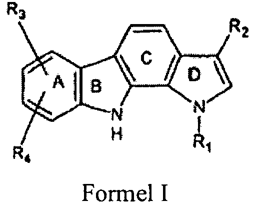 in der
- R₁ H oder eine Sulfophenylgruppe ist,
- R₂, R₃ und R₄ identisch oder verschieden sind und unabhängig voneinander für ein Wasserstoffatom, ein Halogenatom oder eine Nitro-, Nitril-, Hydroxygruppe, eine geradkettige oder verzweigte, gegebenenfalls mit einer oder mehreren Gruppen R₅ substituierte C₁-C₆-Alkoxygruppe, eine gegebenenfalls mit einer oder mehreren Gruppen R₅ substituierte C₅-C₆-Cycloalkoxygruppe, eine 5- oder 6-gliedrige, gegebenenfalls mit einer oder mehreren Gruppen R₅ substituierte Heterocycloalkoxygruppe, -SH, eine geradkettige oder verzweigte, gegebenenfalls mit einer oder mehreren Gruppen R₅ substituierte C₁-C₆-Alkylthiogruppe, eine gegebenenfalls mit einer oder mehreren Gruppen R₅ substituierte C₆-Arylgruppe, eine gegebenenfalls mit einer oder mehreren Gruppen R₅ substituierte C₆-Aryloxygruppe, eine Gruppe der Formel -NRₐR_{b}, -NRₐC(O)-T₁, -C(N-OH)-T₃, -C(O)-T₁, -C(O)-C(O)-T₃, -C(0)-NRₐ-T₁, -NRₐ-C(O)-T₁, O-C(O)-T₁, -C(O)-O-T₁, -O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂Rₐ oder -S(O)ₜ-Rₐ, -S(O)ₜ-ORₐ, -S(O)ₜ-NRₐR_{b}, -P(O)ₜ-Rₐ, -P(O)ₜ-ORₐ, eine geradkettige oder verzweigte, gegebenenfalls mit einer oder mehreren Gruppen R₅ substituierte C₁-C₆-Alkylgruppe, eine geradkettige oder verzweigte, gegebenenfalls mit einer oder mehreren Gruppen R₅ substituierte C₁-C₆-Alkenylgruppe, eine geradkettige oder verzweigte, gegebenenfalls mit einer oder mehreren Gruppen R₅ substituierte C₁-C₆-Alkynyl-gruppe, eine 5- oder 6-gliedrige, gegebenenfalls mit einer oder mehreren Gruppen R₅ substituierte Heteroarylgruppe, eine 5- oder 6-gliedrige, gegebenenfalls mit einer oder mehreren Gruppen R₅ substituierte Heterocycloalkylgruppe, eine 5- oder 6-gliedrige, gegebenenfalls mit einer oder mehreren Gruppen R₅ substituierte Heteroaryloxygruppe stehen,
- R₅ für ein Halogenatom oder eine geradkettige oder verzweigte C₁-C₆-Alkylgruppe, eine C₆-Arylgruppe, eine geradkettige oder verzweigte C₁-C₆-Halogenalkylgruppe, eine Gruppe der Formel -ORₐ, -NRₐR_{b}, -CO₂Rₐ, -C(O)Rₐ und -C(O)NRₐR_{b}, eine Nitril- oder Nitrogruppe, -NRₐC(O)-T₁, eine C₁-C₆-Alkoxygruppe, eine Oxogruppe, eine Gruppe der Formel -S(O)ₜ-Rₐ, -S(O)ₜ-ORₐ, -S(O)ₜ-NRₐR_{b}, -P(O)ₜRₐ, -P(O)ₜ-ORₐ steht,
- Rₐ und R_{b} identisch oder verschieden sind und jeweils unabhängig voneinander für ein Wasserstoffatom oder eine geradkettige oder verzweigte C₁-C₆-Alkylgruppe, eine geradkettige oder verzweigte C₁-C₆-Halogenalkylgruppe, eine C₆-Arylgruppe stehen, wobei Rₐ + R_{b} gemeinsam mit dem Stickstoffatom, an das sie gebunden sind, einen monocyclischen oder bicyclischen, gesättigten oder ungesättigten Heterocyclus von 5 bis 10 Atomen bilden, der gegebenenfalls in den Ringsystemen ein zweites, aus Sauerstoff und Stickstoff ausgewähltes Heteroatom enthält, und gegebenenfalls mit einer oder mehreren Gruppen R₅ substituiert ist,
- T₁ für ein Wasserstoffatom, ein Halogenatom oder eine geradkettige oder verzweigte C₁-C₆-Alkylgruppe steht,die gegebenenfalls mit einer aus -ORₐ, -NRₐR_{b}, -CO₂Rₐ, -C(O)Rₐ und -C(O)NRₐR_{b} ausgewählten Gruppe substituiert ist,
- T₂ für eine geradkettige oder verzweigte Alkylidenkette (C₁-C₆) steht,
- T₃ für eine aus -Halogen, -ORₐ, -NRₐR_{b}, -CO₂Rₐ, -C(O)Rₐ und -C(O)NRₐR_{b} ausgewählte Gruppe steht, in denen Rₐ und R_{b} wie vorausgehend definiert sind,
t für eine ganze Zahl von zwischen einschließlich 0 und 3 steht,
- A, B, C, D die Ringe bezeichnen, welche die Verbindungen der Formel I darstellen und einzig den Zweck haben, jeden dieser Ringe zu identifizieren, oder eines von den Salzen, optischen Isomeren und racemischen Mischungen dieser Verbindungen, für die Bereitung eines Medikamentes zur Hemmung der Aktivität von mindestens einer der Kinasen PIM-1, PIM-2 und PIM-3.

2. Verwendung nach Anspruch 1 von mindestens einer Verbindung der Formel I, in der:
- R₁ H oder eine Sulfophenylgruppe ist,
- R₂ für H oder eine Gruppe der Formel CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂ Br steht, wobei n = 1 oder 2 und R_{c} = OCH₃, OC₂H₅, N(C₂H₅)₂,
- R₃ und R₄ identisch oder verschieden sind und unabhängig voneinander ausgewählt sind aus H, einem Halogenatom, einer 5- oder 6-gliedrigen Heteroarylgruppe mit einem oder zwei aus O und N ausgewählten Heteroatomen, einer geradkettigen oder verzweigten C₁-C₆-Alkylgruppe, einer Methoxy-, Nitro-, Nitril-, Carboxy-, Trifluormethyl-, Trifluormethoxygruppe, SO₂R_{d} einer C₆-Arylgruppe, die gegebenenfalls mit einer Gruppe ausgewählt aus einer Gruppe (C=O)CH₃, einer Phenyl-, Methoxy-, Trifluormethoxy-, Trifluormethyl-, Carboxygruppe oder mit 1 oder 2 Fluoratomen substituiert ist, wobei R_{d} aus einer Gruppe OH, CH₃ oder NH₂ ausgewählt ist.

3. Verwendung nach den Ansprüchen 1 oder 2 von mindestens einer Verbindung der Formel I, in der
- R₁ H oder eine Sulfophenylgruppe ist,
- R₂ für H oder für eine Gruppe der Formel CHO, (CH₂)ₙOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂ oder Br steht, wobei n = 1 oder 2, und R_{c} für eine Gruppe der Formel OCH₃, OC₂H₅oder N(C₂H₅)₂ steht,
- R₃ und R₄ identisch oder verschieden sind und unabhängig voneinander ausgewählt sind aus H, einem Halogenatom, einer Methyl-, Ethyl-, Nitro-, Nitril-, Trifluormethylgruppe, einer C₆-Arylgruppe, die gegebenenfalls mit einer aus (C=O)CH₃, Phenyl, Methoxy, Trifluormethoxy, Trifluormethyl ausgewählten Gruppe oder mit 1 oder 2 Fluoratom(en) substituiert ist.

4. Verwendung nach einem der Ansprüche 1 bis 3, **dadurch gekennzeichnet, dass** die mindestens eine Verbindung der Formel I ausgewählt ist aus 1,10-Dihydropyrrol[2,3-a]carbazol, 1,10-Dihydropyrrol[2,3-a]carbazol-3-carbaldehyd, 1,10-Dihydropyrrol[2,3-a]carbazol-3-carboxamid, 1-(1,10-Dihydropyrrol[2,3-a]carbazol-3-yl-2,2,2-trifluor-ethanon, 7-Brom-1,10-dihydropyrrol[2,3-a]carbazol, 7-Brom-1,10-dihydropyrrol[2,3-a]carbazol-3-carbaldehyd, 7-(2,4-Difluorphenyl)-1,10-dihydropyrrol[2,3-a]carbazol-3-carbaldehyd, 6-Brom-1,10-dihydropyrrol[2,3-a]carbazol-3-carbaldehyd, 9-Brom-1,10-dihydropyrrol[2,3-a]carbazol-3-carbaldehyd, 7-Fluor-1,10-dihydropyrrol[2,3-a]carbazol-3-carbaldehyd, 9-Fluor-1,10-dihydropyrrol[2,3-a]carbazol-3-carbaldehyd, 6,8-Dichlor-1,10-dihydropyrrol[2,3-a]carbazol-3-carbaldehyd, 9-Ethyl-1,10-dihydro-pyrrol[2,3-a]carbazol-3-carbaldehyd und 8-Methyl-1,10-dihydropyrrol[2,3-a]carbazol-3-carbaldehyd.

5. Verwendung nach einem der Ansprüche 1 bis 4, **dadurch gekennzeichnet, dass** die mindestens eine Verbindung der Formel I ausgewählt ist aus 1,10-Dihydropyrrol[2,3-a]carbazol, 1,10-Dihydropyrrol[2,3-a]carbazol-3-carbaldehyd und 1,10-Dihydro-pyrrol[2,3-a]carbazol-3-carboxamid.

6. Verfahren zum Synthetisieren der Verbindungen der folgenden Formel I: 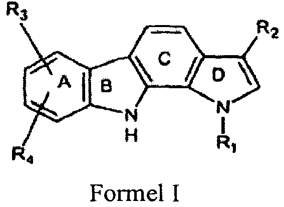 [*in der*
- R₁ *H oder eine Sulfophenylgruppe ist*,
- R₂, R₃ *und* R₄ *identisch oder verschieden sind und unabhängig voneinander stehen für ein Wasserstoffatom, ein Halogenatom*] oder eine Nitro-, Nitril-, Hydroxygruppe, eine geradkettige oder verzweigte, gegebenenfalls mit einer oder mehreren Gruppen R₅ substituierte C₁-C₆-Alkoxygruppe, eine gegebenenfalls mit einer oder mehreren Gruppen R₅ substituierte C₅-C₆-Cycloalkoxygruppe, eine 5- oder 6-gliedrige, gegebenenfalls mit einer oder mehreren Gruppen R₅ substituierte Heterocycloalkoxygruppe, -SH, eine geradkettige oder verzweigte, gegebenenfalls mit einer oder mehreren Gruppen R₅ substituierte C₁-C₆-Alkylthiogruppe, eine gegebenenfalls mit einer oder mehreren Gruppen R₅ substituierte C₆-Arylgruppe, eine gegebenenfalls mit einer oder mehreren Gruppen R₅ substituierte C₆-Aryloxygruppe, -NRₐR_{b}, -NRₐC(O)-T₁, -C(N-OH)-T₃, -C(O)-T₁, -C(O)-C(O)-T₃, -C(O)-NR₃-T₁, -NRₐ-C(O)-T₁, O-C(O)-T₁, -C(O)-O-T₁, -O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂-CO₂R₃ oder -S(O)ₜ-Rₐ, -S(O)ₜ-ORₐ, -S(O)ₜNRₐR_{b}, -P(O)ₜ-Rₐ, -P(O)ₜ-OR₃, eine geradkettige oder verzweigte, gegebenenfalls mit einer oder mehreren Gruppen R₅ substituierte C₁-C₆-Alkylgruppe, eine geradkettige oder verzweigte, gegebenenfalls mit einer oder mehreren Gruppen R₅ substituierte C₁-C₆-Alkenylgruppe, eine geradkettige oder verzweigte, gegebenenfalls mit einer oder mehreren Gruppen R₅ substituierte C₁-C₆-Alkynylgruppe, eine 5- oder 6-gliedrige, gegebenenfalls mit einer oder mehreren Gruppen R₅ substituierte Heteroarylgruppe, eine 5- oder 6-gliedrige, gegebenenfalls mit einer oder mehreren Gruppen R₅ substituierte Heterocycloalkylgruppe, eine 5- oder 6-gliedrige, gegebenenfalls mit einer oder mehreren Gruppen R₅ substituierte Heteroaryloxygruppe,
- R₅ für ein Halogenatom oder eine geradkettige oder verzweigte C₁-C₆Alkylgruppe, eine C₆-Arylgruppe, eine geradkettige oder verzweigte C₁-C₆-Halogenalkylgruppe, eine Gruppe der Formel -ORₐ, -NRₐR_{b3} -CO₂Rₐ, -C(O)Rₐ und -C(O)NRₐR_{b}, eine Nnitril- oder Nitrogruppe, eine Gruppe der Formel -NRₐC(O)-T₁, eine C₁-C₆-Alkoxygruppe, eine Oxogruppe, eine Gruppe der Formel -S(O)ₜ-Rₐ, -S(O)ₜ-ORₐ, -S(O)ₜ-NRₐR_{b}, -P(O)t-Rₐ, -P(O)t-ORₐ steht,
- Rₐ und R_{b} identisch oder verschieden sind und jeweils unabhängig voneinander für ein Wasserstoffatom stehen oder für eine geradkettige oder verzweigte C₁-C₆-Alkylgruppe, eine geradkettige oder verzweigte C₁-C₆-Halogenalkylgruppe, eine C₆-Arylgruppe, wobei Rₐ + R_{b} gemeinsam mit dem Stickstoffatom, an das sie gebunden sind, einen monocyclischen oder bicyclischen, gesättigten oder ungesättigten Heterocyclus von 5 bis 10 Atomen bilden, der gegebenenfalls in den Ringsystemen ein zweites, aus Sauerstoff und Stickstoff ausgewähltes Heteroatom enthält und gegebenenfalls mit einer oder mehreren Gruppen R₅ substituiert ist,
- T₁ für ein Wasserstoffatom, ein Halogenatom oder eine geradkettige oder verzweigte C₁-C₆-Alkylgruppe steht, die gegebenenfalls mit einer aus -ORₐ, -NRₐR_{b}, -CO₂Rₐ, -C(O)Rₐ und -C(O)NRₐR_{b} ausgewählten Gruppe substituiert ist,
- T₂ für eine geradkettige oder verzweigte Alkylidenkette (C₁-C₆) steht,
- T₃ für eine aus -Halogen, -ORₐ, -NRₐR_{b}, -CO₂Rₐ, -C(O)Rₐ und -C(O)NRₐR_{b} ausgewählte Gruppe steht, in der Rₐ und R_{b} wie vorausgehend definiert sind,
- t für eine ganze Zahl von zwischen einschließlich 0 und 3 steht,
- A, B, C, D Ringe bezeichnen, welche die Verbindungen der Formel I darstellen und einzig den Zweck haben, jeden dieser Ringe zu identifizieren,
**dadurch gekennzeichnet, dass** sie eine Fischer-Indolisierung der Verbindung 1-Benzolsulfonyl-1,4,5,6-tetrahydro-7H indol-7-on der nachfolgenden Formel II umfasst: 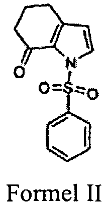 sowie von Phenylhydrazin oder von Phenylhydrazin, das an dem Phenyl mit einer oder mehreren Gruppen R₅ substituiert ist, in Gegenwart einer ionischen Flüssigkeit, bei der es sich um Zinkchlorid-Cholinchlorid 2:1 der nachfolgenden Formel III handelt:

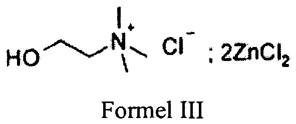

7. Verbindungen der folgenden Formel I: 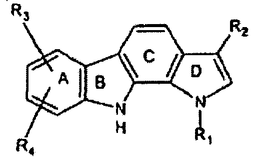 in der:
- R₁ H oder eine Sulfophenylgruppe ist,
- R₂, R₃ und R₄ identisch oder verschieden sind und unabhängig voneinander für ein Wasserstoffatom, ein Halogenatom oder eine Nitro-, Nitril-, Hydroxygruppe, eine geradkettige oder verzweigte, gegebenenfalls mit einer oder mehreren Gruppen R₅ substituierte C₁-C₆-Alkoxygruppe, eine gegebenenfalls mit einer oder mehreren Gruppen R₅ substituierte C₅-C₆-Cycloalkoxygruppe, eine gegebenenfalls mit einer oder mehreren Gruppen R₅ substituierte, 5- oder 6-gliedrige Heterocycloalkoxygruppe, -SH, eine geradkettige oder verzweigte, gegebenenfalls mit einer oder mehreren Gruppen R₅ substituierte C₁-C₆-Alkylthiogruppe, eine gegebenenfalls mit einer oder mehreren Gruppen R₅ substituierte C₆-Arylgruppe, eine gegebenenfalls mit einer oder mehreren Gruppen R₅ substituierte C₆-Aryloxygruppe, eine Gruppe der Formel -NRₐR_{b}, -NR₃C(O)-T₁, -C(N-OH)-T₃, -C(O)-T₁, -C(O)-C(O)-T₃, -C(O)-NRₐ-T₁, -NRₐ C(O)-T₁, O-C(O)-T₁, -C(O)-O-T₁, -O-T₂-NRₐR_{b}, -O-T₂-ORₐ, -O-T₂-CO₂Rₐ, -NRₐ-T₂-NRₐR_{b}, -NRₐ-T₂-ORₐ, -NRₐ-T₂CO₂Rₐ oder -S(O)ₜ-Rₐ, -S(O)ₜ-ORₐ, -S(O)ₜ-NRₐR_{b}, -P(O)ₜ-Rₐ, -P(O)t-ORₐ, eine geradkettige oder verzweigte, gegebenenfalls mit einer oder mehreren Gruppen R₅ substituierte C₁-C₆-Alkylgruppe, eine geradkettige oder verzweigte, gegebenenfalls mit einer oder mehreren Gruppen R₅ substituierte C₁-C₆-Alkenylgruppe, eine geradkettige oder verzweigte, gegebenenfalls mit einer oder mehreren Gruppen R₅ substituierte C₁-C₆-Alkynyl-gruppe, eine 5- oder 6-gliedrige, gegebenenfalls mit einer oder mehreren Gruppen R₅ substituierte Heteroarylgruppe, eine 5- oder 6-gliedrige, gegebenenfalls mit einer oder mehreren Gruppen R₅ substituierte Heterocycloalkylgruppe, eine 5- oder 6-gliedrige, gegebenenfalls mit einer oder mehreren Gruppen R₅ substituierte Heteroaryloxygruppe stehen,
- R₅ für ein Halogenatom oder eine geradkettige oder verzweigte C₁-C₆-Alkylgruppe, eine C₆-Arylgruppe, eine geradkettige oder verzweigte C₁-C₆-Halogenalkylgruppe, eine Gruppe der Formel -ORₐ, -NRₐR_{b}, -CO₂Rₐ, -C(O)Rₐ und -C(O)NRₐR_{b}, eine Nitril- oder Nitrogruppe, -NRₐC(O)-T₁, eine C₁-C₆-Alkoxygruppe, eine Oxogruppe, eine Gruppe der Formel -S(O)ₜ Rₐ, -S(O)ₜ-ORₐ, -S(O)ₜ-NRₐR_{b}, -P(O)t-Rₐ, -P(O)ₜ-ORₐ steht,
- Rₐ und R_{b} identisch oder verschieden sind und jeweils unabhängig voneinander für ein Wasserstoffatom oder eine geradkettige oder verzweigte C₁-C₆-Alkylgruppe, eine geradkettige oder verzweigte C₁-C₆-Halogenalkylgruppe, eine C₆-Arylgruppe stehen, wobei Rₐ + R_{b} gemeinsam mit dem Stickstoffatom, an das sie gebunden sind, einen monocyclischen oder bicyclischen, gesättigten oder ungesättigten Heterocyclus von 5 bis 10 Atomen bilden, der gegebenenfalls in den Ringsystemen ein zweites, aus Sauerstoff und Stickstoff ausgewähltes Heteroatom enthält, und gegebenenfalls mit einer oder mehreren Gruppen R₅ substituiert ist,
- T₁ für ein Wasserstoffatom, ein Halogenatom oder eine geradkettige oder verzweigte C₁-C₆-Alkylgruppe steht,die gegebenenfalls mit einer aus -ORₐ, -NRₐR_{b}, -CO₂Rₐ, -C(O)Rₐ und -C(O)NRₐR_{b} ausgewählten Gruppe substituiert ist,
- T₂ für eine geradkettige oder verzweigte Alkylidenkette (C₁-C₆) steht,
- T₃ für eine aus -Halogen, -ORₐ, -NRₐR_{b}, -CO₂Rₐ, -C(O)Rₐ und -C(O)NRₐR_{b} ausgewählte Gruppe steht, in denen Rₐ und R_{b} wie vorausgehend definiert sind,
- t für eine ganze Zahl von zwischen einschließlich 0 und 3 steht,
- A, B, C, D die Ringe bezeichnen, welche die Verbindungen der Formel I darstellen und einzig den Zweck haben, jeden dieser Ringe zu identifizieren
unter der Bedingung, dass:
- R₁, R₂, R₃ und R₄ nicht alle gleichzeitig H sind,
- R₂, R₃ und R₄ nicht alle gleichzeitig H sind, falls R₁ eine Sulfophenylgruppe ist, und
- R₁, R₃ und R₄ nicht alle gleichzeitig H sind, falls R₂ eine Carboxamid- oder Formylgruppe ist.

8. Verbindungen der Formel I gemäß Anspruch 7, **dadurch gekennzeichnet, dass** in der Formel I:
- R₁ H oder eine Sulfophenylgruppe ist,
- R₂ für H oder eine Gruppe der Formel CHO, (CH₂)nOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂, Br steht, wobei n = 1 oder 2 und R_{c} = OCH₃, OC₂H₅, N(C₂H₅)₂,
- R₃ und R₄ identisch oder verschieden sind und unabhängig voneinander ausgewählt sind aus H, einem Halogenatom, einer 5- oder 6-gliedrigen Heteroarylgruppe mit einem oder zwei Heteroatomen, die aus O und N ausgewählt sind, einer geradkettigen oder verzweigten C₁-C₆-Alkylgruppe, einer Methoxy-, Nitro-, Nitril-, Carboxy-, Trifluormethyl-, Trifluormethoxygruppe, SO₂R_{d}, einer C₆-Arylgruppe, die gegebenenfalls mit einer Gruppe ausgewählt aus einer Gruppe (C=O)CH₃, einer Phenyl-, Methoxy-, Trifluormethoxy-, Trifluormethyl-, Carboxygruppe oder mit 1 oder 2 Fluoratomen substituiert ist, wobei R_{d} aus einer Gruppe OH, CH₃ oder NH₂ ausgewählt ist.

9. Verbindungen gemäß den Ansprüchen 7 oder 8, **dadurch gekennzeichnet, dass** in der Formel I:
- R₁ H oder eine Sulfophenylgruppe ist,
- R₂ für H oder eine Gruppe der Formel CHO, (CH₂)nOH, C(=O)NH₂, C(=O)-CF₃, (C=O)₂R_{c}, (CH₂)₂NEt₂, CH(OH)CH₂N(Et)₂, C(NOH)-(C=O)N(Et)₂, NO₂ oder Br steht, wobei n = 1 oder 2 und R_{c} für eine Gruppe der Formel OCH₃, OC₂H₅ oder N(C₂H₅)₂ steht,
- R₃ und R₄ identisch oder verschieden sind und unabhängig voneinander ausgewählt sind aus H, einem Halogenatom, einer Methyl-, Ethyl-, Nitro-, Nitril- Trifluormethylgruppe, einer C₆-Arylgruppe, die gegebenenfalls mit einer aus (C=O)CH₃, Phenyl, Methoxy, Trifluormethoxy, Trifluormethyl ausgewählten Gruppe oder mit 1 oder 2 Fluoratom(en) substituiert ist.

10. Verbindungen der Formel I nach einem der Ansprüche 7 bis 9, **dadurch gekennzeichnet, dass** sie ausgewählt sind unter:
- (1,10-Dihydropyrrol[2,3-*a*]carbazol-3-yl)methanol,
- 1-(1,10-Dihydropyrrol[2,3-*a*]carbazol-3-yl)-2,2,2-trifluorethanon,
- Methyl 2-(1,10-dihydropyrrol[2,3-*a*]carbazol-3-yl)-2-oxoacetat,
- Ethyl 2-(1,10-dihydropyrrol[2,3-*a*]carbazol-3-yl)-2-oxoacetat,
- 2-(1,10-Dihydropyrrol[2,3-*a*]carbazol-3-yl)ethanol,
- 2-(1,10-Dihydropyrrol[2,3-*a*]carbazol-3-yl)-N,N-diethyl-2-oxoacetamid,
- 2-(1,10-Dihydropyrrol[2,3-*a*]carbazol-3-yl)-N,N-diethylethanamin,
- 1-(1,10-Dihydropyrrol[2,3-*a*]carbazol-3-yl)-2-diethylaminoethanol,
- 2-(1,10-Dihydropyrrol[2,3-*a*]carbazol-3-yl)-N,N-diethyl-2-hydroxyiminoethanamid,
- 1-Benzolsulfonyl-7-brom-1,10-dihydropyrrol[2,3-*a*]carbazol,
- 7-Brom-1,10-dihydropyrrol[2,3-*a*]carbazol,
- 7-Brom-1,10-dihydropyrrol[2,3-*a*]carbazol-3-carbaldehyd,
- 7-(4-Acetylphenyl)-1,10-dihydropyrrol[2,3-*a*]carbazol,
- 7-(3-Methoxyphenyl)-1,10-dihydropyrrol[2,3-*a*]carbazol,
- 7-(4-Biphenyl)-1,10-dihydropyrrol[2,3-*a*]carbazol,
- 7-Phenyl-1,10-dihydropyrrol[2,3-*a*]carbazol,
- 7-(4-Fluorphenyl)-1,10-dihydropyrrol[2,3-*a*]carbazol,
- 7-(2,4-Difluorphenyl)-1,10-dihydropyrrol[2,3-*a*]carbazol,
- 7-(4-Trifluormethylphenyl)-1,10-dihydropyrrol[2,3-*a*]carbazol,
- 7-(4-Trifluormethoxyphenyl)-1,10-dihydropyrrol[2,3-*a*]carbazol,
- 8-Brom-1,10-dihydropyrrol[2,3-*a*]carbazol,
- 6-Brom-1,10-dihydropyrrol[2,3-*a*]carbazol,
- 9-Brom-1,10-dihydropyrrol[2,3-*a*]carbazol,
- 7-Fluor-1,10-dihydropyrrol[2,3-*a*]carbazol,
- 9-Fluor-1,10-dihydropyrrol[2,3-*a*]carbazol,
- 6,8-Dichlor-1,10-dihydropyrrol[2,3-*a*]carbazol,
- 1,10-Dihydropyrrol[2,3-*a*]carbazol-7-carbonitril,
- 7-Nitro-1,10-dihydropyrrol[2,3-*a*]carbazol,
- 9-Ethyl-1,10-dihydropyrrol[2,3-*a*]carbazol,
- 9-(Trifluormethyl)-1,10-dihydropyrrol[2,3-*a*]carbazol,
- 8-Methyl-1,10-dihydropyrrol[2,3-*a*]carbazol,
- 7-(3-Methoxyphenyl)-1,10-dihydropyrrol[2,3-*a*]carbazol-3-carbaldehyd,
- 7-(4-Biphenyl)-1,10-dihydropyrrol[2,3-*a*]carbazol-3-carbaldehyd,
- 7-Phenyl-1,10-dihydropyrrol[2,3-*a*]carbazol-3-carbaldehyd,
- 7-(4-Fluorphenyl)-1,10-dihydropyrrol[2,3-*a*]carbazol-3-carbaldehyd,
- 7-(2,4-Difluorphenyl)-1,10-dihydropyrrol[2,3-*a*]carbazol-3-carbaldehyd,
- 7-(4-Trifluormethylphenyl)-1,10-dihydropyrrol[2,3-*a*]carbazol-3-carbaldehyd,
- 7-(4-Trifluormethoxyphenyl)-1,10-dihydropyrrol[2,3-*a*]carbazol-3-carbaldehyd,
- 8-Brom-1,10-dihydropyrrol[2,3-*a*]carbazol-3-carbaldehyd,
- 6-Brom-1,10-dihydropyrrol[2,3-*a*]carbazol-3-carbaldehyd,
- 9-Brom-1,10-dihydropyrrol[2,3-*a*]carbazol-3-carbaldehyd,
- 7-Fluor-1,10-dihydropyrrol[2,3-*a*]carbazol-3-carbaldehyd,
- 9-Fluor-1,10-dihydropyrrol[2,3-*a*]carbazol-3-carbaldehyd,
- 6,8-Dichlor-1,10-dihydropyrrol[2,3-*a*]carbazol-3-carbaldehyd,
- 3-Formyl-1,10-dihydropyrrol[2,3-*a*]carbazol-7-carbonitril,
- 7-Nitro-1,10-dihydropyrrol[2,3-*a*]carbazol-3-carbaldehyd,
- 9-Ethyl-1,10-dihydropyrrol[2,3-*a*]carbazol-3-carbaldehyd,
- 8-Methyl-1,10-dihydropyrrol[2,3-*a*]carbazol-3-carbaldehyd.

11. Verbindungen der Formel I nach einem der Ansprüche 7 bis 9, **dadurch gekennzeichnet, dass** sie ausgewählt sind aus 1,10-Dihydropyrrol[2,3-*a*]carbazol-3-yl)-2,2,2-Trifluor-ethanon, 7-Brom-1,10-dihydropyrrol[2,3-*a*]carbazol, 7-Brom-1,10-dihydropyrrol[2,3-*a*]carbazol-3-carbaldehyd, 7-(2,4-Difluorphenyl)-1,10-Dihydropyrrol[2,3-*a*]carbazol-3-carbaldehyd, 6-Brom-1,10-dihydropyrrol[2,3-*a*]carbazol-3-carbaldehyd, 9-Brom-1,10-dihydropyrrol[2,3-*a*]carbazol-3-carbaldehyd, 7-Fluor-1,10-dihydropyrrol[2,3-a]carbazol-3-carbaldehyd, 9-Fluor-1,10-dihydropyrrol[2,3-*a*]carbazol-3-carbaldehyd, 6,8-Dichlor-1,10-dihydropyrrol[2,3-*a*]carbazol-3-carbaldehyd, 9-Ethyl-1,10-dihydropyrrol[2,3-*a*]carbazol-3-carbaldehyd und 8-Methyl-1,10-dihydropyrrol[2,3-*a*]carbazol-3-carbaldehyd.
